# EUROPEAN PATENT APPLICATION

(11) **EP 1 705 176 A1**
(43) Date of publication of application: **27.09.2006**
(21) Application number: 05703857.2
(22) Date of filing: 13.01.2005
(51) Int. Cl.: C07D 211/00

(54) **CARBOXAMIDE DERIVATIVE AND USE THEREOF**

(30) Priority: 14.01.2004 JP 2004007373
(71) Applicant: Takeda Pharmaceutical Company Limited, Osaka-shi, Osaka 541-0045 (JP)
(72) Inventor: IKEURA, Yoshinori, 6390251 (JP); SHIRAI, Junya, Amaggasaki-shi, Hyogo 6610033 (JP)
(74) Representative: Rickard, Timothy Mark Adrian
(86) International application number: PCT/JP2005/000627
(87) International publication number: WO 2005/068427

(57) **Abstract**

The present invention relates to a compound represented by the formula 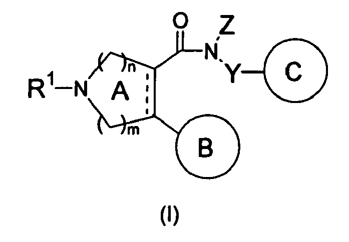 wherein ring A is a nitrogen-containing heterocycle optionally further having substituent(s), ring B and ring C are each an aromatic ring optionally having substituent(s), R¹ is a hydrogen atom, a hydrocarbon group optionally having substituent(s), an acyl group or a heterocyclic group optionally having substituent(s), Z is an optionally halogenated C₁₋₆ alkyl group, Y is a methylene group optionally having substituent(s), m and n are each an integer of 0 to 5, m+n is an integer of 2 to 5, and 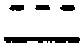 is a single bond or a double bond, or a salt thereof. The compound of the present invention has a superior tachykinin receptor antagonistic action, particularly an SP receptor antagonistic action, and is useful as a pharmaceutical agent, for example, a tachykinin receptor antagonist, an agent for the prophylaxis or treatment of an abnormality of lower urinary tract functions, a digestive organ disease or a central nerve disease, and the like.

## Description

### Technical Field

The present invention relates to a novel carboxamide derivative having excellent antagonistic action for a tachykinin receptor and use thereof.

### Background Art

Tachykinin is a generic term for a group of neuropeptides. Substance P (SP), neurokinin A and neurokinin B are known in mammals, and these peptides are known to bind to the corresponding receptors (neurokinin-1, neurokinin-2 and neurokinin-3) that exist in a living body and thereby to exhibit various biological activities.

Of such neuropeptides, SP has the longest history and has been studied in detail. In 1931, the existence of SP in the extract from equine intestines was confirmed, and in 1971, its structure was determined. SP is a peptide consisting of 11 amino acids.

SP is broadly distributed over the central and peripheral nervous systems, and has various physiological activities such as vasodilation, enhancement of vascular extravasation, contraction of smooth muscles, excitation of neurons, salivation, enhancement of diuresis, immunological enhancement and the like, in addition to the function as a transmitter substance for primary sensory neurons. In particular, it is known that SP released from the terminal of the spinal (dorsal) horn due to a pain impulse transmits the information of pain to secondary neurons, and that SP released from the peripheral terminal induces an inflammatory response in the receptor thereof. Thus, it is considered that SP is involved in various disorders (e.g., pain, headache, particularly migraine, Alzheimer's disease, multiple sclerosis, cardiovascular modulation, chronic inflammatory diseases such as chronic rheumatic arthritis, respiratory diseases including asthma or allergic rhinitis, intestinal inflammatory diseases including ulcerative colitis and Crohn's disease, ocular damage and ocular inflammatory diseases, proliferative vitreous retinopathy, an irritable bowel syndrome, urinary frequency, psychosis, vomiting, etc.) [see, for example, Physiological Reviews, Vol. 73, pp. 229-308 (1993); Journal of Autonomic Pharmacology, Vol. 13, pp. 23-93 (1993)].

At present, the following compounds have been known as those having antagonistic action for SP receptors.

WO01/25219 describes a compound represented by the formula 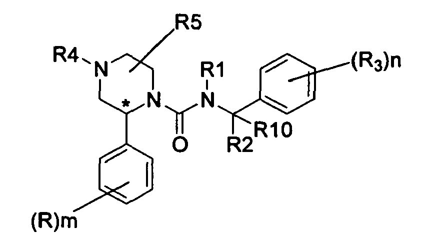 wherein R represents a halogen atom or a C₁₋₄ alkyl group, R₁ represents a hydrogen atom or a C₁₋₄ alkyl group, R₂ represents a hydrogen atom or a C₁₋₄ alkyl group, R₃ represents a trifluoromethyl group, a C₁₋₄ alkyl group, a C₁₋₄ alkoxy group, a trifluoromethoxy group or a halogen atom, R₄ represents a hydrogen atom, a (CH₂)qR₇ group or a (CH₂)rCO(CH₂)pR₇ group, R₅ represents a hydrogen atom, a C₁₋₄ alkyl group or a COR₆ group, R₆ represents a hydrogen atom, hydroxy, amino, methylamino, dimethylamino, a 5-membered heteroaryl group containing 1 to 3 heteroatoms selected from a group consisting of oxygen, sulfur and nitrogen or a 6-membered heteroaryl group containing 1 to 3 nitrogen atoms, R₇ represents a hydrogen atom, hydroxy or NR₈R₉ wherein R₈ and R₉ independently represent a hydrogen atom or a C₁₋₄ alkyl group optionally substituted by hydroxy or amino group, R₁₀ represents a hydrogen atom, m is 0 or an integer of 1 to 3, n is 0 or an integer of 1 to 3, p and r are each independently 0 or an integer of 1 to 4, and q is an integer of 1 to 4, and pharmaceutically acceptable salts and solvates thereof.

In addition, WO02/081457 describes a compound represented by the formula 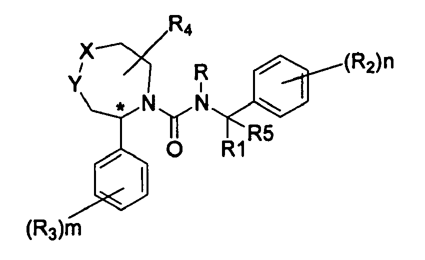 wherein R represents hydrogen or C₁₋₄ alkyl, R₁ represents hydrogen or C₁₋₄ alkyl, R₂ represents trifluoromethyl, C₁₋₄ alkyl, C₁₋₄ alkoxy, trifluoromethoxy or halogen, R₃ represents halogen or C₁₋₄ alkyl, R₄ represents hydrogen, halogen, C₁₋₄ alkyl or C(O)R₆, R₅ represents hydrogen, C₁₋₄ alkyl or R₅ together with R₁ represents C₃₋₇ cycloalkyl, R₆ represents hydroxy, amino, methylamino, dimethylamino, a 5-membered heteroaryl group containing 1 to 3 heteroatoms independently selected from a group consisting of oxygen, sulfur and nitrogen or a 6-membered heteroaryl group containing 1 to 3 nitrogen atoms, m and n are independently 0 or an integer of 1 to 3, X and Y are independently NR₇ or methylene, R₇ represents hydrogen, C₁₋₄ alkyl or C₃₋₇ cycloalkyl, provided that when X is NR₇, Y is methylene and when X is methylene, Y is NR₇, and pharmaceutically acceptable salts and solvates thereof.

Furthermore, WO03/101964 describes a compound represented by the formula 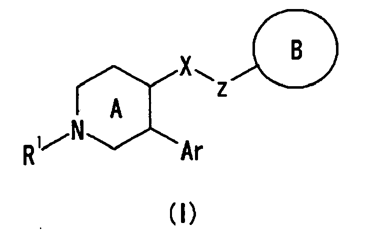 wherein Ar is an aryl group, an aralkyl group or an aromatic heterocyclic group, each of which may be substituted, R¹ is a hydrogen atom, an optionally substituted hydrocarbon group, an acyl group or an optionally substituted heterocyclic group, X is an oxygen atom or an optionally substituted imino group, Z is an optionally substituted methylene group, Ring A is a further optionally substituted piperidine ring, and Ring B is an optionally substituted aromatic ring, provided that when Z is a methylene group substituted with an oxo group, R¹ is not a methyl group, and when Z is a methylene group substituted with a methyl group, Ring B is a substituted aromatic ring, or a salt thereof.

### Disclosure of the Invention

An object of the present invention is to provide a carboxamide derivative having antagonistic action for a tachykinin receptor, etc. with a different chemical structure from the known compounds including the above-mentioned compounds, an agent for ameliorating abnormal micturition comprising the compound, and the like.

The present inventors have made extensive studies in consideration of the above-mentioned situation and, as a result, have found unexpectedly that carboxamide derivatives represented by the formula (I) below or a salt thereof have excellent antagonistic action for a tachykinin receptor (particularly antagonistic action for a SP receptor) as based on their peculiar chemical structures and are sufficiently satisfactory as pharmaceutical agents. On the basis of these findings, the present inventors have completed the present invention.

Specifically, the present invention provides the following:
[1] a compound represented by the formula 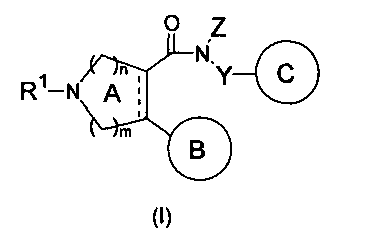 wherein ring A is a nitrogen-containing heterocycle optionally further having substituent(s), ring B and ring C are each an aromatic ring optionally having substituent(s), R¹ is a hydrogen atom, a hydrocarbon group optionally having substituent(s), an acyl group or a heterocyclic group optionally having substituent(s), Z is an optionally halogenated C₁₋₆ alkyl group, Y is a methylene group optionally having substituent(s), m and n are each an integer of 0 to 5, m+n is an integer of 2 to 5, and 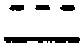 is a single bond or a double bond (hereinafter sometimes to be abbreviated as compound (I)) or a salt thereof;
[2] the compound of [1], wherein ring A is any of the rings shown by 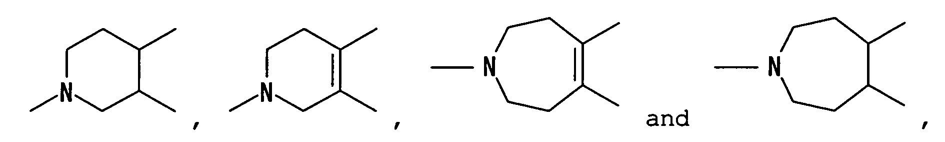 ring B is an optionally substituted phenyl group,
   ring C is a phenyl group optionally having, as a substituent,
   a C₁₋₆ alkyl group optionally substituted by a halogen atom,
   Z is a C₁₋₆ alkyl group, and
   Y is a methylene group optionally substituted by a C₁₋₄ alkyl group;
[3] the compound of [1], wherein ring A is any of the rings shown by 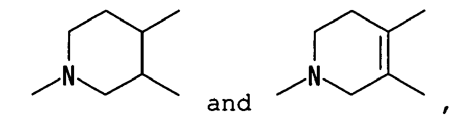 ring B is a phenyl group optionally substituted by substituent(s) selected from a group consisting of
   (1) a halogen atom and
   (2) a C₁₋₆ alkyl group,
   ring C is a phenyl group optionally having, as a substituent,
   a C₁₋₆ alkyl group optionally substituted by a halogen atom,
   R¹ is (1) a hydrogen atom,
   (2) a C₁₋₄ alkyl group having, as a substituent, a 5- to 7-membered aromatic or non-aromatic heterocyclic group containing, besides carbon atoms, 1 to 4 heteroatoms selected from a group consisting of an oxygen atom, a sulfur atom and a nitrogen atom and optionally having one or two oxo as substituents, or
   (3) an acyl group represented by the formula: -(C=O)-R^{2'}, -(C=O)-OR^{2'} or -(C=O)-NR^{2'}R³
      wherein R^{2'} is
      (a) a hydrogen atom,
      (b) a 5- to 7-membered non-aromatic heterocyclic group containing, besides carbon atoms, 1 or 2 nitrogen atoms, and optionally having 1 to 3 substituents selected from a group consisting of oxo, C₁₋₆ alkyl, phenyl, C₁₋₆ alkyl-carbonyl and C₁₋₆ alkyl-carbonylamino,
      (c) a C₁₋₆ alkyl group optionally having substituent(s) selected from a group consisting of
         (i) a 5- to 7-membered aromatic or non-aromatic heterocyclic group containing, besides carbon atoms, 1 to 4 nitrogen atoms, and optionally having one or two oxo as substituents,
         (ii) a C₁₋₆ alkyl-carbonylamino group,
         (iii) a mono- or di-C₁₋₆ alkylamino group, and
         (iv) a C₁₋₆ alkoxy group,
      (d) a C₁₋₆ alkoxy group,
      (e) a C₃₋₈ cycloalkyl group optionally having 1 or 2 substituents selected from a group consisting of a C₁₋₆ alkyl-carbonylamino group, a C₁₋₆ alkoxy-carbonylamino group and an amino group,
      (f) a carbamoyl group,
      (g) a C₁₋₆ alkoxy-carbonyl group, or
      (h) a C₁₋₆ alkyl-carbamoyl group, and
      R³ is a hydrogen atom or a C₁₋₆ alkyl group,
      Z is a C₁₋₆ alkyl group, and
      Y is a methylene group optionally substituted by a C₁₋₄ alkyl group;
[4] a compound selected from a group consisting of
   (3R*,4S*)-1-[(1-acetylpiperidin-4-yl)carbonyl]-N-[3,5-bis(trifluoromethyl)benzyl]-N-methyl-3-phenylpiperidine-4-carboxamide (Example 30),
   (3R*,4R*)-N-[3,5-bis(trifluoromethyl)benzyl]-3-(4-fluoro-2-methylphenyl)-N-methyl-1-[(5-oxo-4,5-dihydro-1H-1,2,4-triazol-3-yl)methyl]piperidine-4-carboxamide (Example 54),
   (3R*,4R*)-1-[amino(oxo)acetyl]-N-[3,5-bis(trifluoromethyl)benzyl]-3-(4-fluoro-2-methylphenyl)-N-methylpiperidine-4-carboxamide (Example 73),
   (3R*,4R*)-N⁴-[3,5-bis(trifluoromethyl)benzyl]-3-(4-fluoro-2-methylphenyl)-N⁴-methylpiperidine-1,4-dicarboxamide (Example 75), and
   (3R*,4R*)-1-(N-acetylglycyl)-N-[3, 5-bis(trifluoromethyl)benzyl]-3-(4-fluoro-2-methylphenyl)-N-methylpiperidine-4-carboxamide (Example 77), or a salt thereof;
[5] a prodrug of the compound of [1];
[6] a pharmaceutical agent comprising the compound of [1] or a prodrug thereof;
[7] the pharmaceutical agent of [6], which is a tachykinin receptor antagonist;
[8] the pharmaceutical agent of [6], which is an agent for the prophylaxis or treatment of an abnormality of lower urinary tract functions, a digestive organ disease or a central nerve disease;
[9] the pharmaceutical agent of [6], which is an agent for the prophylaxis or treatment of an overactive bladder, an irritable bowel syndrome, an inflammatory bowel disease, vomiting, nausea, depression, anxiety neurosis, an anxiety symptom, a pelvic visceral pain or interstitial cystitis;
[10] a method for the prophylaxis or treatment of an abnormality of lower urinary tract functions, a digestive organ disease or a central nerve disease, which comprises administering an effective amount of the compound of [1] or a prodrug thereof to a mammal; and
[11] use of the compound of [1] or a prodrug thereof for the production of an agent for the prophylaxis or treatment of an abnormality of lower urinary tract functions, a digestive organ disease or a central nerve disease.
   Moreover, the present invention provides
[12] the compound of [1], wherein
   ring A is a 5- to 8-membered saturated or unsaturated
   nitrogen-containing heterocycle optionally further having 1 to 3 substituents selected from the following substituent group A,
   substituent group A: (1) a halogen atom, (2) nitro, (3) cyano, (4) C₁₋₆ alkyl optionally having 1 to 5 halogen atoms, (5) C₂₋₆ alkenyl optionally having 1 to 3 halogen atoms, (6) C₂₋₆ alkynyl optionally having 1 to 3 halogen atoms, (7) C₃₋₆ cycloalkyl optionally having 1 to 5 halogen atoms, (8) C₆₋₁₄ aryl, (9) C₇₋₁₆ aralkyl, (10) hydroxy, (11) C₁₋₆ alkoxy optionally having 1 to 3 halogen atoms, (12) C₆₋₁₄ aryloxy, (13) mercapto, (14) C₁₋₆ alkylthio optionally having 1 to 3 halogen atoms, (15) C₆₋₁₄ arylthio, (16) amino, (17) mono-C₁₋₆ alkylamino, (18) mono-C₆₋₁₄ arylamino, (19) di-C₁₋₆ alkylamino, (20) di-C₆₋₁₄ arylamino, (21) formyl, (22) C₁₋₆ alkyl-carbonyl, (23) C₆₋₁₄ aryl-carbonyl, (24) carboxy, (25) C₁₋₆ alkoxy-carbonyl, (26) C₆₋₁₄ aryloxy-carbonyl, (27) carbamoyl, (28) thiocarbamoyl, (29) mono-C₁₋₆ alkyl-carbamoyl, (30) di-C₁₋₆ alkyl-carbamoyl, (31) C₆₋₁₄ aryl-carbamoyl, (32) C₁₋₆ alkylsulfonyl, (33) C₆₋₁₄ arylsulfonyl, (34) C₁₋₆ alkylsulfinyl, (35) C₆₋₁₄ arylsulfinyl, (36) formylamino, (37) C₁₋₆ alkyl-carbonylamino, (38) C₆₋₁₄ aryl-carbonylamino, (39) C₁₋₆ alkoxy-carbonylamino, (40) C₁₋₆ alkylsulfonylamino, (41) C₆₋₁₄ arylsulfonylamino, (42) C₁₋₆ alkyl-carbonyloxy, (43) C₆₋₁₄ aryl-carbonyloxy, (44) C₁₋₆ alkoxy-carbonyloxy, (45) mono-C₁₋₆ alkyl-carbamoyloxy, (46) di-C₁₋₆ alkyl-carbamoyloxy, (47) C₆₋₁₄ aryl-carbamoyloxy, (48) a 5- to 7-membered saturated cyclic amino containing, besides one nitrogen atom and carbon atoms, 1 to 4 heteroatoms of one or two kinds selected from a group consisting of a nitrogen atom, a sulfur atom and an oxygen atom, (49) a 5- to 10-membered aromatic heterocyclic group containing, besides carbon atoms, 1 to 4 heteroatoms of one or two kinds selected from a group consisting of a nitrogen atom, a sulfur atom and an oxygen atom, (50) C₁₋₃ alkylenedioxy and (51) oxo,
   ring B is a C₆₋₁₄ aryl group optionally having 1 to 3 substituents selected from a group consisting of (1) to (50) of the above-mentioned substituent group A, or a 5- or 6-membered aromatic heterocyclic group containing, besides carbon atoms, 1 to 4 heteroatoms of one or two kinds selected from a group consisting of a nitrogen atom, an oxygen atom and a sulfur atom, and optionally having 1 to 3 substituents selected from a group consisting of (1) to (50) of the above-mentioned substituent group A,
   ring C is a C₆₋₁₄ aryl group optionally having 1 to 3 substituents selected from a group consisting of (1) to (50) of the above-mentioned substituent group A, or a 5- or 6-membered aromatic heterocyclic group containing, besides carbon atoms, 1 to 4 heteroatoms of one or two kinds selected from a group consisting of a nitrogen atom, an oxygen atom and a sulfur atom, and optionally having 1 to 3 substituents selected from a group consisting of (1) to (50) of the above-mentioned substituent group A, R¹ is
   (1) a hydrogen atom,
   (2) a C₁₋₆ alkyl group, a C₂₋₆ alkenyl group, a C₂₋₆ alkynyl group, a C₃₋₈ cycloalkyl group, a C₆₋₁₄ aryl group or a C₇₋₁₆ aralkyl group, each optionally having 1 to 3 substituents selected from the above-mentioned substituent group A and a heterocyclic group optionally having substituent(s) (said heterocyclic group is a 5- to 7-membered aromatic or non-aromatic heterocyclic group containing, besides carbon atoms, 1 to 4 heteroatoms selected from a group consisting of a nitrogen atom, an oxygen atom and a sulfur atom, and optionally having 1 to 3 substituents selected from the above-mentioned substituent group A),
   (3) an acyl group represented by the formula: -(C=O)-R², -(C=O)-OR², -(C=O)-NR²R³, -(C=S)-NHR² or -SO₂-R⁴
      wherein R² is
      (a) a hydrogen atom,
      (b) a C₁₋₆ alkyl group, a C₂₋₆ alkenyl group, a C₂₋₆ alkynyl group, a C₃₋₈ cycloalkyl group, a C₆₋₁₄ aryl group or a aralkyl group, each optionally having 1 to 3 substituents selected from the above-mentioned substituent group A and a heterocyclic group optionally having substituent(s) (said heterocyclic group is a 5- to 7-membered aromatic or non-aromatic heterocyclic group containing, besides carbon atoms, 1 to 4 heteroatoms selected from a group consisting of a nitrogen atom, an oxygen atom and a sulfur atom, and optionally having 1 to 3 substituents selected from the above-mentioned substituent group A),
      (c) a 5- to 7-membered aromatic or non-aromatic heterocyclic group containing, besides carbon atoms, 1 to 4 heteroatoms selected from a group consisting of a nitrogen atom, an oxygen atom and a sulfur atom, and optionally having 1 to 3 substituents selected from the above-mentioned substituent group A,
      (d) a C₁₋₆ alkoxy group,
      (e) a carbamoyl group,
      (f) a C₁₋₆ alkoxy-carbonyl group, or
      (g) a C₁₋₆ alkyl-carbamoyl group,
      R³ is a hydrogen atom or a C₁₋₆ alkyl group, and
      R⁴ is
      (a) a C₁₋₆ alkyl group, a C₂₋₆ alkenyl group, a C₂₋₆ alkynyl group, a C₃₋₈ cycloalkyl group, a C₆₋₁₄ aryl group or a C₇₋₁₆ aralkyl group, each optionally having 1 to 3 substituents selected from the above-mentioned substituent group A and a heterocyclic group optionally having substituent(s) (said heterocyclic group is a 5- to 7-membered aromatic or non-aromatic heterocyclic group containing, besides carbon atoms, 1 to 4 heteroatoms selected from a group consisting of a nitrogen atom, an oxygen atom and a sulfur atom, and optionally having 1 to 3 substituents selected from the above-mentioned substituent group A), or
      (b) a 5- to 7-membered aromatic or non-aromatic heterocyclic group containing, besides carbon atoms, 1 to 4 heteroatoms selected from a group consisting of a nitrogen atom, an oxygen atom and a sulfur atom, and optionally having 1 to 3 substituents selected from the above-mentioned substituent group A,
   Z is an optionally halogenated C₁₋₆ alkyl group, and
   Y is a methylene group optionally having 1 to 3 substituents selected from the above-mentioned substituent group A; and
[13] the compound of [1], wherein
   ring A is any of the rings shown by 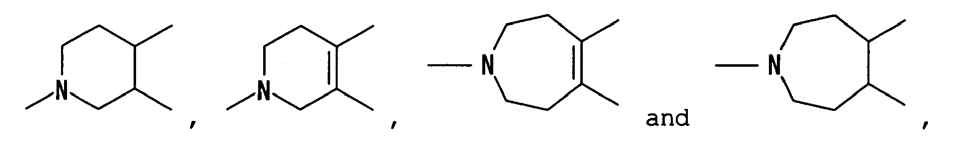 ring B is a phenyl group optionally substituted by substituent(s) selected from a group consisting of
   (1) a halogen atom and
   (2) a C₁₋₆ alkyl group,
   ring C is a phenyl group optionally having, as a substituent,
   a C₁₋₆ alkyl group optionally substituted by a halogen atom,
   R¹ is
   (1) a hydrogen atom,
   (2) a C₇₋₁₆ aralkyl group,
   (3) a C₁₋₄ alkyl group having, as a substituent, a 5- to 7-membered aromatic or non-aromatic heterocyclic group containing, besides carbon atoms, 1 to 4 heteroatoms selected from a group consisting of an oxygen atom, a sulfur atom and a nitrogen atom and optionally having one or two oxo as substituents, or
   (4) an acyl group represented by the formula: -(C=O)-R^{2"}, -(C=O)-OR^{2"} or -(C=O)-NR^{2"}R³
      wherein R^{2"} is
      (a) a hydrogen atom,
      (b) a 5- to 7-membered aromatic or non-aromatic heterocyclic group containing, besides carbon atoms, 1 to 4 heteroatoms selected from a group consisting of an oxygen atom, a sulfur atom and a nitrogen atom, and optionally having 1 to 3 substituents selected from a group consisting of oxo, C₁₋₆ alkyl, phenyl, C₁₋₆ alkyl-carbonyl and C₁₋₆ alkyl-carbonylamino,
      (c) a C₁₋₆ alkyl group optionally having substituent(s) selected from a group consisting of
         (i) a 5- to 7-membered aromatic or non-aromatic heterocyclic group containing, besides carbon atoms, 1 to 4 heteroatoms selected from a group consisting of an oxygen atom, a sulfur atom and a nitrogen atom, and optionally having 1 to 3 substituents selected from a group consisting of oxo, C₁₋₆ alkyl, phenyl, C₁₋₆ alkyl-carbonyl and C₁₋₆ alkyl-carbonylamino,
         (ii) a C₁₋₆ alkyl-carbonylamino group,
         (iii) a mono- or di-C₁₋₆ alkylamino group, and
         (iv) a C₁₋₆ alkoxy group,
      (d) a C₁₋₆ alkoxy group,
      (e) a C₃₋₈ cycloalkyl group optionally having 1 or 2 substituents selected from a group consisting of a C₁₋₆ alkyl-carbonylamino group, a C₁₋₆ alkoxy-carbonylamino group and an amino group,
      (f) a carbamoyl group,
      (g) a C₁₋₆ alkoxy-carbonyl group, or
      (h) a C₁₋₆ alkyl-carbamoyl group, and
      R³ is a hydrogen atom or a C₁₋₆ alkyl group,
      Z is a C₁₋₆ alkyl group, and
      Y is a methylene group optionally substituted by a C₁₋₄ alkyl group.

Compound (I) of the present invention and a salt thereof have a high antagonistic action for a tachykinin receptor, particularly an antagonistic action for substance P receptor, and have low toxicity, and are safe as pharmaceutical agents. Therefore, compound (I) of the present invention and a salt thereof are useful as medicaments, for example, a tachykinin receptor antagonist, an agent for ameliorating abnormal micturition, and the like.

### Best Mode for Embodying the Invention

In the aforementioned formulas, ring A is a nitrogen-containing heterocycle optionally further having substituent(s), m and n are each an integer of 0 to 5, m+n is an integer of 2 to 5, and 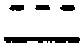 is a single bond or a double bond. Namely, ring A is a 5- to 8-membered saturated or unsaturated nitrogen-containing heterocycle containing, as a ring-constituting atom, one nitrogen atom and 4 to 7 carbon atoms, and optionally having substituent(s) besides R¹, ring B and a partial structure:

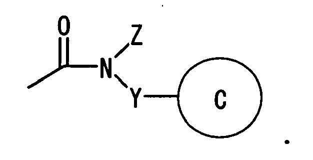

As the substituent for ring A, for example, 1 to 3 substituents selected from a group consisting of (1) a halogen atom (e.g., fluorine, chlorine, bromine, iodine, etc.), (2) nitro, (3) cyano, (4) C₁₋₆ alkyl optionally having 1 to 5 (preferably 1 to 3) halogen atoms (e.g., methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl, etc.), (5) C₂₋₆ alkenyl optionally having 1 to 3 halogen atoms (e.g., vinyl, allyl, isopropenyl, butenyl, isobutenyl, sec-butenyl, 3,3,3-trifluoro-1-propenyl, 4,4,4-trifluoro-1-butenyl, etc.), (6) C₂₋₆ alkynyl optionally having 1 to 3 halogen atoms (e.g., ethynyl, propargyl, butynyl, 1-hexynyl, 3,3,3-trifluoro-1-propynyl, 4,4,4-trifluoro-1-butynyl, etc.), (7) C₃₋₆ cycloalkyl optionally having 1 to 5 (preferably 1 to 3) halogen atoms (e.g., cyclopropyl, cyclobutyl, cyclopentyl, cyclohexyl, 4,4-dichlorocyclohexyl, 2,2,3,3-tetrafluorocyclopentyl, 4-chlorocyclohexyl, etc.), (8) C₆₋₁₄ aryl (e.g., phenyl, 1-naphthyl, 2-naphthyl, biphenylyl, 2-anthryl, etc.), (9) C₇₋₁₆ aralkyl (e.g., benzyl, phenethyl, diphenylmethyl, 1-naphthylmethyl, 2-naphthylmethyl, 2,2-diphenylethyl, 3-phenylpropyl, 4-phenylbutyl, 5-phenylpentyl, etc.), (10) hydroxy, (11) C₁₋₆ alkoxy optionally having 1 to 3 halogen atoms (e.g., methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, hexyloxy, etc.), (12) C₆₋₁₄ aryloxy (e.g., phenyloxy, naphthyloxy, etc.), (13) mercapto, (14) C₁₋₆ alkylthio optionally having 1 to 3 halogen atoms (e.g., methylthio, difluoromethylthio, trifluoromethylthio, ethylthio, propylthio, isopropylthio, butylthio, 4,4,4-trifluorobutylthio, pentylthio, hexylthio, etc.), (15) C₆₋₁₄ arylthio (e.g., phenylthio, naphthylthio, etc.), (16) amino, (17) mono-C₁₋₆ alkylamino (e.g., methylamino, ethylamino, etc.), (18) mono-C₆₋₁₄ arylamino (e.g., phenylamino, 1-naphthylamino, 2-naphthylamino, etc.), (19) di-C₁₋₆ alkylamino (e.g., dimethylamino, diethylamino, etc.), (20) di-C₆₋₁₄ arylamino (e.g., diphenylamino, etc.), (21) formyl, (22) C₁₋₆ alkyl-carbonyl (e.g., acetyl, propionyl, etc.), (23) C₆₋₁₄ aryl-carbonyl (e.g., benzoyl, 1-naphthoyl, 2-naphthoyl, etc.), (24) carboxy, (25) C₁₋₆ alkoxy-carbonyl (e.g., methoxycarbonyl, ethoxycarbonyl, propoxycarbonyl, tert-butoxycarbonyl, etc.), (26) C₆₋₁₄ aryloxy-carbonyl (e.g., phenoxycarbonyl, etc.), (27) carbamoyl, (28) thiocarbamoyl, (29) mono-C₁₋₆ alkyl-carbamoyl (e.g., methylcarbamoyl, ethylcarbamoyl, etc.), (30) di-C₁₋₆ alkyl-carbamoyl (e.g., dimethylcarbamoyl, diethylcarbamoyl, ethylmethylcarbamoyl, etc.), (31) C₆₋₁₄ aryl-carbamoyl (e.g., phenylcarbamoyl, 1-naphthylcarbamoyl, 2-naphthylcarbamoyl, etc.), (32) C₁₋₆ alkylsulfonyl (e.g., methylsulfonyl, ethylsulfonyl, etc.), (33) C₆₋₁₄ arylsulfonyl (e.g., phenylsulfonyl, 1-naphthylsulfonyl, 2-naphthylsulfonyl, etc.), (34) C₁₋₆ alkylsulfinyl (e.g., methylsulfinyl, ethylsulfinyl, etc.), (35) C₆₋₁₄ arylsulfinyl (e.g., phenylsulfinyl, 1-naphthylsulfinyl, 2-naphthylsulfinyl, etc.), (36) formylamino, (37) C₁₋₆ alkyl-carbonylamino (e.g., acetylamino, etc.), (38) C₆₋₁₄ aryl-carbonylamino (e.g., benzoylamino, naphthoylamino, etc.), (39) C₁₋₆ alkoxy-carbonylamino (e.g., methoxycarbonylamino, ethoxycarbonylamino, propoxycarbonylamino, butoxycarbonylamino, etc.), (40) C₁₋₆ alkylsulfonylamino (e.g., methylsulfonylamino, ethylsulfonylamino, etc.), (41) C₆₋₁₄ arylsulfonylamino (e.g., phenylsulfonylamino, 2-naphthylsulfonylamino, 1-naphthylsulfonylamino, etc.), (42) C₁₋₆ alkyl-carbonyloxy (e.g., acetoxy, propionyloxy, etc.), (43) C₆₋₁₄ aryl-carbonyloxy (e.g., benzoyloxy, naphthylcarbonyloxy, etc.), (44) C₁₋₆ alkoxy-carbonyloxy (e.g., methoxycarbonyloxy, ethoxycarbonyloxy, propoxycarbonyloxy, butoxycarbonyloxy, etc.), (45) mono-C₁₋₆ alkyl-carbamoyloxy (e.g., methylcarbamoyloxy, ethylcarbamoyloxy, etc.), (46) di-C₁₋₆ alkyl-carbamoyloxy (e.g., dimethylcarbamoyloxy, diethylcarbamoyloxy, etc.), (47) C₆₋₁₄ aryl-carbamoyloxy (e.g., phenylcarbamoyloxy, naphthylcarbamoyloxy, etc.), (48) a 5- to 7-membered saturated cyclic amino containing, besides one nitrogen atom and carbon atoms, 1 to 4 heteroatoms of one or two kinds selected from a group consisting of a nitrogen atom, a sulfur atom and an oxygen atom (e.g., pyrrolidin-1-yl, piperidino, piperazin-1-yl, morpholino, thiomorpholino, hexahydroazepin-1-yl, etc.), (49) a 5- to 10-membered aromatic heterocyclic group containing, besides carbon atoms, 1 to 4 heteroatoms of one or two kinds selected from a group consisting of a nitrogen atom, a sulfur atom and an oxygen atom (e.g., 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl, etc.), (50) C₁₋₃ alkylenedioxy (e.g., methylenedioxy, ethylenedioxy, etc.), (51) oxo, and the like can be mentioned.

As ring A, one wherein m=1 and n=2, namely, 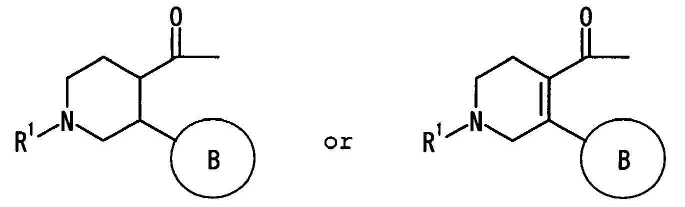 or one wherein m=n=2, namely, 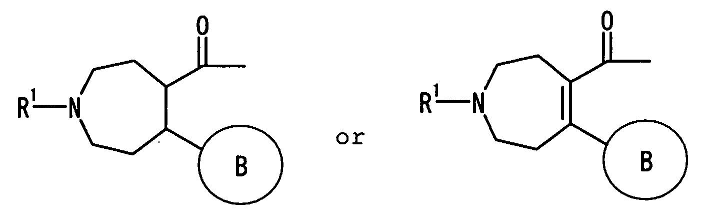 is preferable.

In the aforementioned formulas, ring B and ring C are each an aromatic ring optionally having substituent(s).

As the "aromatic ring", an aryl group or an aromatic heterocyclic group can be mentioned.

As the "aryl group", for example, a C₆₋₁₄ aryl group (e.g., phenyl, naphthyl, anthryl, phenanthryl, etc.) and the like are used, with preference given to phenyl.

As the "aromatic heterocyclic group", for example, a 5-or 6-membered aromatic heterocyclic group containing, besides carbon atoms, 1 to 4 heteroatoms of one or two kinds selected from a group consisting of a nitrogen atom, an oxygen atom and a sulfur atom (e.g., furyl, thienyl, pyridyl, imidazolyl, thiazolyl, oxazolyl, thiadiazolyl, triazolyl, tetrazolyl, etc.), and the like can be used.

As the substituent of the "aromatic ring" for ring B or ring C, 1 to 3 substituents similar to those of (1)-(50) exemplified for the substituent of the above-mentioned ring A can be used.

Ring B is preferably an optionally substituted phenyl group, more preferably a phenyl group optionally having 1 to 3 substituents selected from a group consisting of (1) a C₁₋₆ alkyl group optionally having 1 to 3 halogen atoms (e.g., fluorine, chlorine, bromine, iodine, etc.) and (2) a halogen atom (e.g., fluorine, chlorine, bromine, iodine, etc.), and further preferably a phenyl group optionally having 1 to 3 substituents selected from a group consisting of (1) a C₁₋₆ alkyl group and (2) a halogen atom.

Ring C is preferably a phenyl group having 1 to 3 substituents selected from a group consisting of (1) a C₁₋₆ alkyl group optionally having 1 to 3 halogen atoms (e.g., fluorine, chlorine, bromine, iodine, etc.) and (2) a halogen atom (e.g., fluorine, chlorine, bromine, iodine, etc.). Alternatively, ring C is preferably a phenyl group optionally having, as substituent(s), 1 to 3 C₁₋₆ alkyl groups optionally substituted by 1 to 3 halogen atoms.

In the aforementioned formulas, R¹ is a hydrogen atom, a hydrocarbon group optionally having substituent(s), a heterocyclic group optionally having substituent(s) or an acyl group.

As the "hydrocarbon group" of the "hydrocarbon group optionally having substituent(s)" for R¹, for example, an aliphatic hydrocarbon group, a monocyclic saturated hydrocarbon group, an aromatic hydrocarbon group and the like can be mentioned, with preference given to one having 1 to 16 carbon atoms. To be specific, for example, alkyl, alkenyl, alkynyl, cycloalkyl, aryl, aralkyl and the like are used.

As the "alkyl", for example, C₁₋₆ alkyl (e.g., methyl, ethyl, propyl, isopropyl, butyl, isobutyl, sec-butyl, tert-butyl, pentyl, hexyl, etc.) and the like are preferable, and C₁₋₄ alkyl is more preferable.

As the "alkenyl", for example, C₂₋₆ alkenyl (e.g., vinyl, allyl, isopropenyl, 1-butenyl, 2-butenyl, 3-butenyl, 2-methyl-2-propenyl, 1-methyl-2-propenyl, 2-methyl-1-propenyl, etc.) and the like are preferable.

As the "alkynyl", for example, C₂₋₆ alkynyl (e.g., ethynyl, propargyl, 1-butynyl, 2-butynyl, 3-butynyl, 1-hexynyl, etc.) and the like are preferable.

As the "cycloalkyl", for example, C₃₋₈ cycloalkyl (e.g., cyclopropyl, cyclobutyl, cyclopentyl, cyclohexyl, etc.) and the like are preferable and C₃₋₆ cycloalkyl is more preferable.

As the "aryl", for example, C₆₋₁₄ aryl (e.g., phenyl, 1-naphthyl, 2-naphthyl, 2-biphenylyl, 3-biphenylyl, 4-biphenylyl, 2-anthryl, etc.) and the like are preferable.

As the "aralkyl", for example, C₇₋₁₆ aralkyl (e.g., benzyl, phenethyl, diphenylmethyl, 1-naphthylmethyl, 2-naphthylmethyl, 2,2-diphenylethyl, 3-phenylpropyl, 4-phenylbutyl, 5-phenylpentyl, etc.) and the like are preferable.

As the "heterocyclic group" of the "heterocyclic group optionally having substituent(s)" for R¹, for example, a 5- to 14-membered (preferably 5- to 10-membered) monocyclic to tricyclic (preferably monocyclic or bicyclic) aromatic or non-aromatic heterocyclic group containing, besides carbon atoms, 1 to 4 (preferably 1 to 3) heteroatoms of one or two kinds of selected from a group consisting of a nitrogen atom, an oxygen atom and a sulfur atom and the like can be mentioned. For example, a 5-membered ring group containing, besides carbon atoms, 1 to 4 heteroatoms selected from a group consisting of an oxygen atom, a sulfur atom and a nitrogen atom such as 2-or 3-thienyl, 2- or 3-furyl, 1-, 2- or 3-pyrrolyl, 1-, 2- or 3-pyrrolidinyl, 2-, 4- or 5-oxazolyl, 3-, 4- or 5-isoxazolyl, 2-, 4- or 5-thiazolyl, 3-, 4- or 5-isothiazolyl, 3-, 4- or 5-pyrazolyl, 2-, 3- or 4-pyrazolidinyl, 2-, 4- or 5-imidazolyl, 1-, 2- or 4-imidazolidinyl, 1,2,3-triazolyl, 1,2,4-triazolyl, 1H- or 2H-tetrazolyl and the like; a 6-membered ring group containing, besides carbon atoms, 1 to 4 heteroatoms selected from a group consisting of an oxygen atom, a sulfur atom and a nitrogen atom such as 2-, 3- or 4-pyridyl, N-oxide-2-, 3- or 4-pyridyl, 2-, 4- or 5-pyrimidinyl, N-oxide-2-, 4- or 5-pyrimidinyl, thiomorpholinyl, morpholinyl, piperidino, 2-, 3-or 4-piperidyl, thiopyranyl, 1,4-oxazinyl, 1,4-thiazinyl, 1,3-thiazinyl, piperazinyl, triazinyl, 3- or 4-pyridazinyl, pyrazinyl, N-oxide-3- or 4-pyridazinyl and the like; a bicyclic or tricyclic fused ring group containing, besides carbon atoms, 1 to 4 heteroatoms selected from a group consisting of an oxygen atom, a sulfur atom and a nitrogen atom (preferably, a group formed by condensation of the above-mentioned 5- or 6-membered ring and one or two 5- or 6-membered ring group containing, besides carbon atoms, 1 to 4 heteroatoms selected from a group consisting of an oxygen atom, a sulfur atom and a nitrogen atom), such as indolyl, benzofuryl, benzothiazolyl, benzoxazolyl, benzimidazolyl, quinolyl, isoquinolyl, phthalazinyl, quinazolinyl, quinoxalinyl, indolizinyl, quinolizinyl, 1,8-naphthyridinyl, dibenzofuranyl, carbazolyl, acridinyl, phenanthridinyl, chromanyl, phenothiazinyl, phenoxazinyl and the like; and the like are used. Of these, a 5- to 7-membered (preferably 5- or 6-membered) aromatic or non-aromatic heterocyclic group containing, besides carbon atoms, 1 to 4 heteroatoms selected from a group consisting of an oxygen atom, a sulfur atom and a nitrogen atom is preferable.

As the "substituent" of the "hydrocarbon group optionally having substituent(s)" or "heterocyclic group optionally having substituent(s)" for R¹, 1 to 3 substituents similar to those exemplified for the substituent of the above-mentioned ring A are used.

Furthermore, when R¹ is a "hydrocarbon group optionally having substituent(s)", the substituent may be a "heterocyclic group optionally having substituent(s)". As the "heterocyclic group optionally having substituent(s)", which is a substituent on the hydrocarbon group, those exemplified as the above-mentioned "heterocyclic group optionally having substituent(s)" for R¹ can be mentioned. Preferably, a 5- to 10-membered (preferably 5- to 7-membered, more preferably 5-or 6-membered) aromatic or non-aromatic heterocyclic group containing, besides carbon atoms, 1 to 4 heteroatoms selected from a group consisting of a nitrogen atom, a sulfur atom and an oxygen atom and optionally having one or two oxo as substituents and, for example, 1H- or 2H-tetrazolyl, 1-, 2- or 4-imidazolidinyl, piperidino, 2-, 3- or 4-piperidyl, 1-, 2- or 3-pyrrolidinyl, 4,5-dihydro-1,2,4-triazolyl and the like can be mentioned.

As the "acyl group" for R¹, for example, an acyl group represented by the formula: -(C=O)-R², -(C=O)-OR², -(C=O)-NR²R³, -(C=S)-NHR² or -SO₂-R⁴, wherein R² is a hydrogen atom, a hydrocarbon group optionally having substituent(s), a heterocyclic group optionally having substituent(s), a C₁₋₆ alkoxy group, a carbamoyl group, a C₁₋₆ alkoxy-carbonyl group or a C₁₋₆ alkyl-carbamoyl group, R³ is a hydrogen atom or a C₁₋₆ alkyl group, and R⁴ is a hydrocarbon group optionally having substituent(s) or a heterocyclic group optionally having substituent(s) can be mentioned.

As the "hydrocarbon group optionally having substituent(s)" and "heterocyclic group optionally having substituent(s)" for R² or R⁴, those similar to the "hydrocarbon group optionally having substituent(s)" or "heterocyclic group optionally having substituent(s)" for R¹ are used.

As the "C₁₋₆ alkoxy group" for R², for example, methoxy, ethoxy, propoxy, isopropoxy, butoxy, isobutoxy, sec-butoxy, tert-butoxy, pentyloxy, hexyloxy and the like can be mentioned.

As the "C₁₋₆ alkoxy-carbonyl group" for R², methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl and the like can be mentioned.

As the "C₁₋₆ alkyl-carbamoyl group" for R², methylcarbamoyl, ethylcarbamoyl, propylcarbamoyl, isopropylcarbamoyl, butylcarbamoyl, isobutylcarbamoyl, sec-butylcarbamoyl, tert-butylcarbamoyl, pentylcarbamoyl, hexylcarbamoyl and the like can be mentioned.

As the "C₁₋₆ alkyl group" for R³, for example, methyl, ethyl, propyl, isopropyl, butyl, isobutyl, sec-butyl, tert-butyl, pentyl, hexyl and the like can be mentioned.

R¹ is preferably
(1) a hydrogen atom,
(2) a C₇₋₁₆ aralkyl group (e.g., benzyl, etc.),
(3) a C₁₋₄ alkyl group having, as a substituent, a 5- to 7-membered aromatic or non-aromatic heterocyclic group containing, besides carbon atoms, 1 to 4 heteroatoms selected from a group consisting of an oxygen atom, a sulfur atom and a nitrogen atom and optionally having one or two oxo as substituents, or
(4) an acyl group represented by the formula: -(C=O)-R^{2"}, -(C=O)-OR^{2"} or -(C=O)-NR^{2"}R³
   wherein R^{2"} is
   (a) a hydrogen atom,
   (b) a 5- to 7-membered aromatic or non-aromatic heterocyclic group containing, besides carbon atoms, 1 to 4 heteroatoms selected from a group consisting of an oxygen atom, a sulfur atom and a nitrogen atom, and optionally having 1 to 3 substituents selected from a group consisting of oxo, C₁₋₆ alkyl, phenyl, C₁₋₆ alkyl-carbonyl and C₁₋₆ alkyl-carbonylamino,
   (c) a C₁₋₆ alkyl group optionally having substituent(s) selected from a group consisting of
      (i) a 5- to 7-membered aromatic or non-aromatic heterocyclic group containing, besides carbon atoms, 1 to 4 heteroatoms selected from a group consisting of an oxygen atom, a sulfur atom and a nitrogen atom, and optionally having 1 to 3 substituents selected from a group consisting of oxo, C₁₋₆ alkyl, phenyl, C₁₋₆ alkyl-carbonyl and C₁₋₆ alkyl-carbonylamino,
      (ii) a C₁₋₆ alkyl-carbonylamino group,
      (iii) a mono- or di-C₁₋₆ alkylamino group, and
      (iv) a C₁₋₆ alkoxy group,
   (d) a C₁₋₆ alkoxy group,
   (e) a C₃₋₈ cycloalkyl group optionally having 1 or 2 substituents selected from a group consisting of a C₁₋₆ alkyl-carbonylamino group, a C₁₋₆ alkoxy-carbonylamino group and an amino group,
   (f) a carbamoyl group,
   (g) a C₁₋₆ alkoxy-carbonyl group, or
   (h) a C₁₋₆ alkyl-carbamoyl group, and
   R³ is a hydrogen atom or a C₁₋₆ alkyl group.

More preferably, R¹ is
(1) a hydrogen atom,
(2) a C₁₋₄ alkyl group having, as a substituent, a 5- to 7-membered aromatic or non-aromatic heterocyclic group containing, besides carbon atoms, 1 to 4 heteroatoms selected from a group consisting of an oxygen atom, a sulfur atom and a nitrogen atom and optionally having one or two oxo as substituents (e.g., 4,5-dihydro-1H-1,2,4-triazol-3-yl, etc.) or
(3) an acyl group represented by the formula: -(C=O)-R^{2'}, -(C=O)-OR^{2'} or -(C=O)-NR^{2'}R³
   wherein R^{2'} is
   (a) a hydrogen atom,
   (b) a 5- to 7-membered non-aromatic heterocyclic group containing, besides carbon atoms, 1 or 2 nitrogen atoms, and optionally having 1 to 3 substituents selected from a group consisting of oxo, C₁₋₆ alkyl, phenyl, C₁₋₆ alkyl-carbonyl and C₁₋₆ alkyl-carbonylamino (e.g., piperidin-1-yl, piperidin-4-yl, piperazin-1-yl, etc.),
   (c) a C₁₋₆ alkyl group optionally having substituent(s) selected from a group consisting of
      (i) a 5- to 7-membered aromatic or non-aromatic heterocyclic group containing, besides carbon atoms, 1 to 4 nitrogen atoms, and optionally having one or two oxo as substituents (e.g., 1H-tetrazol-1-yl, imidazolidin-4-yl, piperidin-1-yl, pyrrolidin-1-yl, etc.),
      (ii) a C₁₋₆ alkyl-carbonylamino group,
      (iii) a mono- or di-C₁₋₆ alkylamino group, and
      (iv) a C₁₋₆ alkoxy group,
   (d) a C₁₋₆ alkoxy group,
   (e) a C₃₋₈ cycloalkyl group optionally having 1 or 2 substituents selected from a group consisting of a C₁₋₆ alkyl-carbonylamino group, a C₁₋₆ alkoxy-carbonylamino group and an amino group,
   (f) a carbamoyl group,
   (g) a C₁₋₆ alkoxy-carbonyl group, or
   (h) a C₁₋₆ alkyl-carbamoyl group, and
R³ is a hydrogen atom or a C₁₋₆ alkyl group.

In the aforementioned formulas, Z is an optionally halogenated C₁₋₆ alkyl group.

As the "optionally halogenated C₁₋₆ alkyl group" for Z, C₁₋₆ alkyl optionally having 1 to 5 (preferably 1 to 3) halogen atoms (e.g., methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl, etc.) and the like can be mentioned.

Z is preferably a C₁₋₆ alkyl group, and a C₁₋₄ alkyl group such as methyl and the like is more preferable.

In the aforementioned formulas, Y is a methylene group optionally having substituent(s).

As the "substituent" of the "methylene group optionally having substituent(s)" for Y, 1 to 3 substituents similar to those exemplified as the substituent of the above-mentioned ring A can be used.

Y is preferably a methylene group optionally having a C₁₋₄ alkyl group as a substituent, and more preferably a methylene group.

As compound (I), the following compounds are preferably used.

### [Compound (I)-1]

Compound (I) wherein ring A is any of the rings shown by 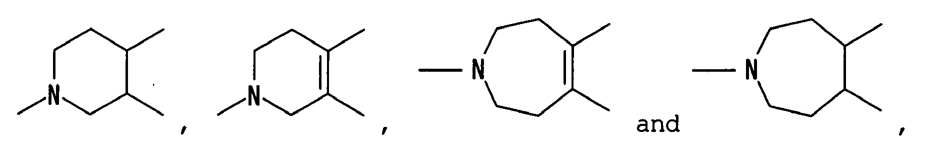 ring B is an optionally substituted phenyl group,
ring C is a phenyl group optionally having, as a substituent,
a C₁₋₆ alkyl group optionally substituted by a halogen atom,
Z is a C₁₋₆ alkyl group, and
Y is a methylene group optionally substituted by a C₁₋₄ alkyl group.

### [Compound (I)-2]

Compound (I) wherein ring A is any of the rings shown by 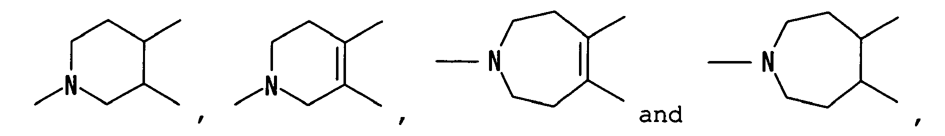 ring B is a phenyl group optionally substituted by substituent(s) selected from a group consisting of
(1) a halogen atom and
(2) a C₁₋₆ alkyl group,
ring C is a phenyl group optionally having, as a substituent,
a C₁₋₆ alkyl group optionally substituted by a halogen atom,
R¹ is
(1) a hydrogen atom,
(2) a C₇₋₁₆ aralkyl group,
(3) a C₁₋₄ alkyl group having, as a substituent, a 5- to 7-membered aromatic or non-aromatic heterocyclic group containing, besides carbon atoms, 1 to 4 heteroatoms selected from a group consisting of an oxygen atom, a sulfur atom and a nitrogen atom and optionally having one or two oxo as substituents, or
(4) an acyl group represented by the formula: -(C=O)-R^{2"}, -(C=O)-OR^{2"} or -(C=O)-NR^{2"}R³
   wherein R^{2"} is
   (a) a hydrogen atom,
   (b) a 5- to 7-membered aromatic or non-aromatic heterocyclic group containing, besides carbon atoms, 1 to 4 heteroatoms selected from a group consisting of an oxygen atom, a sulfur atom and a nitrogen atom, and optionally having 1 to 3 substituents selected from a group consisting of oxo, C₁₋₆ alkyl, phenyl, C₁₋₆ alkyl-carbonyl and C₁₋₆ alkyl-carbonylamino,
   (c) a C₁₋₆ alkyl group optionally having substituent(s) selected from a group consisting of
      (i) a 5- to 7-membered aromatic or non-aromatic heterocyclic group containing, besides carbon atoms, 1 to 4 heteroatoms selected from a group consisting of an oxygen atom, a sulfur atom and a nitrogen atom, and optionally having 1 to 3 substituents selected from a group consisting of oxo, C₁₋₆ alkyl, phenyl, C₁₋₆ alkyl-carbonyl and C₁₋₆ alkyl-carbonylamino,
      (ii) a C₁₋₆ alkyl-carbonylamino group,
      (iii) a mono- or di-C₁₋₆ alkylamino group, and
      (iv) a C₁₋₆ alkoxy group,
   (d) a C₁₋₆ alkoxy group,
   (e) a C₃₋₈ cycloalkyl group optionally having 1 or 2 substituents selected from a group consisting of a C₁₋₆ alkyl-carbonylamino group, a C₁₋₆ alkoxy-carbonylamino group and an amino group,
   (f) a carbamoyl group,
   (g) a C₁₋₆ alkoxy-carbonyl group, or
   (h) a C₁₋₆ alkyl-carbamoyl group, and
R³ is a hydrogen atom or a C₁₋₆ alkyl group,
Z is a C₁₋₆ alkyl group, and
Y is a methylene group optionally substituted by a C₁₋₄ alkyl group.

### [Compound (I)-3]

Compound (I) wherein ring A is any of the rings shown by 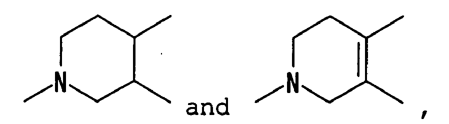 ring B is a phenyl group optionally substituted by substituent(s) selected from a group consisting of
(1) a halogen atom and
(2) a C₁₋₆ alkyl group,
ring C is a phenyl group optionally having, as a substituent,
a C₁₋₆ alkyl group optionally substituted by a halogen atom,
R¹ is (1) a hydrogen atom,
(2) a C₁₋₄ alkyl group having, as a substituent, a 5- to 7-membered aromatic or non-aromatic heterocyclic group containing, besides carbon atoms, 1 to 4 heteroatoms selected from a group consisting of an oxygen atom, a sulfur atom and a nitrogen atom and optionally having one or two oxo as substituents, or
(3) an acyl group represented by the formula: -(C=O)-R^{2'}, -(C=O)-OR^{2'} or -(C=O)-NR^{2'}R³
   wherein R^{2'} is
   (a) a hydrogen atom,
   (b) a 5- to 7-membered non-aromatic heterocyclic group containing, besides carbon atoms, 1 or 2 nitrogen atoms, and optionally having 1 to 3 substituents selected from a group consisting of oxo, C₁₋₆ alkyl, phenyl, C₁₋₆ alkyl-carbonyl and C₁₋₆ alkyl-carbonylamino,
   (c) a C₁₋₆ alkyl group optionally having substituent(s) selected from a group consisting of
      (i) a 5- to 7-membered aromatic or non-aromatic heterocyclic group containing, besides carbon atoms, 1 to 4 nitrogen atoms, and optionally having one or two oxo as substituents,
      (ii) a C₁₋₆ alkyl-carbonylamino group,
      (iii) a mono- or di-C₁₋₆ alkylamino group, and
      (iv) a C₁₋₆ alkoxy group,
   (d) a C₁₋₆ alkoxy group,
   (e) a C₃₋₈ cycloalkyl group optionally having 1 or 2 substituents selected from a group consisting of a C₁₋₆ alkyl-carbonylamino group, a C₁₋₆ alkoxy-carbonylamino group and an amino group,
   (f) a carbamoyl group,
   (g) a C₁₋₆ alkoxy-carbonyl group, or
   (h) a C₁₋₆ alkyl-carbamoyl group, and
   R³ is a hydrogen atom or a C₁₋₆ alkyl group,
Z is a C₁₋₆ alkyl group, and
Y is a methylene group optionally substituted by a C₁₋₄ alkyl group.

### [Compound (I)-4]

A compound selected from a group consisting of (3R*,4S*)-1-[(1-acetylpiperidin-4-yl)carbonyl]-N-[3,5-bis(trifluoromethyl)benzyl]-N-methyl-3-phenylpiperidine-4-carboxamide,

(3R*,4R*)-N-[3,5-bis(trifluoromethyl)benzyl]-3-(4-fluoro-2-methylphenyl)-N-methyl-1-[(5-oxo-4,5-dihydro-1H-1,2,4-triazol-3-yl)methyl]piperidine-4-carboxamide,

(3R*,4R*)-1-[amino(oxo)acetyl]-N-[3,5-bis(trifluoromethyl)benzyl]-3-(4-fluoro-2-methylphenyl)-N-methylpiperidine-4-carboxamide,

(3R*,4R*)-N⁴-[3,5-bis(trifluoromethyl)benzyl]-3-(4-fluoro-2-methylphenyl)-N⁴-methylpiperidine-1,4-dicarboxamide, and

(3R*,4R*) -1-(N-acetylglycyl)-N-[3,5-bis(trifluoromethyl)benzyl]-3-(4-fluoro-2-methylphenyl)-N-methylpiperidine-4-carboxamide, or a salt thereof.

A salt of compound (I) includes, for example, a metal salt, an ammonium salt, a salt with an organic base, a salt with an inorganic acid, a salt with an organic acid, a salt with basic or acidic amino acid, etc. Suitable examples of the metal salt include an alkali metal salt such as a sodium salt, a potassium salt, etc.; an alkaline earth metal salt such as a calcium salt, a magnesium salt, a barium salt, etc.; an aluminum salt, etc. Suitable examples of the salts with an organic base include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, etc. Suitable examples of the salts with an inorganic acid include salts with hydrochloric acid, hydrobromic acid, nitric acid, sulfuric acid, phosphoric acid, etc. Suitable examples of the salts with an organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid, etc. Suitable examples of the salts with basic amino acid include salts with arginine, lysine, ornithine, etc. Suitable examples of the salts with acidic amino acid include salts with aspartic acid and glutamic acid, etc.

Among these, pharmaceutically acceptable salts are preferred. For example, if the compound has acidic functional group, preferred are inorganic salts such as an alkali metal salt (e.g., sodium salt, potassium salt, etc.), an alkaline earth metal salt (e.g., calcium salt, magnesium salt, barium salt, etc.), an ammonium salt, etc. If the compound has a basic functional group, preferred are salts with an inorganic acid such as hydrochloric acid, hydrobromic acid, nitric acid, sulfuric acid, phosphoric acid, etc., or salts with an organic acid such as acetic acid, phthalic acid, fumaric acid, oxalic acid, tartaric acid, maleic acid, citric acid, succinic acid, methanesulfonic acid, p-toluenesulfonic acid, etc.

The prodrug of compound (I) or a salt thereof of the present invention means a compound which is converted to compound (I) of the present invention under the physiological condition in the living body by a reaction with an enzyme, a gastric acid, or the like, that is, by enzymatic oxidation, reduction, hydrolysis, etc.; by hydrolysis with gastric acid, etc.

The prodrug of compound (I) of the present invention includes a compound wherein the amino group of compound (I) is modified with acyl, alkyl or phosphoryl (e.g., a compound wherein the amino group of compound (I) of the present invention is modified with eicosanyl, alanyl, pentylaminocarbonyl, (5-methyl-2-oxo-1,3-dioxolen-4-yl)methoxycarbonyl, tetrahydrofuranyl, pyrrolidylmethyl, pivaloyloxymethyl or t-butyl, etc.); a compound wherein the hydroxy group of compound (I) of the present invention is modified with acyl, alkyl, phosphoric acid or boric acid (e.g., a compound wherein the hydroxy group of compound (I) is modified with acetyl, palmitoyl, propanoyl, pivaloyl, succinyl, fumaryl, alanyl or dimethylaminomethylcarbonyl, etc.); a compound wherein a carboxyl group of compound (I) of the present invention is modified to ester or amide (e.g., a compound wherein a carboxyl group of compound (I) of the present invention is modified to ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester or methylamide, etc.); and the like. These prodrugs can be produced from compound (I) of the present invention by a method known per se.

In addition, the prodrug of compound (I) of the present invention may be a compound, which is converted into compound (I) of the present invention under the physiological conditions, as described in "Pharmaceutical Research and Development", Vol. 7 (Drug Design), pp. 163-198 (1990), published by Hirokawa Publishing Co.

A solvate, for example, hydrate of the compound represented by the formula (I) and a salt thereof are all included in the scope of the present invention. The compound represented by the formula (I) may be labeled with an isotope (e.g., ³H, ¹⁴C, ³⁵S, ¹²⁵I, etc.) and the like.

If compound (I) according to the present invention has chiral center, isomers such as an enantiomer or a diastereomer may exist. Such isomers and a mixture thereof are all included in the scope of the present invention. In addition, there can be instances where the isomers by conformation are generated in cases, but such isomers or a mixture thereof are also included in compound (I) of the present invention or a salt thereof.

A method of preparing compound (I) of the present invention or a salt thereof will be explained in the following.

Compound (I) of the present invention or a salt thereof can be produced using Method A, Method B, Method C or Method D below.

### [Method A]

Compound (I) of the present invention or a salt thereof can be produced by subjecting a compound represented by the formula 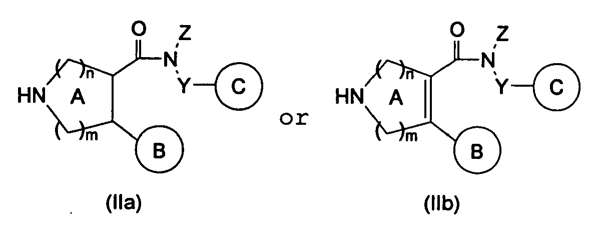 wherein each symbol is as defined above (hereinafter to be referred to as compound (IIa) or (IIb)) or a salt thereof to alkylation reaction or acylation reaction. This reaction can be carried out by a method known per se. For example, compound (IIa) or (IIb) is reacted with a compound represented by the formula

R^{1a}-OH (IX)

wherein R^{1a} is a hydrocarbon group optionally having substituent(s), an acyl group or a heterocyclic group optionally having substituent(s), which is an alkylating agent or acylating agent, or a salt thereof or a reactive derivative thereof.

As the "hydrocarbon group optionally having substituent(s), acyl group or heterocyclic group optionally having substituent(s)" for R^{1a}, those similar to the examples of R¹ can be used.

As a reactive derivative of the compound represented by R^{1a}-OH or a salt thereof, for example, a compound represented by the formula

R^{1a}-L¹ (X)

wherein L¹ is a leaving group and R^{1a} is as defined above, or a salt thereof (hereinafter to be simply referred to as a reactive derivative) can be used.

The leaving group represented by L¹ includes, for example, a hydroxy group, a halogen atom (a chlorine atom, a bromine atom, an iodine atom, etc.), a substituted sulfonyloxy group (a C₁₋₆ alkylsulfonyloxy group such as methanesulfonyloxy, ethanesulfonyloxy, etc.; a C₆₋₁₄ arylsulfonyloxy group such as benzenesulfonyloxy, p-toluenesulfonyloxy, etc.; and a C₇₋₁₆ aralkylsulfonyloxy group such as a benzylsulfonyloxy group, etc.), an acyloxy group (acetoxy, benzoyloxy, etc.), an oxy group substituted with a heterocycle or an aryl group (succinimide, benzotriazole, quinoline, 4-nitrophenyl, etc.), a heterocycle (imidazole, etc.) and the like.

The reaction using the above-mentioned reactive derivative as an alkylating agent can be carried out by reacting compound (IIa) or (IIb) with the reactive derivative, usually in a solvent in the presence of a base. The solvent includes, for example, alcohols (methanol, ethanol, propanol, etc.), ethers (dimethoxyethane, dioxane, tetrahydrofuran, etc.), ketones (acetone, etc.), nitriles (acetonitrile, etc.), amides (N,N-dimethylformamide, etc.), sulfoxides (dimethyl sulfoxide, etc.), water and the like, which may be used in a suitable mixture. The base includes, for example, an organic base (trimethylamine, triethylamine, N-methylmorpholine, pyridine, picoline, N,N-dimethylaniline, etc.) and an inorganic base (potassium carbonate, sodium carbonate, potassium hydroxide, sodium hydroxide, etc.). The amount of the base is, for example, about 1 to about 100 molar equivalents, preferably about 1 to about 10 molar equivalents, relative to 1 mol of the substrate.

The reactive derivative includes, for example, halides (chloride, bromide, iodide, etc.), sulfuric acid esters, or sulfonic acid esters (methanesulfonate, p-toluenesulfonate, benzenesulfonate, etc.) and the like, and particularly halides. The amount of the reactive derivative is, for example, about 1 to 5 molar equivalents, preferably about 1 to 3 molar equivalents, relative to 1 mol of the substrate.

If necessary, the reaction can be promoted by adding an additive. Such additive includes, for example, iodides such as sodium iodide, potassium iodide, etc. and the amount is about 0.1 to 10 molar equivalents, preferably about 0.1 to 5 molar equivalents, relative to 1 mol of the substrate.

The reaction temperature is usually about -10°C to 200°C, preferably about 0°C to 110°C, and the reaction time is usually about 0.5 to 48 hr, preferably about 0.5 to 16 hr.

In addition, if the leaving group L¹ is a hydroxy group in the above-mentioned reactive derivative, the reaction can be also carried out by adding an organic phosphate compound in the presence of a base according to, for example, a method described in JP-A-58-43979 and the like. The organic phosphate compound used herein includes, for example, alkyl o-phenylenephosphate such as methyl phenylenephosphate, ethyl o-phenylenephosphate (EPPA), etc., aryl o-phenylenephosphate such as phenyl o-phenylenephosphate, p-chlorophenyl o-phenylenephosphate, etc. and the like, and particularly EPPA. The base includes, for example, alkylamines such as trimethylamine, triethylamine, diisopropylethylamine, tri(n-butyl)amine, di(n-butyl)amine, diisobutylamine, dicyclohexylamine, etc., cyclic amines such as pyridine, 2,6-lutidine, etc. and the like, and preferably, organic tertiary amines such as diisopropylethylamine, etc. The amounts of the above-mentioned reactive derivative, the base and the organic phosphate compound vary depending on the kinds of compound (IIa) or (IIb), the above-mentioned reactive derivative, the base and the solvent to be used, and further other reaction conditions, but usually about 1 to 10 molar equivalents, preferably about 1 to 5 molar equivalents, respectively to 1 mol of the substrate. The reaction is usually carried out in a solvent inert to the reaction. The solvent includes, for example, halogenated hydrocarbons (dichloromethane, dichloroethane, chloroform, etc.), nitriles (acetonitrile, etc.), esters (ethyl acetate, etc.), ethers (dimethoxyethane, tetrahydrofuran, dioxane, etc.), hydrocarbons (benzene, toluene, etc.), amides (N,N-dimethylformamide, hexamethylphosphoramide, etc.), sulfoxides (dimethyl sulfoxide, etc.), and a mixture thereof, and preferably, halogenated hydrocarbons (dichloromethane, dichloroethane, etc.).

The reaction temperature is for example, about -78°C to 200°C, preferably about -20°C to 150°C. The reaction time varies depending on the kinds of the compound (IIa) or (IIb), the reactive derivative, the base and the solvent to be used, and further other reaction conditions, but for example, about 1 to 72 hr, preferably about 1 to 24 hr.

The reaction using the above-mentioned reactive derivative as an acylating agent depends on the kind of reactive derivative or substrate, but it is usually carried out in a solvent. If necessary, a suitable base may be added to promote the reaction. The solvent includes, for example, hydrocarbons (benzene, toluene, etc.), ethers (diethyl ether, dioxane, tetrahydrofuran, etc.), esters (ethyl acetate, etc.), halogenated hydrocarbons (chloroform, dichloromethane, etc.), esters (ethyl acetate, etc.), amides (N,N-dimethylformamide, etc.), aromatic amines (pyridine, etc.), water and the like, which may be used in a suitable mixture. In addition, the base includes, for example, alkali metal hydroxides (sodium hydroxide, potassium hydroxide, etc.), carbonates (hydrogen carbonates such as sodium hydrogen carbonate, potassium hydrogen carbonate, etc.; sodium carbonate; potassium carbonate, etc.), acetates (sodium acetate, etc.), tertiary amines (trimethylamine, triethylamine, N-methylmorpholine, etc.), aromatic amines (pyridine, picoline, N,N-dimethylaniline, etc.) and the like. The amount of the base is, for example, about 1 to 100 molar equivalents, preferably about 1 to 10 molar equivalents, relative to 1 mol of the substrate.

The acylating agent includes, for example, carboxylic acid, sulfonic acid, phosphoric acid, carbonic acid or a reactive derivative thereof (e.g., acid halide, acid anhydride, mixed acid anhydride, active ester, etc.), isocyanic acid ester, isothiocyanic acid ester and the like.

The amount of such acylating agent is usually about 1 to 10 molar equivalents, preferably about 1 to 3 molar equivalents, relative to 1 mol of the substrate. The reaction temperature is usually about -10°C to 150°C, preferably about 0°C to 100°C, and the reaction time is usually about 15 min to 24 hr, preferably about 30 min to 16 hr.

In addition, compound (I) or a salt thereof can be also produced by reacting compound (IIa) or (IIb) with aldehydes, and reducing the produced imine or iminium ion.

The reaction to produce imine or iminium ion is usually carried out in a solvent that does not adversely affect the reaction. Such solvent includes, for example, aromatic hydrocarbons (toluene, xylene, etc.), aliphatic hydrocarbons (heptane, hexane, etc.), halogenated hydrocarbons (chloroform, dichloromethane, etc.), ethers (diethyl ether, tetrahydrofuran, dioxane, etc.), alcohols (methanol, ethanol, 2-propanol, butanol, benzyl alcohol, etc.), nitriles (acetonitrile, etc.), amides (N,N-dimethylformamide, etc,), sulfoxides (dimethyl sulfoxide, etc.) and the like. Such solvent may be used in a mixture at a suitable ratio. The aldehyde includes, for example, formalin, optionally substituted C₁₋₅ alkyl-aldehyde (e.g., acetaldehyde, etc.), optionally substituted aromatic aldehyde (e.g., benzaldehyde, etc.) and the like, and the amount is, for example, about 1 to 100 molar equivalents, preferably about 1 to 5 molar equivalents, relative to 1 mol of the substrate.

If necessary, the reaction can advantageously proceed by adding a catalyst. Such catalyst includes, for example, mineral acids (hydrochloric acid, hydrobromic acid, sulfuric acid, etc.), carboxylic acids (formic acid, acetic acid, propionic acid, trifluoroacetic acid, etc.), sulfonic acids (methanesulfonic acid, p-toluenesulfonic acid, etc.), Lewis acids (aluminum chloride, zinc chloride, zinc bromide, boron trifluoride, titanium chloride, etc.), acetates (sodium acetate, potassium acetate, etc.) and molecular sieves (molecular sieves 3A, 4A, 5A, etc.). The amount of the catalyst is, for example, about 0.01 to 50 molar equivalents, preferably about 0.1 to 10 molar equivalents, relative to 1 mol of the substrate.

The reaction temperature is usually about 0°C to 200°C, preferably about 20°C to 150°C, and the reaction time is usually about 0.5 to 48 hr, preferably about 0.5 to 24 hr.

The reduction of imine or iminium ion can be carried out by a method of known per se, for example, a method using metal hydride or a method by catalytic hydrogenation.

The metal hydride as the reducing agent includes, for example, metal hydrides (sodium borohydride, lithium borohydride, zinc borohydride, sodium cyanoborohydride, sodium triacetoxyborohydride, lithium cyanoborohydride, dibutylaluminum hydride, aluminum hydride, lithium aluminum hydride, etc.), a borane complex (a borane-tetrahydrofuran complex, catechol borane, etc.) and the like. The metal hydride includes preferably sodium borohydride, sodium cyanoborohydride, sodium triacetoxyborohydride, etc. The amount of the reducing agent is, for example, about 1 to 50 molar equivalents, preferably about 1 to 10 molar equivalents, relative to 1 mol of the substrate. In addition, the reaction solvent includes, for example, aromatic hydrocarbons (toluene, xylene, etc.), aliphatic hydrocarbons (heptane, hexane, etc.), halogenated hydrocarbons (chloroform, dichloromethane, etc.), ethers (diethyl ether, tetrahydrofuran, dioxane, etc.), alcohols (methanol, ethanol, 2-propanol, butanol, benzyl alcohol, etc.), nitriles (acetonitrile, etc.), amides (N,N-dimethylformamide, etc.), sulfoxides (dimethyl sulfoxide, etc.) and the like. Such solvent may be used in a mixture at a suitable ratio. The reaction temperature is usually about -80°C to 80°C, preferably about -40°C to 40°C, and the reaction time is usually about 5 min to 48 hr, preferably about 1 to 24 hr.

The catalytic hydrogenation can be carried out under hydrogen atmosphere and in the presence of a catalyst. The catalyst to be used is preferably palladium compounds (palladium carbon, palladium hydroxide, palladium oxide, etc.), nickel compounds (Raney-nickel, etc.), platinum compounds (platinum oxide, platinum carbon, etc.), rhodium compounds (rhodium acetate, etc.) and the like, and the amount is about 0.001 to 1 equivalent, preferably about 0.01 to 0.5 equivalent. The catalytic hydrogenation proceeds usually in a solvent inert to the reaction. Such solvent includes, for example, alcohols (methanol, ethanol, propanol, butanol, etc.), hydrocarbons (benzene, toluene, xylene, etc.), halogenated hydrocarbons (dichloromethane, chloroform, etc.), ethers (diethyl ether, dioxane, tetrahydrofuran, etc.), esters (ethyl acetate, etc.), amides (N,N-dimethylformamide, etc.), carboxylic acids (acetic acid, etc.), water, or a mixture thereof. The hydrogen pressure under which the reaction proceeds is usually about 1 to 50 atm, preferably about 1 to 10 atm. The reaction temperature is usually about 0°C to 150°C, preferably about 20°C to 100°C, and the reaction time is usually about 5 min to 72 hr, preferably about 0.5 to 40 hr.

Compound (I) can be also produced directly from compound (IIa) or (IIb) in the present process, while carrying out the reaction of producing and of reducing imine or iminium ion at the same time, without isolating the intermediate imine or iminium ion. In this case, pH of the reaction mixture is preferably about 4 to 5.

Compound (IIa) or (IIb) used as the starting compound in Method A can be produced by subjecting compound (Ia) or (Ib) or a salt thereof obtained by Method B, Method C or Method D below to deacylation or dealkylation.

Such deacylation can be carried out according to a known method. For example, it is usually carried out in the presence of an acid or a base, if necessary, in a solvent that does not adversely affect the reaction though it depends on the kinds of the substrate.

The acid is preferably mineral acids (hydrochloric acid, hydrobromic acid, sulfuric acid, etc.), carboxylic acids (acetic acid, trifluoroacetic acid, trichloroacetic acid, etc.), sulfonic acids (methanesulfonic acid, toluenesulfonic acid, etc.), Lewis acids (aluminum chloride, tin chloride, zinc bromide, etc.) and the like. If necessary, it may be used in a mixture of two or more. The amount of the acid varies depending on the kinds of the solvent and other reaction conditions, but it is usually about 0.1 molar equivalents or more, relative to 1 mol of compound (Ia) or (Ib), and the acid can be used as a solvent.

The base is preferably an inorganic base (alkali metal hydroxides such as sodium hydroxide, potassium hydroxide, etc.; alkali metal hydrogen carbonates such as sodium hydrogen carbonate, potassium hydrogen carbonate, etc.; alkali metal carbonates such as sodium carbonate, potassium carbonate, etc.; alkoxides such as sodium methoxide, sodium ethoxide, etc.; and the like), or an organic base (amines such as trimethylamine, triethylamine, diisopropylethylamine, etc.; cyclic amines such as pyridine, 4-dimethylaminopyridine, etc.; and the like) and the like, and preferably, sodium hydroxide, potassium hydroxide, sodium ethoxide and the like.

The amount of the base varies depending on the kinds of the solvent and other reaction conditions, but is usually about 0.1 to 10 molar equivalents, preferably about 0.1 to 5 molar equivalents, relative to 1 mol of compound (Ia) or (Ib).

The solvent that does not adversely affect the reaction includes, for example, alcohols (methanol, ethanol, propanol, 2-propanol, butanol, isobutanol, t-butanol, etc.), aromatic hydrocarbons (benzene, toluene, xylene, etc.), aliphatic hydrocarbons (hexane, heptane, etc.), halogenated hydrocarbons (dichloromethane, chloroform, etc.), ethers (diethyl ether, diisopropyl ether, t-butyl methyl ether, tetrahydrofuran, dioxane, dimethoxyethane, etc.), nitriles (acetonitrile, etc.), esters (ethyl acetate, etc.), carboxylic acids (acetic acid, etc.), amides (N,N-dimethylformamide, etc.), sulfoxides (dimethyl sulfoxide, etc.), water and the like. Such solvent may be used in a mixture of two or more at a suitable ratio.

The reaction temperature is for example, about -50°C to 200°C, preferably about 0°C to 100°C, and the reaction time varies depending on the kinds of compound (Ia) or (Ib) or a salt thereof, the reaction temperature and the like, and it is for example, about 0.5 to 100 hr, preferably about 0.5 to 24 hr.

Dealkylation can be carried out by a known method, for example, the method described in Theodora W. Greene, Peter G. M. Wuts, "Protective Groups in Organic Synthesis, 3rd Ed.," (1999) Wiley-Interscience, and the like, or an analogous method thereto. For example, the dealkylation can be carried out by treatment with an acid, a base, ultraviolet radiation, a transition metal catalyst and the like, or by oxidation, reduction or acylation, followed by hydrolysis, etc., or a combination thereof can be used.

### [Method B]

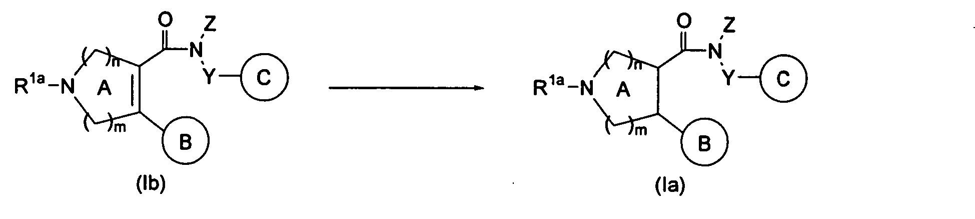

wherein each symbol is as defined above.

A compound represented by compound (Ib) or a salt thereof can be produced by the below-mentioned Method D.

In this reaction, compound (Ib) is converted to compound (Ia) by subjecting the compound to reduction. This reaction can be carried out by a method known *per se*. For example, compound (Ib) is subjected to reduction with a metal and a metal salt, reduction by catalytic hydrogenation using a transition metal catalyst and the like to give compound (Ia).

As the metal and the metal salt to be used for "reduction with a metal and a metal salt", for example, alkali metals (lithium, sodium, potassium, etc.), alkaline earth metals (magnesium, calcium, etc.), other metals (zinc, chromium, titanium, iron, samarium, selenium, etc.), metal salt (zinc-amalgam, zinc-copper alloy, aluminum-amalgam, sodium hydrosulfite, etc.) and the like are preferable. The amount of the reducing agent to be used is, for example, about 1 to 50 molar equivalents, preferably about 1 to 5 molar equivalents, per 1 mol of the substrate.

As the solvent to be used for the reaction, for example, alcohols (methanol, ethanol, 2-propanol, t-butanol, benzyl alcohol, etc.), amines (liquid ammonia, methylamine, ethylamine, ethylenediamine, etc.), ethers (diethyl ether, tetrahydrofuran, dioxane, dimethoxyethane, etc.), mineral acids (hydrochloric acid, hydrobromic acid, sulfuric acid, etc.), carboxylic acids (acetic acid, etc.), amides (hexamethylphosphoramide), water and the like can be mentioned, which can be used alone or in a mixture .

The reaction temperature is generally about -80°C to 150°C, preferably about -80°C to 100°C, and the reaction time is generally about 5 min to 48 hr, preferably about 1 to 24 hr.

As the transition metal catalyst to be used for the "reduction by catalytic hydrogenation using a transition metal catalyst", for example, palladiums (palladium carbon, palladium hydroxide, palladium oxide, etc.), nickels (Raney-nickel, etc.), platinum compounds (platinum oxide, platinum carbon, etc.), rhodium compounds (rhodium acetate, etc.) and the like can be mentioned. The amount thereof to be used is about 0.001 to 1 equivalent, preferably about 0.01 to 0.5 equivalent. The catalytic hydrogenation reaction is generally carried out in a solvent inert to the reaction. As such solvent, for example, alcohols (methanol, ethanol, propanol, butanol, etc.), hydrocarbons (benzene, toluene, xylene, etc.), halogenated hydrocarbons (dichloromethane, chloroform, etc.), ethers (diethyl ether, dioxane, tetrahydrofuran, etc.), esters (ethyl acetate, etc.), amides (N,N-dimethylformamide, etc.), carboxylic acids (acetic acid, etc.), water or a mixture thereof can be used. The hydrogen pressure at which the reaction is carried out is generally about 1 to 50 atm, preferably about 1 to 10 atm. The reaction temperature is generally about 0°C to 150°C, preferably about 20°C to 100°C, and the reaction time is generally about 5 min to 72 hr, preferably about 0.5 to 40 hr.

### [Method C]

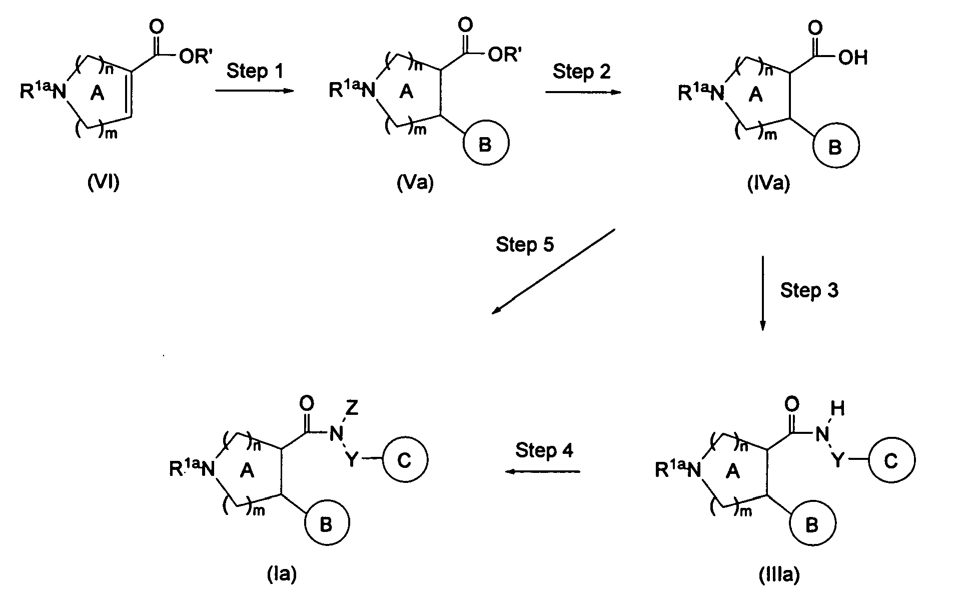

wherein R' is a hydrocarbon group optionally having substituent(s), and other symbols are as defined above.

### (Step 1)

In this step, compound (VI) or a salt thereof and a compound represented by the formula 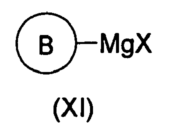 wherein X is a halogen atom, and other symbols are as defined above, or a salt thereof are subjected to addition reaction to give compound (Va).

Compound (VI) or a salt thereof to be the starting material is commercially available, or can be produced by a method known *per se* [e.g., for production of a compound
wherein n=m=1, Journal of Medicinal Chemistry, Vol. 35(2), pp. 233-241 (1992); for production of a compound wherein n=2 and m=1, Journal of the Chemical Society, Perkin Transactions 1: Organic and Bio-Organic Chemistry (1972-1999), Vol. 6, pp. 1754-1762 (1981)].

The Grignard reagent represented by the formula (XI) is commercially available, or can be produced by a method known *per se,* for example, the method described in "4th Ed. Jikken Kagaku Koza (Courses in Experimental Chemistry) 24, Organic Synthesis VI", The Chemical Society of Japan Ed. 1991 and the like, or a method analogous thereto.

In this step, the reaction can be advantageously carried out by the addition of an additive. As such additive, for example, copper salts (e.g., copper chloride, copper bromide, copper iodide, copper cyanide, etc.), lithium salt (e.g., lithium chloride, lithium bromide, lithium iodide, etc.), Lewis acids (e.g., boron trifluoride, trimethylsilyl chloride, aluminum chloride, etc.), Lewis bases (e.g., tributylphosphine, triphenylphosphine, dimethylethylenediamine, etc.) or a mixture thereof and the like can be mentioned. Of these, copper bromide, copper iodide, copper cyanide and the like are preferable. The amount of the additive to be used is about 0.001 to 10 molar equivalents, preferably about 0.1 to 2 molar equivalents, per 1 mol of the Grignard reagent represented by the formula (XI).

This step is carried out in a solvent inert to the reaction. As such solvent, for example, hydrocarbons (hexane, benzene, toluene, xylene, etc.), halogenated hydrocarbons (dichloromethane, chloroform, etc.), ethers (diethyl ether, dioxane, tetrahydrofuran, etc.) or a mixture thereof can be used. The reaction temperature is generally about -80°C to 50°C, preferably about -35°C to 0°C, and the reaction time is generally about 5 min to 48 hr, preferably about 1 to 24 hr.

### (Step 2)

In this step, compound (Va) is subjected to hydrolysis to convert the compound (Va) to compound (IVa). This reaction can be carried out by a method known *per se.* Generally, it is carried out in the presence of an acid or a base, in a solvent that does not adversely affect the reaction, as necessary.

As the acid, for example, mineral acids (hydrochloric acid, hydrobromic acid, sulfuric acid, etc.), carboxylic acids (acetic acid, trifluoroacetic acid, trichloroacetic acid, etc.), sulfonic acids (methanesulfonic acid, toluenesulfonic acid, etc.), Lewis acids (aluminum chloride, tin chloride, zinc bromide, etc.) and the like are used. Where necessary, two or more kinds may be mixed. While the amount of the acid to be used varies depending on the kind of solvent and other reaction conditions, it is generally about 0.1 molar equivalent or more per 1 mol of compound (Va), and the acid can also be used as a solvent.

As the base, for example, inorganic bases (alkali metal hydroxides such as lithium hydroxide, sodium hydroxide, potassium hydroxide, etc.; alkali metal hydrogen carbonates such as sodium hydrogen carbonate, potassium hydrogen carbonate, etc.; alkali metal carbonates such as sodium carbonate, potassium carbonate, etc.; alkoxides such as sodium methoxide, sodium ethoxide, etc.; etc.) or organic bases (amines such as trimethylamine, triethylamine, diisopropylethylamine, etc.; cyclic amines such as pyridine, 4-dimethylaminopyridine, etc.; etc.) and the like are used. Of these, lithium hydroxide, sodium hydroxide, potassium hydroxide, sodium ethoxide and the like are preferable. While the amount of the base to be used varies depending on the kind of solvent and other reaction conditions, it is generally about 0.1 to 10 molar equivalents, preferably about 0.1 to 5 molar equivalents, per 1 mol of compound (Va).

As the solvent that does not adversely affect the reaction, for example, alcohols (methanol, ethanol, propanol, 2-propanol, butanol, isobutanol, t-butanol, etc.), hydrocarbons (benzene, toluene, xylene, hexane, heptane, etc.), halogenated hydrocarbons (dichloromethane, chloroform, etc.), ethers (diethyl ether, diisopropyl ether, t-butyl methyl ether, tetrahydrofuran, dioxane, dimethoxyethane, etc.), nitriles (acetonitrile, etc.), carboxylic acids (acetic acid, etc.), amides (N,N-dimethylformamide, N,N-dimethylacetamide, etc.), sulfoxides (dimethyl sulfoxide, etc.), water and the like can be mentioned. These solvents may be used in a mixture of two or more kinds thereof at an appropriate ratio.

The reaction temperature is, for example, within the range of about -50°C to 200°C, preferably about 0°C to 100°C, and the reaction time varies depending on the kind of compound (Va) or a salt thereof, reaction temperature and the like. For example, it is about 0.5 to 100 hr, preferably about 0.5 to 24 hr.

### (Step 3)

In this reaction step, compound (IVa) or a salt thereof and a compound represented by the formula 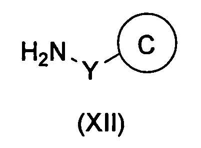 wherein each symbol is as defined above, or a salt thereof, are subjected to dehydrative condensation to give compound (IIIa).

Compound (XII) and a salt thereof are commercially available, or can be produced according to a known method. The amount thereof to be used is about 1 to 10 molar equivalents, preferably about 1 to 2 molar equivalents, per 1 mol of compound (IVa).

As the method for dehydrative condensation, a method known *per se,* for example, the method described in "4th Ed. Jikken Kagaku Koza (Courses in Experimental Chemistry) 22, Organic Synthesis IV", The Chemical Society of Japan Ed. 1991 and the like, or a method analogous thereto can be employed. As such method, for example, a method using a condensing agent, a method via a reactive derivative and the like can be mentioned.

As the condensing agent to be used for "a method using a condensing agent", for example, dicyclohexylcarbodiimide, diisopropylcarbodiimide, N-ethyl-N'-3-dimethylaminopropylcarbodiimide and its hydrochloride, benzotriazol-1-yloxy-tris(dimethylamino)phosphonium hexafluorophosphate, diphenylphosphoryl azide and the like can be mentioned. They may be used alone or in combination with an additive (e.g., N-hydroxysuccinimide, 1-hydroxybenzotriazole, 3-hydroxy-4-oxo-3,4-dihydro-1,2,3-benzotriazine, etc.). The amount of the condensing agent to be used is about 1 to 10 molar equivalents, preferably about 1 to 2 molar equivalents, per 1 mol of compound (IVa). The amount of the additive to be used is about 1 to 10 molar equivalents, preferably about 1 to 2 molar equivalents, per 1 mol of compound (IVa). The above-mentioned reaction is generally carried out in a solvent that does not adversely affect the reaction, and a suitable base may be added to promote the reaction. As the solvent, for example, hydrocarbons (benzene, toluene, etc.), ethers (diethyl ether, dioxane, tetrahydrofuran, etc.), esters (ethyl acetate, etc.), halogenated hydrocarbons (chloroform, dichloromethane, etc.), amides (N,N-dimethylformamide, etc.), aromatic amines (pyridine, etc.), water and the like can be mentioned, which may be appropriately mixed. As the base, for example, alkali metal hydroxides (sodium hydroxide, potassium hydroxide, etc.), hydrogen carbonates (sodium hydrogen carbonate, potassium hydrogen carbonate, etc.), carbonates (sodium carbonate, potassium carbonate, etc.), acetates (sodium acetate, etc.), tertiary amines (trimethylamine, triethylamine, N-methylmorpholine, etc.), aromatic amines (pyridine, picoline, N,N-dimethylaniline, etc.) and the like can be mentioned. The amount of the base to be used is generally about 1 to 100 molar equivalents, preferably about 1 to 5 molar equivalents, per 1 mol of the substrate. The reaction temperature is generally about -80°C to 150°C, preferably about 0°C to 50°C, and the reaction time is generally about 0.5 to 48 hr, preferably about 0.5 to 16 hr.

As the reactive derivative of the "method via a reactive derivative", for example, acid halide, acid anhydride, mixed acid anhydride, active ester and the like can be mentioned. Conversion to a reactive derivative can be performed according to a method known *per se.* For example, for conversion to an acid halide, a method using an acid halide (e.g., thionyl chloride, oxalyl chloride, etc.), a method using a halide of phosphorus and phosphoric acid (e.g., phosphorus trichloride, phosphorus pentachloride, etc.) and the like can be mentioned. The above-mentioned reaction using a reactive derivative is generally carried out in a solvent that does not adversely affect the reaction and a base suitable for promoting the reaction can be added, though subject to change depending on the kind of the reactive derivative or a substrate. The kind and the amount of the solvent and base to be used for the reaction, the reaction temperature and reaction time are the same as those described for the above-mentioned "method using a condensing agent".

### (Step 4)

In this step, compound (IIIa) is reacted with a compound represented by the formula

Z-L² (XIII)

wherein L² is a leaving group and Z is as defined above, or a salt thereof, to give compound (Ia).

As the leaving group for L², for example, a halogen atom (chlorine atom, bromine atom, iodine atom, etc.), a substituted sulfonyloxy group (methanesulfonyloxy group, ethanesulfonyloxy group, benzenesulfonyloxy group, toluenesulfonyloxy group, benzylsulfonyloxy group, etc.), an acyloxy group (acetoxy group, benzoyloxy group, etc.), an oxy group substituted by a heterocycle or an aryl group (succinimide, benzotriazole, quinoline, 4-nitrophenyl, etc.), a heterocycle (imidazole, etc.) and the like are used, and a halogen atom is particularly preferable. The amount of compound (XIII) to be used is, for example, about 1 to 5 molar equivalents, preferably about 1 to 3 molar equivalents, per 1 mol of compound (IIIa).

This reaction can be carried out by reacting compound (IIIa) with compound (XIII) or a salt thereof generally in a solvent in the presence of a base. As the solvent, for example, alcohols (methanol, ethanol, propanol, etc.), ethers (dimethoxyethane, dioxane, tetrahydrofuran, etc.), ketones (acetone, etc.), nitriles (acetonitrile, etc.), amides (N,N-dimethylformamide, etc.), sulfoxides (dimethyl sulfoxide, etc.), water and the like can be mentioned, which may be used in an appropriate mixture. The base includes, for example, organic bases (trimethylamine, triethylamine, N-methylmorpholine, pyridine, picoline, N,N-dimethylaniline, etc.), inorganic bases (potassium carbonate, sodium carbonate, potassium hydroxide, sodium hydroxide, etc.), alkali metal alkoxides (sodium methoxide, sodium ethoxide, sodium tert-butoxide, potassium tert-butoxide, etc.) and the like. The amount of the base to be used is for example, about 1 to 100 molar equivalents, preferably about 1 to 10 molar equivalents, per 1 mol of substrate.

Where necessary, an additive may be added to promote the reaction. As such additive, for example, iodides (sodium iodide, potassium iodide, etc.) and the like can be mentioned. The amount thereof to be used is about 0.1 to 10 molar equivalents, preferably about 0.1 to 5 molar equivalents, per 1 mol of compound (IIIa).

The reaction temperature is generally about -10°C to 200°C, preferably about 0°C to 110°C and the reaction time is generally about 0.5 to 48 hr, preferably about 0.5 to 16 hr.

### (Step 5)

In this reaction step, compound (IVa) or a salt thereof and a compound represented by the formula 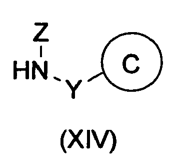 wherein each symbol is as defined above, or a salt thereof, are subjected to dehydrative condensation to give compound (Ia).

Compound (XIV) and a salt thereof are commercially available, or can be produced according to a known method (e.g., WO01/25219, etc.). The amount thereof to be used is about 1 to 5 molar equivalents, preferably about 1 to 2 molar equivalents, per 1 mol of compound (IVa). This reaction can be carried out in the same manner as in the method described in Method C, Step 3.

### [Method D]

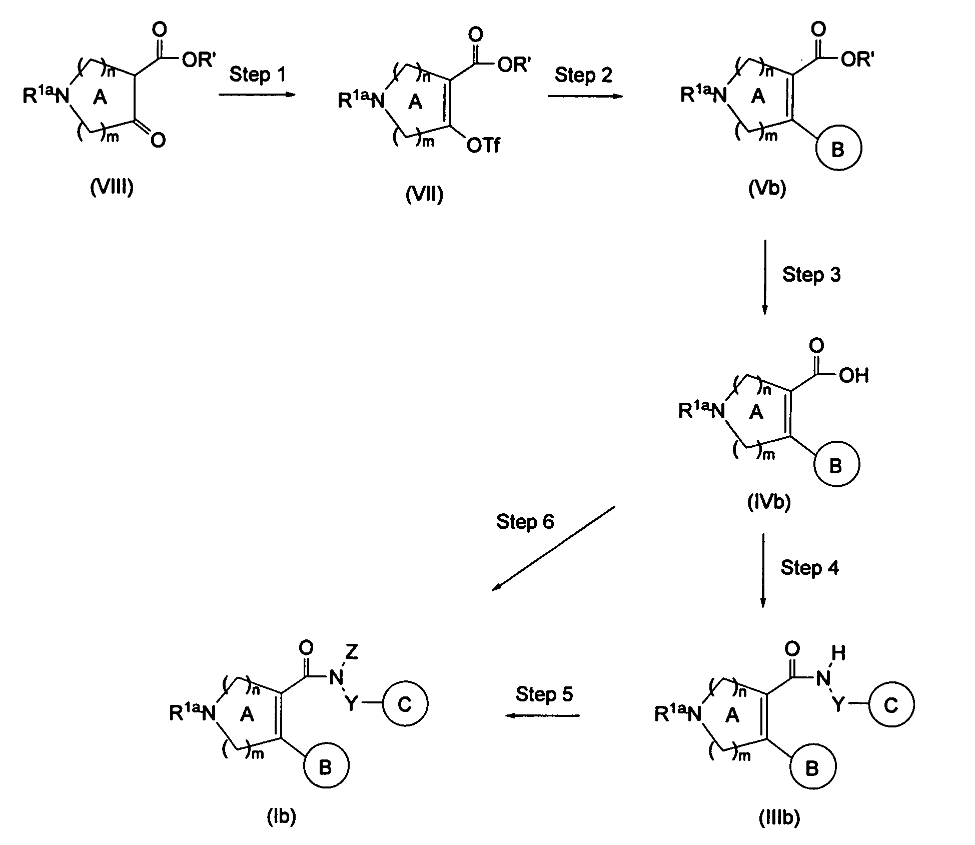

wherein Tf is a trifluoromethanesulfonyl group, and other symbols are as defined above.

### (Step 1)

In this step, compound (VIII) or a salt thereof is triflated to give compound (VII) or a salt thereof.

Compound (VIII) and a salt thereof to be the starting material are commercially available, or can be produced according to a known method [e.g., when a compound wherein n=m=2 is produced, Heterocycles, Vol. 11, pp. 267-273 (1978) and the like].

This step can be performed by a method known per se, for example, the method described in "4th Ed. Jikken Kagaku Koza (Courses in Experimental Chemistry) 24, Organic Synthesis VI", The Chemical Society of Japan Ed. 1991 and the like, or a method analogous thereto. For example, a triflating agent is allowed to react in the presence of a base in a solvent that does not adversely affect the reaction.

As the base to be used, for example, organic amines (trimethylamine, triethylamine, diisopropylamine, N-methylmorpholine, 1,8-diazabicyclo[5.4.0]undec-7-ene, pyridine, N,N-dimethylaniline, etc.), alkali metal salts (sodium hydrogen carbonate, potassium hydrogen carbonate, sodium carbonate, potassium carbonate, cesium carbonate, sodium hydroxide, potassium hydroxide, etc.), metal hydrides (potassium hydride, sodium hydride, etc.) and the like can be mentioned. Of these, organic amines such as triethylamine, diisopropylamine, etc.; metal hydrides such as sodium hydride, etc.; and the like are preferable. The amount of the base to be used is about 0.1 to 10 molar equivalents, preferably about 1 to 5 molar equivalents, per 1 mol of compound (VIII).

The solvent to be used may be any as long as it does not adversely affect the reaction and, for example, hydrocarbons (benzene, toluene, xylene, etc.), halogenated hydrocarbons (chloroform, 1,2-dichloroethane, etc.), esters (ethyl acetate, etc.), nitriles (acetonitrile, etc.), ethers (dimethoxyethane, tetrahydrofuran), aprotic polar solvents (N,N-dimethylformamide, dimethyl sulfoxide, hexamethylphosphoramide, etc.) or a mixture thereof can be used.

As the triflating agent, sulfonic acid anhydrides (e.g., trifluoromethanesulfonic anhydride, etc.), sulfonyl halides (e.g., trifluoromethanesulfonyl chloride, etc.), sulfonimides (e.g., N-phenyl-bis(trifluoromethanesulfonimide), etc.), sulfonic acid esters (e.g., ethyl trifluoromethanesulfonate, etc.) and the like can be mentioned. Of these, sulfonic acid anhydrides such as trifluoromethanesulfonic anhydride and the like, sulfonimides such as N-phenyl-bis(trifluoromethanesulfonimide) and the like are preferable. The amount of the triflating agent to be used is about 0.1 to 10 molar equivalents, preferably about 1 to 5 molar equivalents, per 1 mol of compound (VIII).

The reaction temperature is generally about -80°C to 100°C, preferably about -80°C to 20°C, and the reaction time is generally about 5 min to 48 hr, preferably about 5 min to 8 hr.

### (Step 2)

In this step, compound (VII) or a salt thereof is subjected to a coupling reaction with a compound represented by the formula 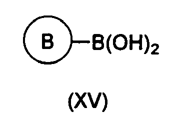 wherein each symbol is as defined above, or a salt thereof to give compound (Vb).

This step can be performed by a method known *per se* [e.g., Chemical Reviews, Vol. 95, p. 2457 (1995) and the like] and, for example, performed in the presence of a transition metal catalyst and a base in a solvent that does not adversely affect the reaction.

As the transition metal catalyst to be used, for example, palladium catalysts (palladium acetate, palladium chloride, tetrakis(triphenylphosphine)palladium, etc.), nickel catalysts (nickel chloride, etc.) and the like are used. Where necessary, ligands (triphenylphosphine, tri-t-butylphosphine, etc.) may be added or metal oxides (copper oxide, silver oxide, etc.) and the like may be used as cocatalysts. While the amount of the catalyst to be used varies depending on the kind of the catalyst, it is generally about 0.0001 to 1 molar equivalent, preferably about 0.01 to 0.5 molar equivalents, per 1 mol of compound (VII). The amount of the ligand to be used is generally about 0.0001 to 4 molar equivalents, preferably about 0.01 to 2 molar equivalents, per 1 mol of compound (VII), and the amount of the cocatalyst to be used is about 0.0001 to 4 molar equivalents, preferably about 0.01 to 2 molar equivalents, per 1 mol of compound (VII).

As the base to be used, for example, organic amines (trimethylamine, triethylamine, diisopropylamine, N-methylmorpholine, 1,8-diazabicyclo[5.4.0]undec-7-ene, pyridine, N,N-dimethylaniline, etc.), alkali metal salts (sodium hydrogen carbonate, potassium hydrogen carbonate, sodium carbonate, potassium carbonate, cesium carbonate, sodium phosphate, potassium phosphate, sodium hydroxide, potassium hydroxide, etc.), metal hydrides (potassium hydride, sodium hydride, etc.), alkali metal alkoxides (sodium methoxide, sodium ethoxide, sodium t-butoxide, potassium t-butoxide, etc.), alkali disilazides (lithium disilazide, sodium disilazide, potassium disilazide, etc.) and the like can be mentioned. Of these, alkali metal salts such as potassium carbonate, cesium carbonate, sodium phosphate, potassium phosphate and the like; alkali metal alkoxides such as sodium t-butoxide, potassium t-butoxide and the like; organic amines such as triethylamine, diisopropylamine and the like; and the like are preferable. The amount of the base to be used is about 0.1 to 10 molar equivalents, preferably about 1 to 5 molar equivalents, per 1 mol of compound (VII).

The solvent to be used may be any as long as it does not adversely affect the reaction and, for example, hydrocarbons (benzene, toluene, xylene, etc.), halogenated hydrocarbons (chloroform, 1,2-dichloroethane, etc.), nitriles (acetonitrile, etc.), ethers (dimethoxyethane, tetrahydrofuran), alcohols (methanol, ethanol, etc.), aprotic polar solvent (N,N-dimethylformamide, dimethyl sulfoxide, hexamethylphosphoramide, etc.), water or a mixture thereof can be used. The reaction temperature is generally about -10°C to 200°C, preferably about 0°C to 150°C, and the reaction time is generally about 0.5 to 48 hr, preferably about 0.5 to 16 hr.

### (Step 3)

In this reaction step, compound (Vb) is subjected to hydrolysis to give compound (IVb). This step can be performed by a method similar to the method described in Method C, Step 2.

### (Step 4)

In this reaction step, compound (IVb) or a salt thereof and a compound represented by the formula 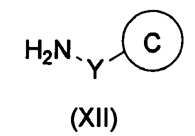 wherein each symbol is as defined above, or a salt thereof, are subjected to dehydrative condensation to give compound (IIIb). This step can be performed by a method similar to the method described in Method C, Step 3.

### (Step 5)

In this reaction step, compound (IIIb) is reacted with a compound represented by the formula

Z-L² (XIII)

wherein each symbol is as defined above, or a salt thereof, to give compound (Ib). This step can be performed by a method similar to the method described in Method C, Step 4.

### (Step 6)

In this reaction step, compound (IVb) or a salt thereof and a compound represented by the formula 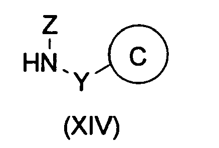 wherein each symbol is as defined above, or a salt thereof are subjected to dehydrative condensation to give compound (Ib). This step can be performed by a method similar to the method described in Method C, Step 5.

In each of the reactions for the synthesis of the objective compounds and the starting materials, when the starting compounds have an amino group, a carboxyl group or a hydroxy group as a substituent, such groups may be protected with the protecting groups which are generally used in peptide chemistry, etc. In such case, if necessary, such protecting groups can be removed to obtain the objective compounds after the reactions.

Such protecting group includes, for example, protecting groups described in "Protective Groups in Organic Synthesis, 3rd Ed. (1999)", edited by Theodora W. Greene, Peter G. M. Wuts, published by Wiley-Interscience.

The protecting group for the amino group includes, for example, a formyl group, a C₁₋₆ alkyl-carbonyl group (an acetyl group, a propionyl group, etc.), a phenylcarbonyl group, a C₁₋₆ alkyl-oxycarbonyl group (a methoxycarbonyl group, an ethoxycarbonyl group, etc.), an aryloxycarbonyl group (a phenyloxycarbonyl group, etc.), a C₇₋₁₀ aralkyl-carbonyl group (a benzyloxycarbonyl group, etc.), a benzyl group, a benzhydryl group, a trityl group, a phthaloyl, etc., each of which may be substituted. Such substituent includes, for example, a halogen atom (a fluorine atom, a chlorine atom, a bromine atom, an iodine atom, etc.), a C₁₋₆ alkyl-carbonyl group (an acetyl group, a propionyl group, a butylcarbonyl group, etc.), a nitro group and the like. The number of substituent is in the order of 1 to 3.

A protecting group for the carboxyl group includes, for example, a C₁₋₆ alkyl group (a methyl group, an ethyl group, an n-propyl group, an isopropyl group, an n-butyl group, a tert-butyl group, etc.), a phenyl group, a trityl group, a silyl group and the like, each of which may be substituted. Such substituent includes, for example, a halogen atom (a fluorine atom, a chlorine atom, a bromine atom, an iodine atom, etc.), a formyl group, a C₁₋₆ alkyl-carbonyl group (an acetyl group, a propionyl group, a butylcarbonyl group, etc.), a nitro group and the like. The number of substituent is in the order of 1 to 3.

The protecting group for the hydroxy group includes, for example, a C₁₋₆ alkyl group (a methyl group, an ethyl group, an n-propyl group, an isopropyl group, an n-butyl group, a tert-butyl group, etc.), a phenyl group, a C₇₋₁₀ aralkyl group (a benzyl group, etc.), a formyl group, a C₁₋₆ alkyl-carbonyl group (an acetyl group, a propionyl group, etc.), an aryloxycarbonyl group (a phenyloxycarbonyl group, etc.), a C₇₋₁₀ aralkyl-carbonyl group (a benzyloxycarbonyl group, etc.), a pyranyl group, a furanyl group, a silyl group and the like, each of which may be substituted. Such substituent includes, for example, a halogen atom (a fluorine atom, a chlorine atom, a bromine atom, an iodine atom, etc.), a C₁₋₆ alkyl group, a phenyl group, a C₇₋₁₀ aralkyl group, a nitro group and the like. The number of substituent is in the order of 1 to 4.

Such protecting groups can be removed by a known deprotection method or the method described in "Protective Groups in Organic Synthesis, 3rd Ed. (1999)", edited by Theodora W. Greene, Peter G. M. Wuts, published by Wiley-Interscience, or an analogous method thereto. For example, treatment with an acid, a base, reduction, ultraviolet radiation, hydrazine, phenylhydrazine, sodium N-methyldithiocarbamate, tetrabutylammonium fluoride, palladium acetate or the like, can be used.

When compound (I) is obtained as a free compound in the above-mentioned method, a salt with for example, inorganic acids (hydrochloric acid, sulfuric acid, hydrobromic acid, etc.), organic acids (methanesulfonic acid, benzenesulfonic acid, toluenesulfonic acid, oxalic acid, fumaric acid, maleic acid, tartaric acid, etc.), inorganic bases (alkali metals such as sodium, potassium, etc., alkaline earth metals such as calcium, magnesium, etc., aluminum, ammonium, etc.), or organic bases (trimethylamine, triethylamine, pyridine, picoline, ethanolamine, diethanolamine, triethanolamine, dicyclohexylamine, N,N'-dibenzylethylenediamine, etc.) and the like can be produced in a routine manner. When compound (I) is obtained in the form of a salt, the compound can be converted to a free compound or another salt in a routine manner.

In addition, when the starting compound forms a salt in each of the above-mentioned reactions, the compound may be used as a salt. Such salt includes, for example, those exemplified as a salt of compound (I).

Compound (I) of the present invention thus produced by such method, can be isolated and purified by a typical separation means such as recrystallization, distillation, chromatography, etc.

When compound (I) contains an optical isomer, a stereoisomer, a regioisomer or a rotamer, these are also encompassed in compound (I), and can be obtained as a single product according to synthesis and separation methods known *per se* (concentration, solvent extraction, column chromatography, recrystallization, etc.). For example, when compound (I) has an optical isomer, an optical isomer resolved from this compound is also encompassed in compound (I).

The optical isomer can be produced by a method known per se. To be specific, an optically active synthetic intermediate is used, or the final racemate product is subjected to optical resolution according to a conventional method to give an optical isomer.

The method of optical resolution may be a method known per se, such as a fractional recrystallization method, a chiral column method, a diastereomer method, etc.

### 1) Fractional recrystallization method

A method wherein a salt of a racemate with an optically active compound (e.g., (+)-mandelic acid, (-)-mandelic acid, (+)-tartaric acid, (-)-tartaric acid, (+)-1-phenethylamine, (-)-1-phenethylamine, cinchonine, (-)-cinchonidine, brucine, etc.) is formed, which is separated by a fractional recrystallization method, and if desired, a free optical isomer is obtained by a neutralization step.

### 2) Chiral column method

A method wherein a racemate or a salt thereof is applied to a column for separation of an optical isomer (a chiral column) to allow separation. In the case of a liquid chromatography, for example, a mixture of the optical isomers is applied to a chiral column such as ENANTIO-OVM (manufactured by Tosoh Corporation), CHIRAL series (manufactured by Daicel Chemical Industries, Ltd.) and the like, and developed with water, various buffers (phosphate buffer, etc.) and organic solvents (ethanol, methanol, isopropanol, acetonitrile, trifluoroacetic acid, diethylamine, etc.) solely or in admixture to separate the optical isomer. In the case of a gas chromatography, for example, a chiral column such as CP-Chirasil-DeX CB (manufactured by GL Sciences Inc.) and the like is used to allow separation.

### 3) Diastereomer method

A method wherein a racemic mixture is prepared into a diastereomeric mixture by chemical reaction with an optically active reagent, which is made into a single substance by a typical separation means (a fractional recrystallization method, a chromatography method, etc.) and the like, and is subjected to a chemical treatment such as hydrolysis and the like to separate an optically active reagent moiety, whereby an optical isomer is obtained. For example, when compound (I) contains hydroxy, or primary or secondary amino in a molecule, the compound and an optically active organic acid (MTPA [α-methoxy-α-(trifluoromethyl)phenylacetic acid], (-)-menthoxyacetic acid, etc.) and the like are subjected to condensation reaction to give diastereomers in the ester form or in the amide form, respectively. When compound (I) has a carboxylic acid group, this compound and an optically active amine or an alcohol reagent are subjected to condensation reaction to give diastereomers in the amide form or in the ester form, respectively. The separated diastereomer is converted to an optical isomer of the original compound by acid hydrolysis or base hydrolysis.

Compound (I) or a salt thereof may be in the form of a crystal.

The crystal of compound (I) or a salt thereof (hereinafter, it may be referred to as crystal of the present invention) can be produced by crystallization of compound (I) or a salt thereof by a crystallization method known per se.

Examples of the crystallization method include a method of crystallization from a solution, a method of crystallization from vapor, a method of crystallization from the melts and the like.

The "crystallization from a solution" is typically a method of shifting a non-saturated state to supersaturated state by varying factors involved in solubility of compounds (solvent composition, pH, temperature, ionic strength, redox state, etc.) or the amount of solvent. To be specific, for example, a concentration method, a cooling method, a reaction method (a diffusion method, an electrolysis method), a hydrothermal growth method, a flux method and the like can be mentioned. Examples of the solvent to be used include aromatic hydrocarbons (benzene, toluene, xylene, etc.), halogenated hydrocarbons (dichloromethane, chloroform, etc.), saturated hydrocarbons (hexane, heptane, cyclohexane, etc.), ethers (diethyl ether, diisopropyl ether, tetrahydrofuran, dioxane, etc.), nitriles (acetonitrile, etc.), ketones (acetone, etc.), sulfoxides (dimethyl sulfoxide, etc.), amides (N,N-dimethylformamide, etc.), esters (ethyl acetate, etc.), alcohols (methanol, ethanol, isopropyl alcohol, etc.), water and the like. These solvents are used alone or in a combination of two or more at a suitable ratio (e.g., 1:1 to 1:100 (a volume ratio)).

The "crystallization from vapor" is, for example, a vaporization method (a sealed tube method, a gas stream method), a gas phase reaction method, a chemical transportation method and the like.

The "crystallization from the melts" is, for example, a normal freezing method (a Czockralski method, a temperature gradient method and a Bridgman method, etc.), a zone melting method (a zone leveling method and a floating zone method, etc.), a special growth method (a VLS method and a liquid phase epitaxy method, etc.) and the like.

Preferable examples of the crystallization method include a method of dissolving compound (I) or a salt thereof in a suitable solvent (e.g., alcohols such as methanol, ethanol, etc., etc.) at a temperature of 20 to 120°C, and cooling the resulting solution to a temperature not higher than the temperature of dissolution (e.g., 0 to 50°C, preferably 0 to 20°C) and the like.

The thus obtained crystals of the present invention can be isolated, for example, by filtration and the like.

In the present specification, the melting point means that measured using, for example, a micromelting point apparatus (Yanako, MP-500D) or a DSC (differential scanning calorimetry) device (SEIKO, EXSTAR6000) and the like.

In the present specification, the peak by a powder X-ray diffraction means that measured using, for example, RINT2100 (Rigaku Corporation), etc. with a Cu-Kα1 ray (tube voltage: 40 KV; tube current: 50 mA) as a ray source.

In the present specification, moreover, the specific rotation ([α]_{D}) means, for example, a specific rotation measured using a polarimeter (JASCO, P-1030 polarimeter (No. AP-2)) and the like.

In general, the melting points and the peak by a powder X-ray diffraction vary depending on the measurement apparatuses, the measurement conditions and the like. The crystal in the present specification may show different values from the melting point described in the present specification or the peak by a powder X-ray diffraction vary depending on the measurement apparatuses, as long as they are within each of a general error range.

The crystal of the present invention is superior in physicochemical properties (melting point, solubility, stability, etc.) and biological properties (pharmacokinetics (absorption, distribution, metabolism, excretion), efficacy expression, etc.), and thus it is extremely useful as a medicament.

Compound (I) or a salt thereof or a prodrug of the present invention (hereinafter, it may be briefly referred to as the compound of the present invention) has excellent antagonistic action for a tachykinin receptor, particularly substance P receptor antagonistic action including inhibitory action for the increased permeability of blood vessel of a trachea induced by capsaicin, neurokinin A receptor antagonistic action. The compound of the present invention has low toxicity and thus it is safe.

Accordingly, the compound of the present invention having excellent antagonistic action for substance P receptors and neurokinin A receptors, etc. can be used as a safe medicine for preventing and treating the following diseases related to substance P in mammals (e.g., mice, rats, hamsters, rabbits, cats, dogs, bovines, sheep, monkeys, humans, etc.).
(1) Abnormality of lower urinary tract functions [for example, an overactive bladder, interstitial cystitis, abnormal micturition (e.g., urinary frequency, urinary incontinence, urgency due to overactive bladder, detrusor underactivity associated with overactive bladder, etc.), a pelvic visceral pain, etc.]
(2) Digestive organ diseases [for example, an irritable bowel syndrome, ulcerative colitis, Crohn's disease, diseases caused by a spiral urease-positive gram-negative bacterium (e.g., Helicobacter pylori, etc.) (e.g., gastritis, gastric ulcer, etc.), gastric cancer, postgastrostomy disorder, indigestion, esophageal ulcer, pancreatitis, polyp of the colon, cholelithiasis, hemorrhoids, peptic ulcer, situational ileitis, vomiting, nausea, NUD (non-ulcer dyspepsia), etc.]
(3) Inflammatory or allergic diseases [for example, inflammatory bowel disease, allergic rhinitis, conjunctivitis, gastrointestinal allergy, pollinosis, anaphylaxis, dermatitis, herpes, psoriasis, bronchitis, expectoration, retinopathy, postoperative and posttraumatic inflammation, regression of puffiness, pharyngitis, cystitis, meningitidis, inflammatory ophthalmic diseases, etc.]
(4) Osteoarthropathy diseases [for example, rheumatoid arthritis (chronic rheumatoid arthritis), arthritis deformans, rheumatoid myelitis, osteoporosis, abnormal growth of cells, bone fracture, bone refracture, osteomalacia, osteopenia, osseous Behcet's disease, rigid myelitis, articular tissue destruction by gonarthrosis deformans and similar diseases thereto, etc.]
(5) Respiratory diseases [for example, cold syndrome, pneumonia, asthma, pulmonary hypertension, pulmonary thrombi/pulmonary obliteration, pulmonary sarcoidosis, pulmonary tuberculosis, interstitial pneumonia, silicosis, adult respiratory distress syndrome, chronic obstructive pulmonary diseases, cough, etc.]
(6) Infectious diseases [HIV infectious diseases, virus infectious diseases due to cytomegalo virus, influenza virus, herpes virus and the like, rickettsia infectious diseases, bacterial infectious diseases, sexually-transmitted diseases, carinii pneumonia, helicobacter pylori infectious disease, systemic fungal infectious diseases, tuberculosis, invasive staphylococcal infectious diseases, acute viral encephalitis, acute bacterial meningitidis, AIDS encephalitis, septicemia, sepsis, sepsis gravis, septic shock, endotoxin shock, toxic shock syndromes, etc.]
(7) Cancers [for example, primary, metastatic or recurrent breast cancer, prostatic cancer, pancreatic cancer, gastric cancer, lung cancer, colorectal cancer (colon cancer, rectal cancer, anal cancer), esophagus cancer, duodenal cancer, head and neck cancer (tongue cancer, pharynx cancer, larynx cancer), brain tumor, neurinoma, non-small cell lung cancer, small cell lung cancer, hepatic cancer, renal cancer, colic cancer, uterine cancer (cancer of the uterine body, uterine cervical cancer), ovarian cancer, bladder cancer, skin cancer, hemangioma, malignant lymphoma, malignant melanoma, thyroid cancer, bone tumor, hemangioma, angiofibroma, retinosarcoma, penis cancer, pediatric solid cancer, Kaposi's sarcoma, Kaposi's sarcoma caused by acquired immunodeficiency syndrome (AIDS), tumor of the maxillary sinus, fibrous histiocytoma, smooth muscle sarcoma, rhabdomyosarcoma, liposarcoma, fibroid tumors of the uterus, osteoblastoma, osteosarcoma, chondrosarcoma, carcinomatous mesothelial tumor, tumors such as leukemia, Hodgkin's disease, etc.]
(8) Central nerve diseases [for example, neurodegenerative diseases (e.g., Alzheimer's disease, Down's disease, Parkinson's disease, Creutzfeldt-Jakob's disease, amyotrophic lateral sclerosis (ALS), Huntington chorea, diabetic neuropathy, multiple sclerosis, etc.), mental diseases (e.g., schizophrenia, depression, mania, anxiety neurosis, obsessive-compulsive neurosis, panic disorder, epilepsy, alcohol dependence, an anxiety symptom, anxious mental state, etc.), central and peripheral nerve disorders (e.g., head trauma, spinal cord injury, brain edema, disorders of sensory function, abnormality of sensory function, disorders of autonomic nervous function and abnormality of autonomic nervous function, whiplash injury, etc.), memory disorders (e.g., senile dementia, amnesia, cerebrovascular dementia, etc.), cerebrovascular disorders (e.g., disorders and aftereffect and/or complication from intracerebral hemorrhage, brain infarction, etc., asymptomatic cerebro-vascular accident, transient cerebral ischemic attack, hypertensive encephalopathia, blood-brain barrier disorder, etc.), recurrence and aftereffect of cerebro-vascular accident (neural symptoms, mental symptoms, subjective symptoms, disorders of daily living activities, etc.), post-cerebrovascular occlusion central hypofunction; disorder or abnormality of autoregulation of cerebral circulation and/or renal circulation]
(9) Circulatory diseases [for example, acute coronary artery syndromes (e.g., acute cardiac infarction, unstable angina, etc.), peripheral arterial obstruction, Raynaud's disease; Buerger disease; restenosis after coronary-artery intervention (percutaneous transluminal coronary angioplasty (PTCA), directional coronary atherectomy (DCA), stenting, etc.), restenosis after coronary-artery bypass operation, restenosis after intervention (angioplasty, atherectomy, stenting, etc.) or bypass operation in other peripheral artery, ischemic cardiac diseases (e.g., cardiac infarction, angina, etc.), myocarditis, intermittent claudication, lacunar infarction, arteriosclerosis (e.g., atherosclerosis, etc.), cardiac failure (acute cardiac failure, chronic cardiac failure accompanied by congestion), arrhythmia, progress of atherosclerotic plaque, thrombosis, hypertension, hypertensive tinnitus; hypotension, etc.]
(10) Pains [e.g., migraine, neuralgia, etc.]
(11) Autoimmune diseases [for example, collagen disease, systemic lupus erythematosus, scleroderma, polyarteritis, myasthenia gravis, multiple sclerosis, Sjogren's syndrome, Behcet's disease, etc.]
(12) Hepatic diseases [e.g., hepatitis (including chronic hepatitis), cirrhosis, interstitial hepatic diseases, etc.]
(13) Pancreatic diseases [e.g., pancreatitis (including chronic pancreatitis), etc.]
(14) Renal diseases [e.g., nephritis, glomerulonephritis, glomerulosclerosis, renal failure, thrombotic microangiopathy, dialysis complications, organ disorders including nephropathia by radiation, diabetic nephropathia, etc.]
(15) Metabolic diseases [e.g., diabetic diseases (insulin-dependent diabetes, diabetic complications, diabetic retinopathy, diabetic microangiopathy, diabetic neuropathy, etc.); glucose tolerance abnormality, obesity, benign prostatic hypertrophy, sexual dysfunction, etc.]
(16) Endocrine diseases [e.g., Addison's disease, Cushing's syndrome, melanocytoma, primary aldosteronism, etc.]
(17) Other diseases
   (i) Transplant rejection [e.g., posttransplantational rejection, posttransplantational polycythemia, hypertension, organ disorder and/or vascular hypertrophy, graft-versus-host disease, etc.]
   (ii) Abnormality in characteristic of blood and/or blood components [e.g., enhancement in platelet aggregation, abnormality of erythrocyte deformability, enhancement in leukocyte adhesiveness, increase in blood viscosity, polycythemia, vascular peliosis, autoimmune hemolytic anemia, disseminated intravascular coagulation syndrome (DIC), multiple myelopathy, etc.]
   (iii) Gynecologic diseases [e.g., climacteric disorder, gestational toxicosis, endometriosis, hysteromyoma, ovarian disease, mammary disease, etc.]
   (iv) Dermatic diseases [e.g., keloid, angioma, psoriasis, pruritus, etc.]
   (v) Ophthalmic diseases [e.g., glaucoma, ocular hypertension disease, etc.]
   (vi) Otolaryngological diseases [e.g., Menuel syndrome, tinnitus, gustation disorder, dizziness, disequilibrium, dysphagia, etc.]
   (vii) Diseases due to environmental and/or occupational factors (e.g., radiation disorder, disorders by ultraviolet ray-infrared ray-laser ray, altitude sickness, etc.)
   (viii) Ataxia
   (ix) Chronic fatigue syndrome

Particularly, the compounds of the present invention are useful as tachykinin receptor antagonists and agents for treating lower urinary tract dysfunctions such as urinary frequency, urinary incontinence, etc., and agents for treating digestive organ diseases such as irritable bowel disease, ulcerative colitis and the like.

Pharmaceutical preparations comprising compound of the present invention may be in any solid forms of powders, granules, tablets, capsules, suppositories, etc., and in any liquid forms of syrups, emulsions, injections, suspensions, etc.

The pharmaceutical preparations of the present invention can be produced by any conventional methods, for example, blending, kneading, granulation, tabletting, coating, sterilization, emulsification, etc., in accordance with the forms of the preparations to be produced. For the production of such pharmaceutical preparations, for example, each of the items in General Rules for Preparations in the Japanese Pharmacopoeia, can be made reference to. In addition, the pharmaceutical preparations of the present invention may be formulated into a sustained release preparation containing active ingredients and biodegradable polymer compounds. The sustained release preparation can be produced according to the method described in JP-A-9-263545.

In the pharmaceutical preparations of the present invention, the content of the compound of the present invention or a salt thereof varies depending on the forms of the preparations, but is generally in the order of about 0.01 to 100 % by weight, preferably about 0.1 to 50 % by weight, more preferably 0.5 to 20 % by weight, relative to the total weight of each preparation.

When the compound of the present invention is used in the above-mentioned pharmaceutical preparations, it may be used alone, or in admixture with a suitable, pharmaceutically acceptable carrier, for example, excipients (e.g., starch, lactose, sucrose, calcium carbonate, calcium phosphate, etc.), binders (e.g., starch, arabic gum, carboxymethyl cellulose, hydroxypropyl cellulose, crystalline cellulose, alginic acid, gelatin, polyvinyl pyrrolidone, etc.), lubricants (e.g., stearic acid, magnesium stearate, calcium stearate, talc, etc.), disintegrants (e.g., calcium carboxymethylcellulose, talc, etc.), diluents (e.g., water for injection, physiological saline, etc.) and if desired, with the additives (e.g., a stabilizer, a preservative, a colorant, a fragrance, a dissolution aid, an emulsifier, a buffer, an isotonic agent, etc.) and the like, by ordinary methods. It can be formulated into the solid preparations such as powders, fine granules, granules, tablets, capsules, etc., or into the liquid preparations such as injections, etc., and can be administered non-parenterally or parenterally.

The dose of the pharmaceutical preparation of the present invention varies depending on the kinds of the compound of the present invention or a pharmaceutically acceptable salt thereof, the administration route, the condition and the age of patients, etc. For example, the dose for oral administration of the pharmaceutical preparation to an adult patient suffering from abnormal micturition is generally from about 0.005 to 50 mg/kg body/day, preferably from about 0.05 to 10 mg/kg body/day, more preferably from about 0.2 to 4 mg/kg body/day, in terms of the compound of the present invention, which may be administered once a day or in two or three divided portions a day.

The dose when the pharmaceutical composition of the present invention is a sustained release preparation varies depending on the kinds and the content of compound (I) or a salt thereof, the formulation, the duration time of drug release, the animals to be administered (e.g., mammals such as humans, rats, mice, cats, dogs, rabbits, bovines, pigs, etc.), and the purpose of administration. For example, when it is applied by parenteral administration, preferably about 0.1 to about 100 mg of compound (I) or a salt thereof is released from the preparation for 1 week.

The compound of the present invention can be used in a mixture or combination with other pharmaceutically active ingredients at a suitable ratio.

Combination of the compound of the present invention with other pharmaceutically active ingredients can give the following excellent effects:
(1) a dose can be reduced as compared with separate administration of the compound of the present invention or other pharmaceutically active ingredients. More specifically, when the compound of the present invention is combined with anticholinergic agents or NK-2 receptor antagonists, the dose can be reduced as compared with separate administration of anticholinergic agents or NK-2 receptor antagonists, and therefore, side effects such as dry mouth can be reduced;
(2) according to symptoms of patient (mild symptoms, severe symptoms, etc.), a drug to be combined with the compound of the present invention can be selected;
(3) by choosing other pharmaceutically active ingredients which have different mechanism of action from that of the compound of the present invention, the therapeutic period can be designed longer;
(4) by choosing other pharmaceutically active ingredients which have different mechanism of action from that of the compound of the present invention, continuation of therapeutic effects can be obtained; and
(5) by combining the compound of the present invention and other pharmaceutically active ingredients, excellent effects such as synergic effects can be obtained.

A drug which is mixed or combined with the compound of the present invention (hereinafter, briefly referred to as combination drugs) includes the following:
(1) Agent for treating diabetes
   Insulin preparations (e.g., animal insulin preparations extracted from the bovine or swine pancreas; human insulin preparations synthesized by a genetic engineering technique using Escherichia coli or a yeast; insulin zinc; protamine zinc insulin; a fragment or a derivative of insulin (e.g., INS-1, etc.), insulin sensitizers (e.g., pioglitazone hydrochloride, troglitazone, rosiglitazone or its maleate, JTT-501, MCC-555, YM-440, GI-262570, KRP-297, FK-614, CS-011, etc.), α-glucosidase inhibitors (e.g., voglibose, acarbose, miglitol, emiglitate, etc.), biguanides (e.g., phenformin, metformin, buformin, etc.), sulfonylureas (e.g., tolbutamide, glibenclamide, gliclazide, chlorpropamide, tolazamide, acetohexamide, glyclopyramide, glimepiride, etc.) and other insulin secretagogues (e.g., repaglinide, senaglinide, mitiglinide or its calcium salt hydrate, GLP-1, nateglinide, etc.), dipeptidylpeptidase IV inhibitors (e.g., NVP-DPP-278, PT-100, P32/98, etc.), β₃ agonists (e.g., CL-316243, SR-58611-A, UL-TG-307, AJ-9677, AZ40140, etc.), amylin agonists (e.g., pramlintide, etc.), phosphotyrosine phosphatase inhibitors (e.g., vanadic acid, etc.), gluconeogenesis inhibitors (e.g., glycogen phosphorylase inhibitors, glucose-6-phosphatase inhibitors, glucagon antagonists, etc.), SGLT (sodium-glucose cotransporter) inhibitors (e.g., T-1095, etc.) and the like.
(2) Agent for treating diabetic complications
   Aldose reductase inhibitors (e.g., tolrestat, epalrestat, zenarestat, zopolrestat, fidarestat (SNK-860), minalrestat (ARI-509), CT-112, etc.), neurotrophic factors (e.g., NGF, NT-3, etc.), AGE inhibitors (e.g., ALT-945, pimagedine, pyratoxathine, N-phenacylthiazolium bromide (ALT-766), EXO-226, etc.), active oxygen scavengers (e.g., thioctic acid, etc.), cerebral vasodilators (e.g., tiapuride, etc.) and the like.
(3) Antihyperlipidemic agent
   Statin compounds inhibiting cholesterol synthesis (e.g., pravastatin, simvastatin, lovastatin, atorvastatin, fluvastatin, cerivastatin or their salt (e.g., sodium salt, etc.), etc.), squalene synthase inhibitors or fibrate compounds having triglyceride lowering action (e.g., bezafibrate, clofibrate, simfibrate, clinofibrate, etc.) and the like.
(4) Hypotensive agent
   Angiotensin converting enzyme inhibitors (e.g., captopril, enalapril, delapril, etc.), angiotensin II antagonists (e.g., losartan, candesartan cilexetil, etc.), calcium antagonists (e.g., manidipine, nifedipine, amlodipine, efonidipine, nicardipine, etc.), clonidine, and the like.
(5) Antiobesity agent
   Antiobesity drugs acting on the central nervous system (e.g. dexfenfluramine, fenfluramine, phentermine, sibutramine, anfepramone, dexamphetamine, mazindol, phenylpropanolamine, clobenzorex, etc.), pancreatic lipase inhibitors (e.g. orlistat, etc.), β₃ agonists (e.g. CL-316243, SR-58611-A, UL-TG-307, AJ-9677, AZ40140, etc.), anorectic peptides (e.g. leptin, CNTF (Ciliary Neurotrophic Factor), etc.), cholecystokinin agonists (e.g. lintitript, FPL-15849, etc.).
(6) Diuretic agent
   Xanthine derivatives (e.g., theobromine sodium salicylate, theobromine calcium salicylate, etc.), thiazide preparations (e.g., ethiazide, cyclopenthiazide, trichlormethiazide, hydrochlorothiazide, hydroflumethiazide, benzylhydrochlorothiazide, penflutizide, polythiazide, methyclothiazide, etc.), antialdosterone preparations (e.g., spironolactone, triamterene, etc.), carbonic anhydrase inhibitors (e.g., acetazolamide, etc.), chlorobenzenesulfonamide preparations (e.g., chlorthalidone, mefruside, indapamide, etc.), azosemide, isosorbide, ethacrynic acid, piretanide, bumetanide, furosemide, etc.
(7) Chemotherapeutic agent
   Alkylating agents (e.g., cyclophosphamide, ifosamide, etc.), metabolic antagonists (e.g., methotrexate, 5-fluorouracil, etc.), antitumor antibiotics (e.g., mitomycin, adriamycin, etc.), plant-derived antitumor agents (e.g., vincristine, vindesine, taxol, etc.), cisplatin, carboplatin, etoposide, etc. Among these, 5-fluorouracil derivatives such as Furtulon and Neo-Furtulon are preferred.
(8) Immunotherapeutic agent
   Microorganism- or bacterium-derived components (e.g., muramyl dipeptide derivatives, Picibanil, etc.), immunopotentiator polysaccharides (e.g., lentinan, schizophyllan, krestin, etc.), genetically engineered cytokines (e.g., interferons, interleukins (IL), etc.), colony stimulating factors (e.g., granulocyte colony stimulating factor, erythropoietin, etc.) and the like. Among these, IL-1, IL-2, IL-12, etc. are preferred.
(9) Therapeutic agent recognized to ameliorate cachexia in animal models or clinical practice
   Progesterone derivatives (e.g., megestrol acetate) [Journal of Clinical Oncology, vol. 12, pp. 213-225, 1994], metoclopramide pharmaceuticals, tetrahydrocannabinol pharmaceuticals (the above references are applied to both), fat metabolism ameliorating agents (e.g., eicosapentanoic acid) [British Journal of Cancer, vol. 68, pp. 314-318, 1993], growth hormones, IGF-1, and antibodies to the cachexia-inducing factors such as TNF-α, LIF, IL-6 and oncostatin M.
(10) Antiinflammatory agent
   Steroids (e.g., dexamethasone, etc.), sodium hyaluronate, cyclooxygenase inhibitors (e.g., indomethacin, ketoprofen, loxoprofen, meloxicam, ampiroxicam, celecoxib, rofecoxib, etc.) and the like.
(11) Miscellaneous

Glycosylation inhibitors (e.g., ALT-711, etc.), nerve regeneration promoting drugs (e.g., Y-128, VX853, prosaptide, etc.), drugs acting on the central nervous system (e.g., antidepressants such as desipramine, amitriptyline, imipramine, fluoxetine, paroxetine, doxepin, etc.), anticonvulsants (e.g., lamotrigine, carbamazepine), antiarrhythmic drugs (e.g., mexiletine), acetylcholine receptor ligands (e.g., ABT-594), endothelin receptor antagonists (e.g., ABT-627), monoamine uptake inhibitors (e.g., tramadol), indoleamine uptake inhibitors (e.g., fluoxetine, paroxetine), narcotic analgesics (e.g., morphine), opioid receptor complete agonist (e.g., pentazocine), opioid receptor partial agonist (e.g., buprenorphine, axomadol, TRK-130), γ-aminobutyric acid (GABA) receptor agonists, GABA uptake inhibitors (e.g., tiagabine), α₂ receptor agonists (e.g., clonidine), local analgesics (e.g., capsaicin), protein kinase C inhibitors (e.g., LY-333531), antianxiety drugs (e.g., benzodiazepines), phosphodiesterase inhibitors (e.g., sildenafil), dopamine receptor agonists (e.g., apomorphine), dopamine receptor antagonists (e.g., haloperidol), serotonin receptor agonists (e.g., tandospirone citrate, sumatryptan), serotonin receptor antagonists (e.g., cyproheptadine hydrochloride, ondansetron), serotonin uptake inhibitors (e.g., fluvoxamine maleate, fluoxetine, paroxetine), serotonin noradrenaline uptake inhibitors (e.g., duloxetine, venlafaxine), hypnotics (e.g., triazolam, zolpidem), anticholinergic agents, α₁ receptor blocking agents (e.g., tamsulosin, alfuzosin, silodosin), muscle relaxants (e.g., baclofen, etc.), potassium channel openers (e.g., nicorandil), calcium channel blocking agents (e.g., nifedipine, gabapentin), agents for preventing and/or treating Alzheimer's disease (e.g., donepezil, rivastigmine, galanthamine), agents for treating Parkinson's disease (e.g., L-dopa), agents for preventing and/or treating multiple sclerosis (e.g., interferon β-1a), histamine H₁ receptor inhibitors (e.g., promethazine hydrochloride), proton pump inhibitors (e.g., lansoprazole, omeprazole), antithrombotic agents (e.g., aspirin, cilostazol), NK-2 receptor antagonists, agents of treating HIV infection (saquinavir, zidovudine, lamivudine, nevirapine), agents of treating chronic obstructive pulmonary diseases (salmeterol, thiotropium bromide, cilomilast), etc.

Anticholinergic agents include, for example, atropine, scopolamine, homatropine, tropicamide, cyclopentolate, butylscopolamine bromide, propantheline bromide, methylbenactyzium bromide, mepenzolate bromide, flavoxate, pirenzepine, ipratropium bromide, trihexyphenidyl, oxybutynin, propiverine, darifenacin, tolterodine, temiverine, trospium chloride or a salt thereof (e.g., atropine sulfate, scopolamine hydrogen bromide, homatropine hydrogen bromide, cyclopentolate hydrochloride, flavoxate hydrochloride, pirenzepine hydrochloride, trihexyphenidyl hydrochloride, oxybutynin chloride, tolterodine tartrate, etc.), preferably, oxybutynin, propiverine, darifenacin, tolterodine, temiverine, trospium chloride or a salt thereof (e.g., oxybutynin chloride, tolterodine tartrate, etc.). In addition, acetylcholinesterase inhibitors (e.g., distigmine, etc.) and the like can be used.

NK-2 receptor antagonists include, for example, a piperidine derivative such as GR159897, GR149861, SR48968 (saredutant), SR144190, YM35375, YM38336, ZD7944, L-743986, MDL105212A, ZD6021, MDL105172A, SCH205528, SCH62373, R-113281, etc., a perhydroisoindole derivative such as RPR-106145, etc., a quinoline derivative such as SB-414240, etc., a pyrrolopyrimidine derivative such as ZM-253270, etc., a pseudopeptide derivative such as MEN11420 (nepadutant), SCH217048, L-659877, PD-147714 (CAM-2291), MEN10376, S16474, etc., and others such as GR100679, DNK333, GR94800, UK-224671, MEN10376, MEN10627, or a salt thereof, and the like.

The pharmaceutical composition comprising a mixture or combination of the compound of the present invention and the combination drugs may be formulated into
(1) a single formulation as a pharmaceutical composition containing the compound of the present invention and the combination drugs, or
(2) a formulation comprising the compound of the present invention and the combination drugs which are separately formulated. Hereinafter, it is generally briefly referred to as the combination preparation of the present invention.

The combination preparation of the present invention can be formulated by mixing the compound of the present invention and active ingredients of the combination drugs separately or at the same time as itself or with pharmaceutically acceptable carriers in the same manner as in the method of producing the pharmaceutical preparation comprising the compound of the present invention.

A daily dose of the combination preparation of the present invention varies depending on severity of the symptoms, age, sex, weight and sensitivity of the subject to be administered, time and interval of administration, property, formulation and kinds of pharmaceutical preparation, kinds of active ingredients, etc., and is not particularly limited. The dose in terms of the compound of the present invention is not particularly limited if it causes no problems of side effects. In the case of oral administration, a daily dosage is usually in a range of about 0.005 to 100 mg, preferably about 0.05 to 50 mg, and more preferably about 0.2 to 30 mg, per 1 kg body weight of mammals, which may be administered once a day or in two or three divided portions a day.

The dose of the compound or the combination preparation of the present invention may be set within the range such that it causes no problems of side effects. The daily dose as the compound or the combination preparation of the present invention varies depending on severity of symptoms, age, sex, weight and sensitivity of the subject to be administered, time and interval of administration, property, formulation and kinds of pharmaceutical preparation, kinds of active ingredients, etc., and is not particularly limited. In the case of oral administration, a daily dosage in terms of active ingredients is usually in the order of about 0.001 to 2000 mg, preferably about 0.01 to 500 mg, and more preferably about 0.1 to 100 mg, per 1 kg body weight of mammals, which may be administered once a day or in two to four divided portions a day.

In administering the combination preparation of the present invention, the compound of the present invention and the combination drugs may be administered at the same time or, the combination drugs may be administered before administering the compound of the present invention, and vice versa. In case of staggered administration, the time interval varies depending on the active ingredients to be administered, a formulation and an administration route. For example, if the combination drugs are administered first, the compound of the present invention may be administered 1 minute to 3 days, preferably 10 minutes to 1 day, more preferably 15 minutes to 1 hour after administering the combination drugs. If the compound of the present invention is administered first, the combination drugs may be administered 1 minute to 1 day, preferably 10 minutes to 6 hours, more preferably 15 minutes to 1 hour after administering the compound of the present invention.

In a preferred administration method, about 0.001 to 200 mg/kg of the combination drugs formulated as an oral preparation is administered orally and then after about 15 minutes, about 0.005 to 100 mg/kg of the compound of the present invention formulated as an oral preparation is administered orally as a daily dose.

In the combination preparation of the present invention, the content of the compound of the present invention varies depending on the forms of the preparation, but usually in the order of 0.01 to 100 wt%, preferably 0.1 to 50 wt%, and further preferably 0.5 to 20 wt%, relative to the total preparation.

### Examples

The present invention is further described in detail with reference to Reference Examples, Examples, Preparative Examples and Experimental Example, which are not intended to restrict the invention and may be modified without departing from the scope of the invention.

Elution in the column chromatography in the following Reference Examples and Examples was conducted under observation by TLC (thin layer chromatography), unless otherwise indicated. In the TLC observation, 60F254, TLC plates, produced by Merck & Co., Inc. was used, and the solvent employed as an elution solvent in the column chromatography was used as an eluent. For the detection, a UV detector was used. As silica gel for the column chromatography, Silica Gel 60 (70 to 230 mesh) produced by Merck & Co., Inc. was used. The "room temperature" here means a temperature of generally from about 10°C to 35°C. For drying extracts, sodium sulfate or magnesium sulfate was used.

The abbreviations used in the following Examples and Reference Examples mean the following:
LC: liquid chromatography
MS: mass spectrum
ESI: electrospray ionization
M: molecular ion peak
NMR: nuclear magnetic resonance
Hz: hertz
J: coupling constant
m: multiplet
q: quartet
t: triplet
d: doublet
s: singlet
br: broad
^{t}Bu: tert-butyl group, t-butyl group
Boc: tert-butyloxycarbonyl
Rf: retardation factor
Rt: retention time
N: normal concentration
MPa: mega pascal
wt%: percent by weight
DMF: N,N-dimethylformamide
THF: tetrahydrofuran
DMSO: dimethyl sulfoxide
IPE: diisopropyl ether
HOBt·H2O: 1-hydroxybenzotriazole hydrate
WSC·HCl: 1-ethyl-3-(dimethylaminopropyl)carbodiimide hydrochloride
Boc₂O: di-tert-butyl dicarbonate
LC-MS in Examples and Reference Examples was measured under the following conditions.
Analysis by LC-MS
Measurement instrument: LC-MS system, Waters Corporation
HPLC part: HP1100, Agilent Technologies, Inc.
MS part: Micromass ZMD
HPLC conditions
Column: CAPCELL PAK C18UG120, S-3 µm, 1.5 × 35 mm (Shiseido Co., Ltd.)
Solvent: Solution A; 0.05% trifluoroacetic acid-containing water, Solution B; 0.05% trifluoroacetic acid-containing acetonitrile
Gradient cycle: 0.00 minute (Solution A/Solution B=90/10), 2.00 minutes (Solution A/Solution B=5/95), 2.75 minutes (Solution A/Solution B=5/95), 2.76 minutes (Solution A/Solution B=90/10), 3.60 minutes (Solution A/Solution B=90/10)
Injection volume: 2 µL, Flow rate: 0.5 mL/min,
Detection method: UV 220 nm
MS conditions
Ionization method: ESI
Analysis by LC
Measurement instrument: CLASS-VP system, Shimadzu Corporation
HPLC conditions
Column: Inertsil ODS-2, CAPCELL PAK C18UG120, 5 µm, 4.6 × 150 mm (GL Sciences Inc.)
Solvent: Solution A; 0.1% trifluoroacetic acid-containing water, Solution B; 0.1% trifluoroacetic acid-containing acetonitrile
Gradient cycle: 0.00 minute (Solution A/Solution B=70/30), 15.00 minutes (Solution A/Solution B=15/85), 15.01 minutes (Solution A/Solution B=5/95), 20.00 minutes (Solution A/Solution B=5/95), 20.01 minutes (Solution A/Solution B=70/30), 25.00 minutes (Solution A/Solution B=70/30)
Injection volume: 10 µL, Flow rate: 1.0 mL/min,
Detection method: UV 220 nm
Purification by preparative HPLC in Examples and
Reference Examples was carried out under the following conditions.
Instrument: High Throughput Purification System, Gilson Company, Inc.
Column: YMC CombiPrep ODS-A S-5 µm, 50 × 20 mm
Solvent: Solution A; 0.1%trifluoroacetic acid-containing water, Solution B; 0.1% trifluoroacetic acid-containing acetonitrile
Gradient cycle: 0.00 minute (Solution A/Solution
B=95/5), 1.00 minutes (Solution A/Solution B=95/5), 5.20 minutes (Solution A/Solution B=5/95), 6.40 minutes (Solution A/Solution B=5/95), 6.50 minutes (Solution
A/Solution B=95/5), 6.60 minutes (Solution A/Solution B=95/5)
Flow rate: 25 mL/min, Detection method: UV 220 nm

### Reference Example 1

### tert-butyl (3R*,4S*)-4-[[[3,5-bis(trifluoromethyl)benzyl]amino]carbonyl]-3-phenylpiperidine-1-carboxylate

### tert-butyl (3R*,4R*)-4-[[[3,5-bis(trifluoromethyl)benzyl]amino]carbonyl]-3-phenylpiperidine-1-carboxylate

### (Step 1)

To a solution of ethyl 1-methyl-1,2,3,6-tetrahydropyridine-4-carboxylate (32.2 g) and copper(I) iodide (7.62 g) in Et₂O (250 mL) was slowly added a 1 mol/L phenylmagnesium bromide/THF (360 mL) solution at -10°C, and the mixture was stirred at -10°C for 30 min. Saturated aqueous ammonium chloride solution and ethyl acetate were added to the reaction mixture, and insoluble materials were filtered off. The organic layer was separated from the mother liquor, washed with brine and dried. The solvent was evaporated under reduced pressure. The obtained residue was distilled under reduced pressure (90-115°C/l mmHg) to give ethyl 1-methyl-3-phenylpiperidine-4-carboxylate (37.6 g, 76%) as a cis and trans mixture, a pale-yellow oil.

### (Step 2)

To a solution of the oil (37.0 g) obtained in Step 1 in 1,2-dichloroethane (117 mL) was added 1-chloroethyl chloroformate (21.4 g), and the mixture was stirred at 100°C for 1 hr. The reaction mixture was concentrated under reduced pressure, methanol (150 mL) was added to the residue and the mixture was stirred at 80°C for 1 hr. The reaction mixture was concentrated under reduced pressure. To a solution of the residue in acetonitrile (200 mL) were added Et₃N (20 mL) and Boc₂O (46.4 g), and the mixture was stirred at room temperature for 14 hr. The reaction mixture was concentrated under reduced pressure, water and ethyl acetate were added to the residue, and the organic layer was separated, washed with brine and dried. The solvent was evaporated under reduced pressure. The obtained residue was purified by silica gel column chromatography (solvent gradient; 5→10% ethyl acetate/hexane) to give 4-ethyl 1-tert-butyl 3-phenylpiperidine-1,4-dicarboxylate (50.0 g, 99%) as a cis and trans mixture, colorless oil.

### (Step 3)

To a solution of the oil (56.0 g) obtained in Step 2 in ethanol (150 mL) was added 2N aqueous potassium hydroxide solution (375 mL), and the mixture was stirred at 50°C for 8 hr. The reaction mixture was neutralized with aqueous citric acid solution, and concentrated under reduced pressure. Water and ethyl acetate were added to the residue, and the organic layer was separated, washed with brine and dried. The solvent was evaporated under reduced pressure to give 1-(tert-butoxycarbonyl)-3-phenylpiperidine-4-carboxylic acid (36.7 g, 80%) as a cis and trans mixture, white powder.

### (Step 4)

To a solution of the white powder (4.60 g) obtained in Step 3 in DMF (45 mL) were added WSC·HCl (4.31 g), HOBt·H2O (3.44 g) and [3,5-bis(trifluoromethyl)benzyl]amine (4.40 g), and the mixture was stirred at room temperature for 24 hr. The reaction mixture was poured into water, and the product was extracted with ethyl acetate. The organic layer was washed with 10% aqueous citric acid solution and brine and dried, and the solvent was evaporated under reduced pressure. The obtained residue was purified by silica gel column chromatography (solvent gradient; 5→10% ethyl acetate/hexane) to give a (3R*,4S*)-form (cis form) of the title compound (1.35 g, 17%) as a white powder, and a (3R*,4R*)-form (trans form) of the title compound (3.71 g, 46%) as a white powder.
(3R*,4S*)-form: MS(ESI+): 475(M-^{t}Bu+2H)
Rf=0.68 (50% ethyl acetate/hexane)
(3R*,4R*)-form: MS (ESI+): 475(M-^{t}Bu+2H)
Rf=0.63 (50% ethyl acetate/hexane)

In the same manner as in Reference Example 1, the compounds of Table 1 were obtained.

**Table 1**

| Ref.Ex. No. | Compound name | **MS(ESI)** |
|---|---|---|
| 2 | tert-butyl (3R*,4S*)-4-[[[3,5-bis(trifluoromethyl)benzyl]amino]carbonyl]-3-(4-fluorophenyl)piperidine-1-carboxylate | **493 (M-**^{***t***}**Bu+2H)** |
| | tert-butyl (3R*,4R*)-4-[[[3,5-bis(trifluoromethyl)benzyl]amino]carbonyl]-3-(4-fluorophenyl)piperidine-1-carboxylate | **493 (M-**^{***t***}**Bu+2H)** |
| 3 | tert-butyl (3R*,4S*)-4-[[[3,5-bis(trifluoromethyl)benzyl]amino]carbonyl]-3-(4-fluoro-2-methylphenyl)piperidine-1-carboxylate | **507 (M-**^{***t***}**Bu+2H)** |
| | tert-butyl (3R*,4R*)-4-[[[3,5-bis(trifluoromethyl)benzyl]amino]carbonyl]-3-(4-fluoro-2-methylphenyl)piperidine-1-carboxylate | **507 (M-**^{***t***}**Bu+2H)** |
| 4 | tert-butyl (3R*,4S*)-4-[[[3-methyl-5-(trifluoromethyl)benzyl]amino]carbonyl]-3-phenylpiperidine-1-carboxylate | **421 (M-**^{***t***}**Bu+2H)** |
| | tert-butyl (3R*,4R*)-4-[[[3-methyl-5-(trifluoromethyl)benzyl]amino]carbonyl]-3-phenylpiperidine-1-carboxylate | **421 (M-**^{***t***}**Bu+2H)** |

### Reference Example 5

### 1-benzyl-5-phenyl-1,2,3,6-tetrahydropyridine-4-carboxylic acid hydrochloride

### (Step 1)

To a solution of sodium hydride (60% in oil, 0.76 g) in DMF (20 mL) was added ethyl 1-benzyl-3-oxopiperidine-4-carboxylate (3.32 g) at 0°C, and the mixture was stirred for 5 min. N-Phenyl-bis(trifluoromethanesulfonimide) (5.0 g) was added, and the mixture was stirred at 0°C for 1 hr. The reaction mixture was poured into ice water, and the product was extracted with ethyl acetate. The organic layer was washed with water and brine and dried, and the solvent was evaporated under reduced pressure. To a mixture of the obtained residue in toluene (50 mL) and water (3 mL) were added dihydroxyphenylborane (2.32 g), potassium carbonate (1.75 g) and tetrakis(triphenylphosphine)palladium(0) (1.46 g), and the mixture was stirred under an argon atmosphere at 100°C for 14 hr. The reaction mixture was poured into water, and the product was extracted with ethyl acetate. The organic layer was washed with saturated aqueous sodium hydrogen carbonate solution and brine and dried, and the solvent was evaporated under reduced pressure. The obtained residue was purified by silica gel column chromatography (solvent gradient; 5→15% ethyl acetate/hexane) to give ethyl 1-benzyl-5-phenyl-1,2,3,6-tetrahydropyridine-4-carboxylate (4.02 g) as a pale-yellow oil.

### (Step 2)

A solution of the compound (2.00 g) obtained in Step 1 in a mixture of hydrochloric acid (5.0 mL) and acetic acid (5.0 mL) was stirred at 100°C for 14 hr. The reaction mixture was concentrated under reduced pressure and the obtained residue was collected by filtration with IPE to give the title compound (1.03 g) as a white powder.
MS(ESI+): 294(M-HCl+H)
In the same manner as in Reference Example 5, the compounds of Table 2 were obtained.

**Table 2**

| Ref. Ex. No. | Compound name | **MS (ESI)** |
|---|---|---|
| 6 | 1-benzyl-5-(4-fluorophenyl)-1,2,3,6-tetrahydropyridine-4-carboxylic acid hydrochloride | **312 (M-HCl+H)** |
| 7 | 1-benzyl-5-(4-fluoro-2-methylphenyl)-1,2,3,6-tetrahydropyridine-4-carboxylic acid hydrochloride | **326 (M-HCl+H)** |

### Reference Example 8

### ethyl 4-[[[3,5-bis(trifluoromethyl)benzyl]amino]carbonyl]-5-phenyl-2,3,6,7-tetrahydro-1H-azepine-1-carboxylate

### (Step 1)

To a solution of diethyl 5-oxoazepane-1,4-dicarboxylate (0.90 g) synthesized by a known method (Heterocycles, 1978, (11), 267-273) in DMF (15 mL) was added sodium hydride (60% in oil, 0.20 g), and the mixture was stirred at room temperature for 30 min. The reaction mixture was cooled to 0°C, N-phenyl-bis(trifluoromethanesulfonimide) (1.87 g) was added, and the mixture was stirred at room temperature for 2 hr. The reaction mixture was poured into saturated aqueous ammonium chloride solution, and the product was extracted with ethyl acetate. The organic layer was washed with water and brine and dried, and the solvent was evaporated under reduced pressure. The obtained residue was purified by silica gel column chromatography (solvent gradient; 10→60% ethyl acetate/hexane) to give a colorless oil (0.49 g). To a mixture of the obtained oil (0.49 g) in toluene (10 mL) and water (2 mL) were added dihydroxyphenylborane (0.23 g), potassium carbonate (0.44 g) and tetrakis(triphenylphosphine)palladium(0) (0.28 g), and the mixture was stirred under an argon atmosphere at 100°C for 14 hr. The reaction mixture was poured into water, and the product was extracted with ethyl acetate. The organic layer was washed with aqueous potassium carbonate solution and brine and dried, and the solvent was evaporated under reduced pressure. The obtained residue was purified by silica gel column chromatography (solvent gradient; 10→60% ethyl acetate/hexane) to give diethyl 5-phenyl-2,3,6,7-tetrahydro-1H-azepine-1,4-dicarboxylate (0.49 g) as a colorless oil.

### (Step 2)

A solution of the compound (1.7 g) obtained in Step 1 in a mixture of 1N aqueous sodium hydroxide solution (10 ml) and ethanol (30 mL) was heated under reflux for 20 hr. The reaction mixture was concentrated under reduced pressure, diluted with 1N aqueous sodium hydroxide solution and washed with Et₂O. The aqueous layer was acidified with hydrochloric acid, extracted with ethyl acetate, washed with brine and dried, and the solvent was evaporated under reduced pressure. To a solution of the obtained residue in DMF (15 mL) were added WSC·HCl (1.54 g), HOBt·H2O (1.23 g) and [3,5-bis(trifluoromethyl)benzyl]amine (1.95 g), and the mixture was stirred at room temperature for 16 hr. The reaction mixture was poured into water, and the product was extracted with ethyl acetate. The organic layer was washed with saturated aqueous sodium hydrogen carbonate solution and brine and dried. The solvent was evaporated under reduced pressure to give the title compound (1.25 g) as colorless prism crystals.
MS(ESI+): 515 (M+H)

### Reference Example 9

### 1-benzyl-5-(3,4-dichlorophenyl)-1,2,3,6-tetrahydropyridine-4-carboxylic acid hydrochloride

### (Step 1)

To a solution of sodium hydride (60% in oil, 1.83 g) in DMF (40 mL) was added ethyl 1-benzyl-3-oxopiperidine-4-carboxylate (8.00 g) at 0°C, and the mixture was stirred for 5 min. N-Phenyl-bis(trifluoromethanesulfonimide) (12.1 g) was added, and the mixture was stirred at 0°C for 1 hr. The reaction mixture was poured into ice water, and the product was extracted with ethyl acetate. The organic layer was washed with water and brine and dried, and the solvent was evaporated under reduced pressure. The obtained residue was purified by silica gel column chromatography (solvent gradient; 10→20% ethyl acetate/hexane) to give a brown oil (19.0 g). To a mixture of the obtained oil (19.0 g) in toluene (100 mL) and water (6 mL) were added 3,4-dichlorophenylboronic acid (8.76 g), potassium carbonate (4.23 g) and tetrakis (triphenylphosphine) palladium (0) (2.96 g), and the mixture was stirred under an argon atmosphere at 100°C for 14 hr. The reaction mixture was poured into water, and the product was extracted with ethyl acetate. The organic layer was washed with saturated aqueous sodium hydrogen carbonate solution and brine and dried, and the solvent was evaporated under reduced pressure. The obtained residue was purified by silica gel column chromatography (solvent gradient; 10→20% ethyl acetate/hexane) to give crude ethyl 1-benzyl-5-(3,4-dichlorophenyl)-1,2,3,6-tetrahydropyridine-4-carboxylate (12.0 g) as a pale-yellow oil.

### (Step 2)

A solution of the compound (11.8 g) obtained in Step 1 in a mixture of hydrochloric acid (30 mL) and acetic acid (30 mL) was stirred at 100°C for 14 hr. The reaction mixture was concentrated under reduced pressure and the obtained residue was collected by filtration with IPE to give the title compound (9.98 g) as a white powder.
MS (ESI+) : 362(M-HCl+H)

### Example 1

### tert-butyl (3R*,4S*)-4-[[[3,5-bis(trifluoromethyl)benzyl](methyl)amino]carbonyl]-3-phenylpiperidine-1-carboxylate

To a solution of (3R*,4S*)-form (0.86 g) obtained in Reference Example 1 in THF (10 mL) were added sodium tert-butoxide (0.31 g) and methyl iodide (0.20 mL) at 0°C, and the mixture was stirred at room temperature for 1 hr. The reaction mixture was poured into water, and the product was extracted with ethyl acetate. The organic layer was washed with 10% aqueous citric acid solution and brine and dried, and the solvent was evaporated under reduced pressure. The obtained residue was purified by silica gel column chromatography (ethyl acetate:hexane=1:5) to give the title compound (0.89 g, 99%) as a white powder.
MS(ESI+): 489(M-^{t}Bu+2H)
Using the compounds obtained in Reference Examples 2-4 and in the same manner as in Example 1, the following compounds were obtained.

### Example 2

### tert-butyl (3R*,4R*)-4-[[[3,5-bis(trifluoromethyl)benzyl](methyl)amino]carbonyl]-3-phenylpiperidine-1-carboxylate

### Example 3

### tert-butyl (3R*,4S*)-4-[[[3,5-bis(trifluoromethyl)benzyl](methyl)amino]carbonyl]-3-(4-fluorophenyl)piperidine-1-carboxylate

### Example 4

### tert-butyl (3R*,4R*)-4-[[[3,5-bis(trifluoromethyl)benzyl](methyl)amino]carbonyl]-3-(4-fluorophenyl)piperidine-1-carboxylate

### Example 5

### tert-butyl (3R*,4R*)-4-[[[3,5-bis(trifluoromethyl)benzyl](methyl)amino]carbonyl]-3-(4-fluoro-2-methylphenyl)piperidine-1-carboxylate

### Example 6

### tert-butyl (3R*,4S*)-4-[[[3-methyl-5-(trifluoromethyl)benzyl](methyl)amino]carbonyl]-3-phenylpiperidine-1-carboxylate

### Example 7

### tert-butyl (3R*,4R*)-4-[[[3-methyl-5-(trifluoromethyl)benzyl](methyl)amino]carbonyl]-3-phenylpiperidine-1-carboxylate

The chemical structures of the compounds obtained in Examples 1-7 are as shown in Table 1.

**[Table 3]**

| 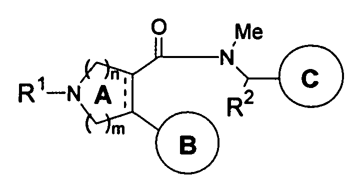 | | | | | | | |
|---|---|---|---|---|---|---|---|
| Ex. No. | R¹ | **R**^{**2**} | 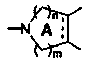 | 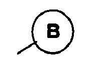 | 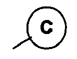 | Additives | MS (ESI) |
| 1 | Boc | H | 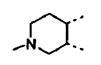 | 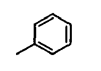 | 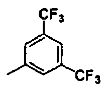 | | **489 (M-**^{***t***}**Bu+2H)** |
| 2 | Boc | H | 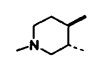 | 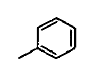 | 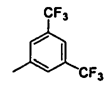 | | **489 (M-**^{***t***}**Bu+2H)** |
| 3 | Boc | H | 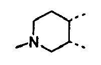 | 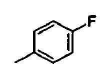 | 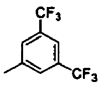 | | **507 (M-**^{***t***}**Bu+2H)** |
| 4 | Boc | H | 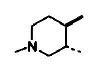 | 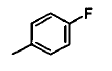 | 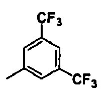 | | **507 (M-**^{***t***}**Bu+2H)** |
| 5 | Boc | H | 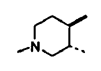 | 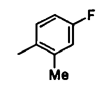 | 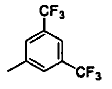 | | **521 (M-**^{***t***}**Bu+2H)** |
| 6 | Boc | H | 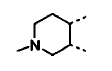 | 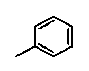 | 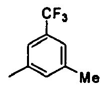 | | **435 (M-**^{***t***}**Bu+2H)** |
| 7 | Boc | H | 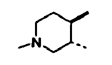 | 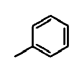 | 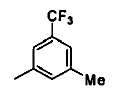 | | **435 (M-**^{***t***}**Bu+2H)** |

### Example 8

### (3R*,4S*)-N-[3,5-bis(trifluoromethyl)benzyl]-N-methyl-3-phenylpiperidine-4-carboxamide hydrochloride

To the (3R*,4S*)-form (0.49 g) obtained in Example 1 was added 4N hydrogen chloride/ethyl acetate solution (0.90 mL), and the mixture was stirred at 50°C for 2 hr. The reaction mixture was concentrated under reduced pressure and crystallized from diisopropyl ether to give the title compound as a white powder (0.39 g, 90%).
MS(ESI+): 445(M-HCl+H)
Using the compounds obtained in Examples 2-7 and in the same manner as in Example 8, the following compounds were obtained.

### Example 9

### (3R*,4R*)-N-[3,5-bis(trifluoromethyl)benzyl]-N-methyl-3-phenylpiperidine-4-carboxamide hydrochloride

### Example 10

### (3R*,4S*)-N-[3,5-bis(trifluoromethyl)benzyl]-3-(4-fluorophenyl)-N-methylpiperidine-4-carboxamide hydrochloride

### Example 11

### (3R*,4R*)-N-[3,5-bis(trifluoromethyl)benzyl]-3-(4-fluorophenyl)-N-methylpiperidine-4-carboxamide hydrochloride

### Example 12

### (3R*,4R*)-N-[3,5-bis(trifluoromethyl)benzyl]-3-(4-fluoro-2-methylphenyl)-N-methylpiperidine-4-carboxamide hydrochloride

### Example 13

### (3R*,4S*)-N-methyl-N-[3-methyl-5-(trifluoromethyl)benzyl]-3-phenylpiperidine-4-carboxamide hydrochloride

### Example 14

### (3R*,4R*)-N-methyl-N-[3-methyl-5-(trifluoromethyl)benzyl]-3-phenylpiperidine-4-carboxamide hydrochloride

The chemical structures of the compounds obtained in Examples 8-14 are as shown in Table 4.

**[Table 4]**

| | | | | | | | |
|---|---|---|---|---|---|---|---|
| 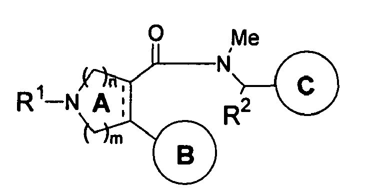 | | | | | | | |

| Ex. No. | R¹ | R² | 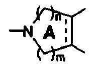 | 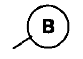 | 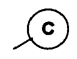 | Additives | MS (ESI) |
|---|---|---|---|---|---|---|---|
| 8 | H | H | 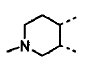 | 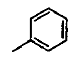 | 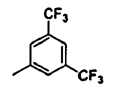 | **HCl** | **445 (M-HCl+H)** |
| 9 | H | H | 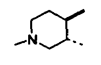 | 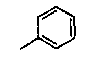 | 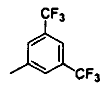 | **HCl** | **445 (M-HCl+H)** |
| 10 | H | H | 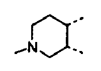 | 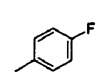 | 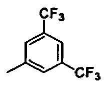 | **HCl** | **463 (M-HCl+H)** |
| 11 | H | H | 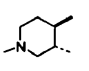 | 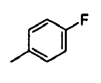 | 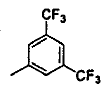 | **HCl** | **463 (M-HCl+H)** |
| 12 | H | H | 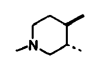 | 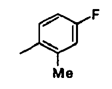 | 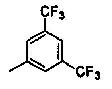 | **HCl** | **477 (M-HCl+H)** |
| 13 | H | H | 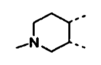 | 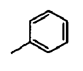 | 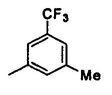 | **HCl** | **391 (M-HCl+H)** |
| 14 | H | H | 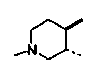 | 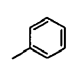 | 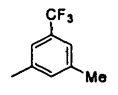 | **HCl** | **391 (M-HCl+H)** |

### Example 15

### 1-benzyl-N-[3,5-bis(trifluoromethyl)benzyl]-N-methyl-5-phenyl-1,2,3,6-tetrahydropyridine-4-carboxamide hydrochloride

A solution of the compound (0.50 g) obtained in Reference Example 5 in thionyl chloride (3.0 mL) was stirred at 90°C for 1 hr. The reaction solution was concentrated under reduced pressure and the obtained residue was dissolved in acetonitrile (20 mL). Et₃N (0.96 mL) and [3,5-bis(trifluoromethyl)benzyl]methylamine (0.67 g) were added at 0°C, and the mixture was stirred at room temperature for 14 hr. The reaction mixture was poured into water, and the product was extracted with ethyl acetate. The organic layer was washed with saturated aqueous ammonium chloride solution and brine and dried, and the solvent was evaporated under reduced pressure. The obtained residue was purified by silica gel column chromatography (solvent gradient; 10→80% ethyl acetate/hexane). The obtained product was treated with 1 equivalent of 4N hydrogen chloride/ethyl acetate solution to give the title compound as a pale-yellow amorphous solid (0.70 g, 86%).
MS(ESI+): 533(M-HCl+H)
Using the compound obtained in Reference Example 5 and corresponding each amine, and in the same manner as in Example 15, the following compounds were obtained.

### Example 16

### 1-benzyl-N-[(1S)-1-[3,5-bis(trifluoromethyl)phenyl]ethyl]-N-methyl-5-phenyl-1,2,3,6-tetrahydropyridine-4-carboxamide hydrochloride

### Example 17

### 1-benzyl-N-[(1R)-1-[3,5-bis(trifluoromethyl)phenyl]ethyl]-N-methyl-5-phenyl-1,2,3,6-tetrahydropyridine-4-carboxamide hydrochloride

The chemical structures of the compounds obtained in Examples 15-17 are as shown in Table 5.

**[Table 5]**

| 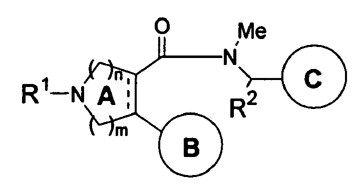 | | | | | | | |
|---|---|---|---|---|---|---|---|
| Ex. No. | R¹ | R² | 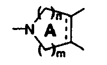 | 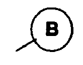 | 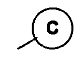 | Additives | MS (ESI) |
| 15 | PhCH₂ | H | 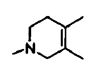 | 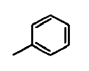 | 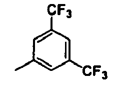 | HCl | **533 (M-HCl+H)** |
| 16 | PhCH₂ | (*S*)-Me | 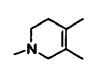 | 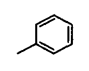 | 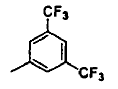 | HCl | **547 (M-HCl+H)** |
| 17 | PhCH₂ | (*R*)-Me | 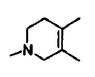 | 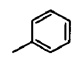 | 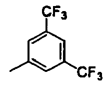 | HCl | **547 (M-HCl+H)** |

### Example 18

### N-[3,5-bis(trifluoromethyl)benzyl]-N-methyl-5-phenyl-1,2,3,6-tetrahydropyridine-4-carboxamide hydrochloride

To a solution of the compound (2.05 g) obtained in Example 15 in 1,2-dichloroethane (20 mL) was added 1-chloroethyl chloroformate (3.3 mL) at room temperature, and the mixture was stirred at 100°C for 1 hr. The reaction solution was concentrated under reduced pressure and the obtained residue was dissolved in methanol (30 mL). The mixture was stirred at 80°C for 1 hr. The reaction solution was concentrated under reduced pressure and the obtained residue was dissolved in acetonitrile (20 mL). Et₃N (0.64 mL) and Boc₂O (1.0 g) were added, and the mixture was stirred at room temperature for 14 hr. The reaction mixture was poured into water, and the product was extracted with ethyl acetate. The organic layer was washed with saturated aqueous ammonium chloride solution and brine and dried, and the solvent was evaporated under reduced pressure. The obtained residue was purified by silica gel column chromatography (solvent gradient; 10→50% ethyl acetate/hexane) to give a colorless oil (1.24 g). To a solution of the obtained oil (1.24 g) in methanol (15 mL) was added 4N hydrogen chloride/ethyl acetate (2.4 mL) solution and the mixture was stirred at 50°C for 2 hr. The reaction solution was concentrated under reduced pressure and the precipitated product was collected by filtration to give the title compound as a white powder (1.06 g, 57%).
MS(ESI+): 443(M-HCl+H)
Using the compounds obtained in Examples 16-17, and in the same manner as in Example 18, the following compounds were obtained.

### Example 19

### N-[(1S)-1-[3,5-bis(trifluoromethyl)phenyl]ethyl]-N-methyl-5-phenyl-1,2,3,6-tetrahydropyridine-4-carboxamide hydrochloride

### Example 20

### N-[(1R)-1-[3,5-bis(trifluoromethyl)phenyl]ethyl]-N-methyl-5-phenyl-1,2,3,6-tetrahydropyridine-4-carboxamide hydrochloride

The chemical structures of the compounds obtained in Examples 18-20 are as shown in Table 6.

**[Table 6]**

| 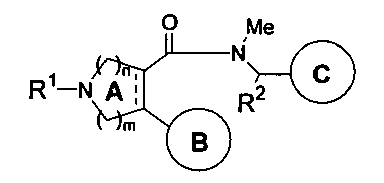 | | | | | | | |
|---|---|---|---|---|---|---|---|
| Ex. No. | R¹ | R² | 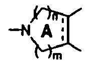 | 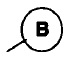 | 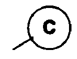 | Additives | MS (ESI) |
| 18 | H | H | 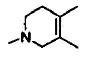 | 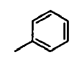 | 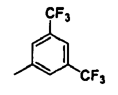 | HCl | **443 (M-HCl+H)** |
| 19 | H | (*S*)-Me | 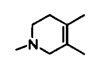 | 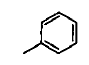 | 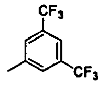 | HCl | **457 (M-HCl+H)** |
| 20 | H | (*R*)-Me | 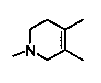 | 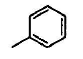 | 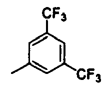 | HCl | **457 (M-HCl+H)** |

### Examples 21-22

### (3R*,4S*)-N-[(1S)-1-[3,5-bis(trifluoromethyl)phenyl]ethyl]-N-methyl-3-phenylpiperidine-4-carboxamide hydrochloride (less polar compound: Example 21, more polar compound: Example 22)

### (Step 1)

A solution of the compound (0.90 g) obtained in Example 19 and 10% palladium-carbon (0.60 g) in ethanol (30 mL) was stirred under a hydrogen atmosphere of 5 atm at 70°C for 16 hr. The catalyst was filtered off and the reaction solution was concentrated under reduced pressure. The obtained residue was dissolved in acetonitrile (20 mL)., and Et₃N (0.38 mL) and Boc₂O (0.48 g) were added. The mixture was stirred at room temperature for 14 hr. The reaction mixture was poured into water, and the product was extracted with ethyl acetate. The organic layer was washed with saturated aqueous ammonium chloride solution and brine and dried, and the solvent was evaporated under reduced pressure. The obtained residue was purified by silica gel column chromatography (solvent gradient; 5→10% ethyl acetate/hexane) to give two diastereomers as a colorless oil (0.33 g, 30%, Rf=0.77 (33% ethyl acetate/hexane)) and a white powder (0.73 g, 70%, Rf=0.36 (33% ethyl acetate/hexane)).

### (Step 2)

To a solution of the less polar colorless oil (0.33 g) obtained in Step 1 in methanol (5 mL) was added 4N hydrogen chloride/ethyl acetate solution (0.60 mL) and the mixture was stirred at 50°C for 2 hr. The reaction mixture was concentrated under reduced pressure, and crystallized from IPE to give the title compound as a white powder (0.28 g, 95%).
MS(ESI+): 459(M-HCl+H)
LC(Rt): 10.7 min
[α]_{D}²⁵=-129.7° (c 1.0, MeOH)

### (Step 3)

To a solution of the more polar white powder (0.78 g) obtained in Step 1 in methanol (5 mL) was added 4N hydrogen chloride/ethyl acetate solution (1.3 mL), and the mixture was stirred at 50°C for 2 hr. The reaction mixture was concentrated under reduced pressure and crystallized from IPE to give the title compound as a white powder (0.54 g, 83%).
MS(ESI+): 459(M-HCl+H)
LC(Rt): 10.5 min
[α]_{D}²⁵=+1.9° (c 1.0, MeOH)
Using the compound obtained in Example 20, and in the same manner as in Examples 21-22, the following compounds were obtained.

### Example 23

### (3R*,4S*)-N-[(1R)-1-[3,5-bis(trifluoromethyl)phenyl]ethyl]-N-methyl-3-phenylpiperidine-4-carboxamide hydrochloride (less polar compound)

### Example 24

### (3R*,4S*)-N-[(1R)-1-[3,5-bis(trifluoromethyl)phenyl]ethyl]-N-methyl-3-phenylpiperidine-4-carboxamide hydrochloride (more polar compound)

The chemical structures of the compounds obtained in Examples 21-24 are as shown in Table 7.

**[Table 7]**

| 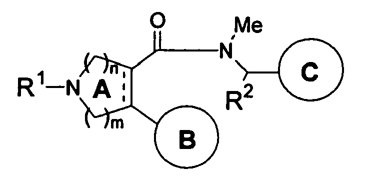 | | | | | | | |
|---|---|---|---|---|---|---|---|
| Ex. No. | R¹ | R² | 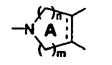 | 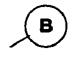 | 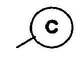 | Additives | MS (ESI) |
| 21 (less polar) | H | (*S*)-Me | 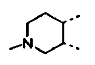 | 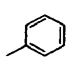 | 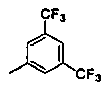 | HCl | **459 (M-HCl+H)** |
| 22 (more polar) | H | (*S*)-Me | 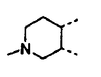 | 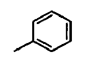 | 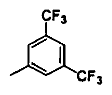 | HCl | **459 (M-HCl+H)** |
| 23 (less polar) | H | (*R*)-Me | 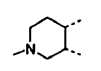 | 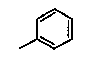 | 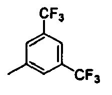 | HCl | **459 (M-HCl+H)** |
| 24 (more polar) | H | (*R*)-Me | 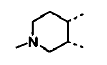 | 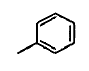 | 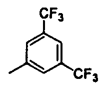 | HCl | **459 (M-HCl+H)** |

### Example 25

### (3R*,4S*)-N⁴-[3, 5-bis(trifluoromethyl)benzyl]-N¹,N⁴-dimethyl-3-phenylpiperidine-1,4-dicarboxamide

To a solution of the compound (0.15 g) obtained in Example 8 and Et₃N (0.044 mL) in acetonitrile (3.0 mL) was added methyl isocyanate (0.045 mL), and the mixture was stirred at room temperature for 14 hr. The reaction mixture was poured into water, and the product was extracted with ethyl acetate. The organic layer was washed with 10% aqueous citric acid solution and brine and dried, and the solvent was evaporated under reduced pressure. The obtained residue was purified by silica gel column chromatography (solvent gradient; 0→5% methanol/ethyl acetate) to give the title compound as a white powder (0.14 g, 88%).
MS(ESI+): 502(M+H)
Using the compounds obtained in Examples 9 and 18 and methyl isocyanate or ethyl isocyanate, and in the same manner as in Example 25, the following compounds were obtained.

### Example 26

### (3R*,4R*)-N⁴-[3,5-bis(trifluoromethyl)benzyl]-N¹,N⁴-dimethyl-3-phenylpiperidine-1,4-dicarboxamide

### Example 27

### N⁴-[3,5-bis (trifluoromethyl)benzyl]-N¹-ethyl-N⁴-methyl-5-phenyl-3,6-dihydropyridine-1,4(2H)-dicarboxamide

The chemical structures of the compounds obtained in Examples 25-27 are as shown in Table 8.

**[Table 8]**

| 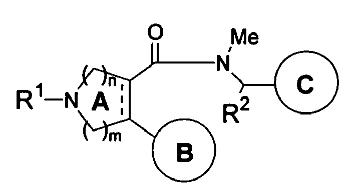 | | | | | | | |
|---|---|---|---|---|---|---|---|
| Ex. No. | R¹ | R² | 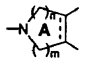 | 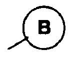 | 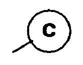 | Additives | MS (ESI) |
| 25 | CH₃NHCO | H | 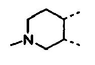 | 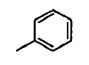 | 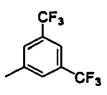 | | **502 (M+H)** |
| 26 | CH₃NHCO | H | 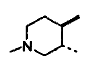 | 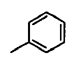 | 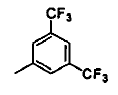 | | **502 (M+H)** |
| 27 | C₂H₅NHCO | H | 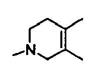 | 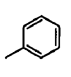 | 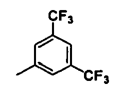 | | **514 (M+H)** |

### Example 28

### (3R*,4S*)-1-acetyl-N-[3,5-bis(trifluoromethyl)benzyl]-N-methyl-3-phenylpiperidine-4-carboxamide

To a solution of the compound (0.15 mg) obtained in Example 8 and Et₃N (0.055 mL) in THF (3.0 mL) was added acetyl chloride (0.042 mL), and the mixture was stirred at room temperature for 14 hr. The reaction mixture was poured into water, and the product was extracted with ethyl acetate. The organic layer was washed with 10% aqueous citric acid solution and brine and dried, and the solvent was evaporated under reduced pressure. The obtained residue was purified by preparative HPLC to give the title compound as a colorless oil (0.10 g, 66%).
MS(ESI+): 487 (M+H)
Using the compound obtained in Example 9, and in the same manner as in Example 28, the following compound was obtained.

### Example 29

### (3R*,4R*)-1-acetyl-N-[3,5-bis(trifluoromethyl)benzyl]-N-methyl-3-phenylpiperidine-4-carboxamide

The chemical structures of the compounds obtained in Examples 28-29 are as shown in Table 9.

**[Table 9]**

| 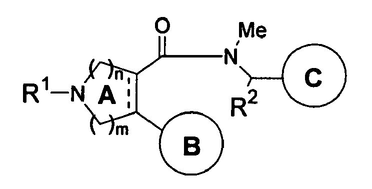 | | | | | | | |
|---|---|---|---|---|---|---|---|
| Ex. No. | R¹ | R² | 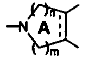 | 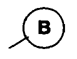 | 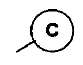 | Additives | MS (ESI) |
| 28 | CH₃CO | H | 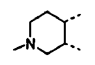 | 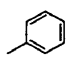 | 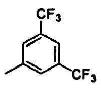 | | **487 (M+H)** |
| 29 | CH₃CO | H | 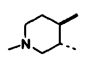 | 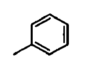 | 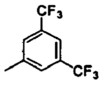 | | **487 (M+H)** |

### Example 30

### (3R*,4S*)-1-[(1-acetylpiperidin-4-yl)carbonyl]-N-[3,5-bis(trifluoromethyl)benzyl]-N-methyl-3-phenylpiperidine-4-carboxamide

To a solution of the compound (0.24 g) obtained in Example 8, 1-acetylpiperidine-4-carboxylic acid (0.13 g) and Et₃N (0.070 mL) in DMF (5.0 mL) were added WSC·HCl (0.14 g) and HOBt·H2O (0.12 g), and the mixture was stirred at room temperature for 24 hr. The reaction mixture was poured into water, and the product was extracted with ethyl acetate. The organic layer was washed with 10% aqueous citric acid solution and brine and dried, and the solvent was evaporated under reduced pressure. The obtained residue was purified by preparative HPLC to give the title compound as a colorless oil (0.21 g, 70%).
¹H-NMR(CDCl₃)δ: 1.60-2.00 (6H, m), 2.08&2.11 (3H, each s), 2.40-4.00 (12H, m), 4.30-4.70 (4H, m), 7.17-7.80 (8H, m)
MS(ESI+): 598 (M+H)

Using the compounds obtained in Examples 8-14 and 18-24 and corresponding each carboxylic acid, and in the same manner as in Example 30, the following compounds were obtained.

### Example 31

### (3R*,4S*)-1-[(1-acetylpiperidin-4-yl)carbonyl]-N-[(1S)-1-[3,5-bis(trifluoromethyl)phenyl]ethyl]-N-methyl-3-phenylpiperidine-4-carboxamide (less polar compound)

### Example 32

### (3R*,4S*)-1-[(1-acetylpiperidin-4-yl)carbonyl]-N-[(1S)-1-[3,5-bis(trifluoromethyl)phenyl]ethyl]-N-methyl-3-phenylpiperidine-4-carboxamide (more polar compound)

### Example 33

### (3R*,4S*)-1-[(1-acetylpiperidin-4-yl)carbonyl]-N-[(1R)-1-[3,5-bis(trifluoromethyl)phenyl]ethyl]-N-methyl-3-phenylpiperidine-4-carboxamide (less polar compound)

### Example 34

### (3R*,4S*)-1-[(1-acetylpiperidin-4-yl)carbonyl]-N-[(1R)-1-[3,5-bis(trifluoromethyl)phenyl]ethyl]-N-methyl-3-phenylpiperidine-4-carboxamide (more polar compound)

### Example 35

### (3R*,4R*)-1-[(1-acetylpiperidin-4-yl)carbonyl]-N-[3,5-bis(trifluoromethyl)benzyl]-N-methyl-3-phenylpiperidine-4-carboxamide

### Example 36

### (3R*,4R*)-N-[3,5-bis(trifluoromethyl)benzyl]-1-[(2,6-dioxopiperidin-4-yl)carbonyl]-N-methyl-3-phenylpiperidine-4-carboxamide

### Example 37

### (3R*,4R*)-1-[(acetylamino) acetyl]-N-[3,5-bis(trifluoromethyl)benzyl]-N-methyl-3-phenylpiperidine-4-carboxamide

### Example 38

### (3R*,4R*)-1-[3-(acetylamino)propanoyl]-N-[3,5-bis(trifluoromethyl)benzyl]-N-methyl-3-phenylpiperidine-4-carboxamide

### Example 39

### (3R*,4R*)-1-[4-(acetylamino)butanoyl]-N-[3,5-bis(trifluoromethyl)benzyl]-N-methyl-3-phenylpiperidine-4-carboxamide

### Example 40

### (3R*,4R*)-N-[3,5-bis(trifluoromethyl)benzyl]-3-(4-fluoro-2-methylphenyl)-1-(methoxyacetyl)-N-methylpiperidine-4-carboxamide

### Example 41

### (3R*,4R*)-N-[3,5-bis(trifluoromethyl)benzyl]-1-(N,N-diethyl-β-alanyl)-3-(4-fluoro-2-methylphenyl)-N-methylpiperidine-4-carboxamide hydrochloride

### Example 42

### (3R*,4R*)-N-[3,5-bis(trifluoromethyl)benzyl]-3-(4-fluoro-2-methylphenyl)-N-methyl-1-(1H-tetrazol-1-ylacetyl)piperidine-4-carboxamide

### Example 43

### (3R*,4R*)-N-[3,5-bis(trifluoromethyl)benzyl]-1-[(2,5-dioxoimidazolidin-4-yl)acetyl]-3-(4-fluoro-2-methylphenyl)-N-methylpiperidine-4-carboxamide

### Example 44

### (3R*,4S*)-1-[(1-acetylpiperidin-4-yl)carbonyl]-N-[3,5-bis(trifluoromethyl)benzyl]-3-(4-fluorophenyl)-N-methylpiperidine-4-carboxamide

### Example 45

### (3R*,4R*)-1-[(1-acetylpiperidin-4-yl)carbonyl]-N-[3,5-bis(trifluoromethyl)benzyl]-3-(4-fluorophenyl)-N-methylpiperidine-4-carboxamide

### Example 46

### (3R*,4R*)-1-[(1-acetylpiperidin-4-yl)carbonyl]-N-[3,5-bis(trifluoromethyl)benzyl]-3-(4-fluoro-2-methylphenyl)-N-methylpiperidine-4-carboxamide

### Example 47

### (3R*,4S*)-1-[(1-acetylpiperidin-4-yl)carbonyl]-N-methyl-N-[3-methyl-5-(trifluoromethyl)benzyl]-3-phenylpiperidine-4-carboxamide

### Example 48

### (3R*,4R*)-1-[(1-acetylpiperidin-4-yl)carbonyl]-N-methyl-N-[3-methyl-5-(trifluoromethyl)benzyl]-3-phenylpiperidine-4-carboxamide

### Example 49

### 1-(N-acetylglycyl)-N-[3,5-bis(trifluoromethyl)benzyl]-N-methyl-5-phenyl-1,2,3,6-tetrahydropyridine-4-carboxamide

### Example 50

### 1-[(1-acetylpiperidin-4-yl)carbonyl]-N-[3,5-bis(trifluoromethyl)benzyl]-N-methyl-5-phenyl-1,2,3,6-tetrahydropyridine-4-carboxamide

### Example 51

### 1-[(1-acetylpiperidin-4-yl)carbonyl]-N-[(1S)-1-[3,5-bis(trifluoromethyl)phenyl]ethyl]-N-methyl-5-phenyl-1,2,3,6-tetrahydropyridine-4-carboxamide

### Example 52

### 1-[(1-acetylpiperidin-4-yl)carbonyl]-N-[(1R)-1-[3,5-bis(trifluoromethyl)phenyl]ethyl]-N-methyl-5-phenyl-1,2,3,6-tetrahydropyridine-4-carboxamide

The chemical structures of the compounds obtained in Examples 30-52 are as shown in Table 10.

**[Table 10]**

| | | | | | | | |
|---|---|---|---|---|---|---|---|
| 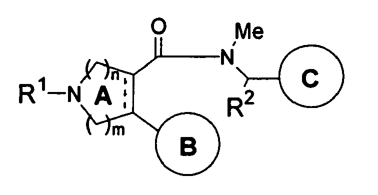 | | | | | | | |

| Ex. No. | R¹ | R² | 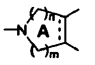 | 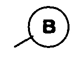 | 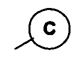 | Additives | MS (ESI) |
|---|---|---|---|---|---|---|---|
| 30 | 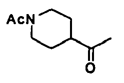 | H | 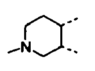 | 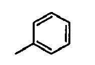 | 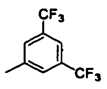 | | **598 (M+H)** |
| 31 (less polar) | 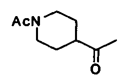 | (*S*)-Me | 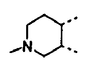 | 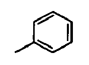 | 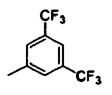 | | **612 (M+H)** |
| 32 (more polar) | 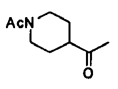 | (*S*)-Me | 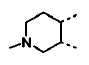 | 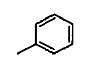 | 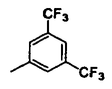 | | **612 (M+H)** |
| 33 (less polar) | 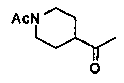 | (*R*)-Me | 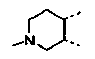 | 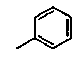 | 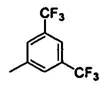 | | **612 (M+H)** |
| 34 (more polar) | 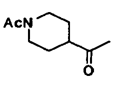 | (*R*)-Me | 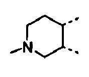 | 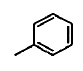 | 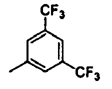 | | **612 (M+H)** |
| 35 | 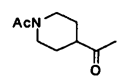 | H | 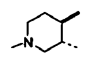 | 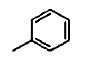 | 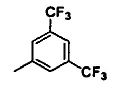 | | **598 (M+H)** |
| 36 | 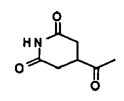 | H | 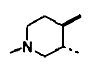 | 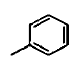 | 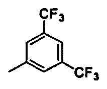 | | **584 (M+H)** |
| 37 | 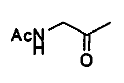 | H | 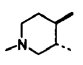 | 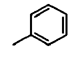 | 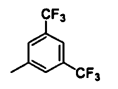 | | **544 (M+H)** |
| 38 | 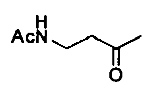 | H | 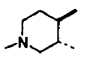 | 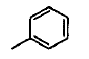 | 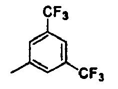 | | **558 (M+H)** |
| 39 | 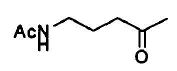 | H | 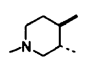 | 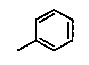 | 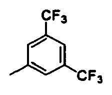 | | **572 (M+H)** |
| 40 | 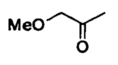 | H | 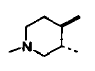 | 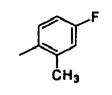 | 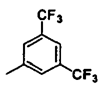 | | **549 (M+H)** |
| 41 | 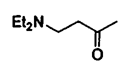 | H | 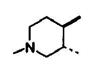 | 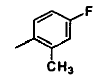 | 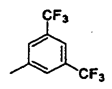 | HCl | **604 (M-HCl+H)** |
| 42 | 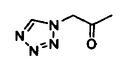 | H | 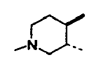 | 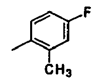 | 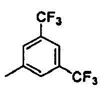 | | **587 (M+H)** |
| 43 | 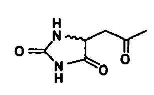 | H | 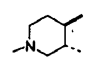 | 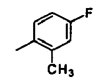 | 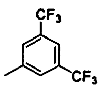 | | **617 (M+H)** |
| 44 | 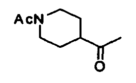 | H | 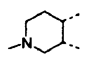 | 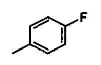 | 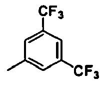 | | **616 (M+H)** |
| 45 | 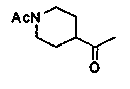 | H | 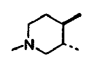 | 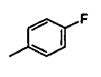 | 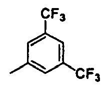 | | **616 (M+H)** |
| 46 | 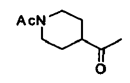 | H | 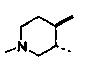 | 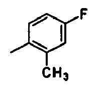 | 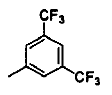 | | **630 (M+H)** |
| 47 | 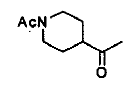 | H | 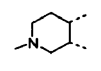 | 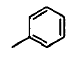 | 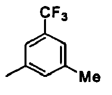 | | **544 (M+H)** |
| 48 | 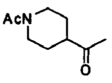 | H | 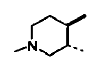 | 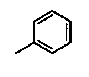 | 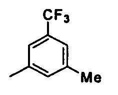 | | **544 (M+H)** |
| 49 | 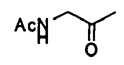 | H | 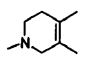 | 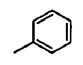 | 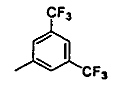 | | 542 (M+H) |
| 50 | 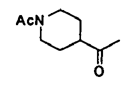 | H | 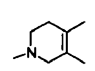 | 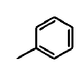 | 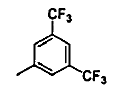 | | 596 (M+H) |
| 51 | 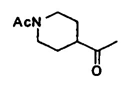 | (S)-Me | 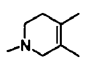 | 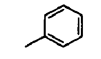 | 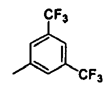 | | 610 (M+H) |
| 52 | 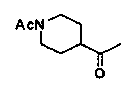 | (R)-Me | 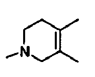 | 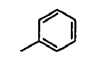 | 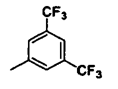 | | 610 (M+H) |

### Example 53

### (3R*,4R*)-N-[3,5-bis(trifluoromethyl)benzyl]-3-(4-fluorophenyl)-N-methyl-1-[(5-oxo-4,5-dihydro-1H-1,2,4-triazol-3-yl)methyl]piperidine-4-carboxamide hydrochloride

To a solution of the compound (0.30 g) obtained in Example 11 and potassium carbonate (0.083 mg) in 1% H₂O-DMF (5.0 mL) was added 5-(chloromethyl)-2,4-dihydro-3H-1,2,4-triazol-3-one (0.096 g) at 0°C, and the mixture was stirred at room temperature for 14 hr. The reaction mixture was poured into water, and the product was extracted with ethyl acetate. The organic layer was washed with brine and dried, and the solvent was evaporated under reduced pressure. The obtained residue was purified by preparative HPLC. The obtained product was treated with 1 equivalent of 4N hydrogen chloride/ethyl acetate solution to give the title compound as a white powder (0.30 g, 89%).
MS(ESI+): 560(M-HCl+H)

### Example 54

### (3R*,4R*)-N-[3,5-bis(trifluoromethyl)benzyl]-3-(4-fluoro-2-methylphenyl)-N-methyl-1-[(5-oxo-4,5-dihydro-1H-1,2,4-triazol-3-yl)methyl]piperidine-4-carboxamide hydrochloride

To a solution of the compound (0.30 g) obtained in Example 12 and potassium carbonate (0.083 mg) in 1% H₂O-DMF (5.0 mL) was added 5-(chloromethyl)-2,4-dihydro-3H-1,2,4-triazol-3-one (0.094 g) at 0°C, and the mixture was stirred at room temperature for 14 hr. The reaction mixture was poured into water, and the product was extracted with ethyl acetate. The organic layer was washed with brine and dried, and the solvent was evaporated under reduced pressure. The obtained residue was purified by preparative HPLC.

¹H-NMR(CDCl₃)δ: 1.70-2.00 (1H, m), 2.05-2.40 (6H, m), 2.75-3.10 (6H, m), 3.45-3.49 (2H, m), 3.65-3.80 (1H, m), 4.16 (1H, d, J=15.0Hz), 4.88 (1H, d, J=15.0Hz), 6.70-6.80 (2H, m), 7.00-7.18 (1H, m), 7.33-7.40 (2H, m), 7.70-7.82 (1H, m), 10.21 (1H, br s), 10.3-11.0 (1H, br)

The obtained product was treated with 1 equivalent of 4N hydrogen chloride/ethyl acetate solution to give the title compound as a white powder (0.28 g, 78%).
MS(ESI+): 574(M-HCl+H)

The chemical structures of the compounds obtained in Examples 53-54 are as shown in Table 11.

**[Table 11]**

| 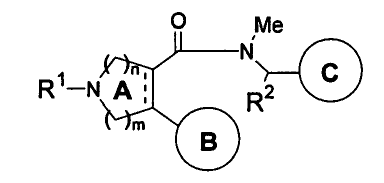 | | | | | | | |
|---|---|---|---|---|---|---|---|
| Ex. No. | R¹ | R² | 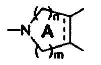 | 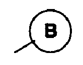 | 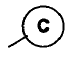 | Additives | MS (ESI) |
| 53 | 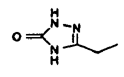 | H | 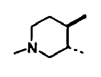 | 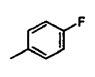 | 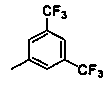 | HCl | **560 (M-HCl+H)** |
| 54 | 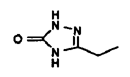 | H | 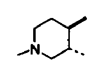 | 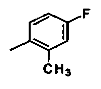 | 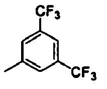 | HCl | **574 (M-HCl+H)** |

### Example 55

### (3R*,4R*)-N-[3,5-bis(trifluoromethyl)benzyl]-N-methyl-1-[(1-methyl-2,6-dioxopiperidin-4-yl)carbonyl]-3-phenylpiperidine-4-carboxamide

A solution of the compound (0.40 g) obtained in Example 36, methyl iodide (0.18 mL) and potassium carbonate (0.19 g) in DMF (5.0 mL) was stirred at 60°C for 2 hr. The reaction mixture was poured into water, and the product was extracted with ethyl acetate. The organic layer was washed with 10% aqueous citric acid solution and brine and dried, and the solvent was evaporated under reduced pressure. The obtained residue was purified by preparative HPLC to give the title compound as a white powder (0.40 g, 98%).
MS(ESI+): 598 (M+H)

The chemical structure of the compound obtained in Examples 55 is as shown in Table 12.

**[Table 12]**

| 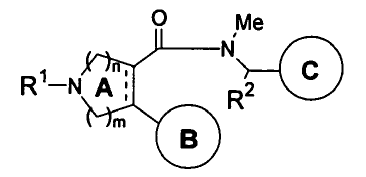 | | | | | | | |
|---|---|---|---|---|---|---|---|
| Ex. No. | R¹ | R² | 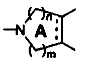 | 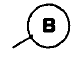 | 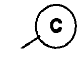 | Additives | MS (ESI) |
| 55 | 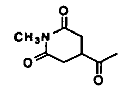 | H | 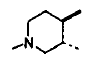 | 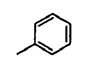 | 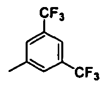 | | **598 (M+H)** |

### Example 56

### (3R*,4R*)-1-[(4-acetyl-4-phenylpiperidin-1-yl)carbonyl]-N-[3,5-bis(trifluoromethyl)benzyl]-N-methyl-3-phenylpiperidine-4-carboxamide

To a solution of the compound (0.48 g) obtained in Example 9 and Et₃N (0.28 mL) in CH₂Cl₂ (5.0 mL) was added 4-nitrophenyl chloroformate (0.24 g) at 0°C, and the mixture was stirred at room temperature for 1 hr. The reaction mixture was poured into water, and the product was extracted with ethyl acetate. The organic layer was washed with saturated aqueous ammonium chloride solution and brine and dried, and the solvent was evaporated under reduced pressure. The obtained residue was purified by silica gel column chromatography (50% ethyl acetate/hexane) to give a white amorphous solid (0.61 g, 100%).

A solution of the obtained white amorphous solid (0.32 g), 4-acetyl-4-phenylpiperidine hydrochloride (0.16 g) and potassium carbonate (0.15 g) in DMF (5.0 mL) was stirred at 120°C for 2 hr. The reaction mixture was poured into water, and the product was extracted with ethyl acetate. The organic layer was washed with saturated aqueous ammonium chloride solution and brine and dried, and the solvent was evaporated under reduced pressure. The residue was purified by preparative HPLC to give the title compound as a colorless oil (0.28 g, 78%).
MS(ESI+): 674(M+H)

Using the compound obtained in Example 9 and corresponding each amine derivative, and in the same manner as in Example 56, the following compounds were obtained.

### Example 57

### (3R*,4R*)-1-[(4-acetyl-1-piperazinyl)carbonyl]-N-[3,5-bis(trifluoromethyl)benzyl]-N-methyl-3-phenylpiperidine-4-carboxamide

### Example 58

### (3R*,4R*)-1-[[4-(acetylamino)-4-phenylpiperidin-1-yl]carbonyl]-N-[3,5-bis(trifluoromethyl)benzyl]-N-methyl-3-phenylpiperidine-4-carboxamide

The chemical structures of the compounds obtained in Examples 56-58 are as shown in Table 13.

**[Table 13]**

| 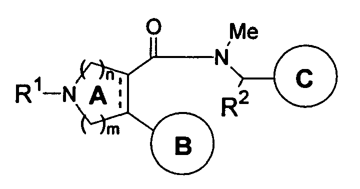 | | | | | | | |
|---|---|---|---|---|---|---|---|
| Ex. No. | R¹ | R² | 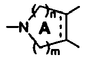 | 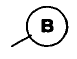 | 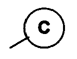 | Additives | MS (ESI) |
| 56 | 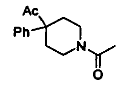 | H | 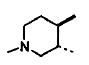 | 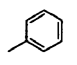 | 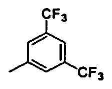 | | **674 (M+H)** |
| 57 | 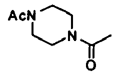 | H | 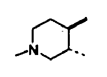 | 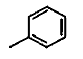 | 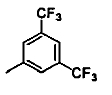 | | **599 (M+H)** |
| 58 | 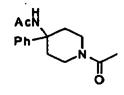 | H | 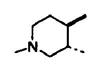 | 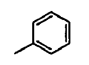 | 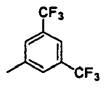 | | **689 (M+H)** |

### Example 59

### ethyl 4-[[[3,5-bis(trifluoromethyl)benzyl](methyl)amino]carbonyl]-5-phenyl-2,3,6,7-tetrahydro-1H-azepine-1-carboxylate

To a solution of the compound (1.20 g) obtained in Reference Example 8 in DMF (15 mL) was added sodium hydride (60% in oil, 0.13 g), and the mixture was stirred at room temperature for 30 min and then cooled to 0°C. Methyl iodide (2.0 mL) was added, and the mixture was stirred at room temperature for 2 hr. The reaction mixture was poured into diluted hydrochloric acid, and the product was extracted with ethyl acetate. The organic layer was washed with water and brine and dried, and the solvent was evaporated under reduced pressure. The obtained residue was purified by silica gel column chromatography (solvent gradient; 10→50% ethyl acetate/hexane) to give the title compound (0.73 g, 59%) as a white powder.
MS(ESI+): 529(M+H)

The chemical structure of the compound obtained in Example 59 is as shown in Table 14.

**[Table 14]**

| 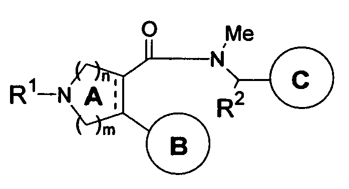 | | | | | | | |
|---|---|---|---|---|---|---|---|
| Ex. No. | R¹ | R² | 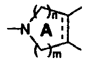 | 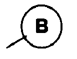 | 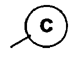 | Additives | MS (ESI) |
| 59 | C₂H₅OCO | H | 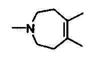 | 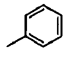 | 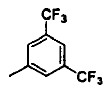 | | **529 (M+H)** |

### Example 60

### 1-[(1-acetylpiperidin-4-yl)carbonyl]-N-[3,5-bis(trifluoromethyl)benzyl]-N-methyl-5-phenyl-2,3,6,7-tetrahydro-1H-azepine-4-carboxamide

A solution of the compound (0.50 g) obtained in Example 59 in a mixture of acetic acid (3 mL) and hydrochloric acid (3 mL) was stirred at 140°C for 22 hr. The reaction mixture was basified with aqueous potassium carbonate solution and the product was extracted with ethyl acetate and dried, and the solvent was evaporated under reduced pressure. To a solution of the obtained residue in DMF (10 mL) were added 1-acetylpiperidine-4-carboxylic acid (0.67 g), WSC·HCl (1.10 g) and HOBt·H2O (0.80 g), and the mixture was stirred at room temperature for 24 hr. The reaction mixture was poured into water, and the product was extracted with ethyl acetate. The organic layer was washed with diluted hydrochloric acid and diluted aqueous sodium hydroxide solution and dried, and the solvent was evaporated under reduced pressure. The residue was purified by silica gel column chromatography (solvent gradient; 0→20% methanol/ethyl acetate) to give the title compound (0.59 g, 74%) as a pale-yellow oil.
MS(ESI+): 610(M+H)

The chemical structure of the compound obtained in Example 60 is as shown in Table 15.

**[Table 15]**

| 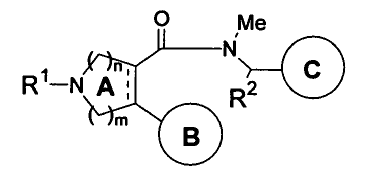 | | | | | | | |
|---|---|---|---|---|---|---|---|
| Ex. No. | R¹ | R² | 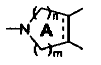 | 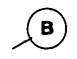 | 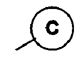 | Additives | MS (ESI) |
| 60 | 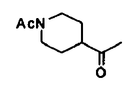 | H | 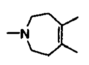 | 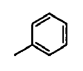 | 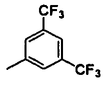 | | **610 (M+H)** |

### Example 61

### (4S*,5R*)-1-[(1-acetylpiperidin-4-yl)carbonyl]-N-[3,5-bis(trifluoromethyl)benzyl]-N-methyl-5-phenylazepane-4-carboxamide

A solution of the compound (0.34 g) obtained in Example 60 and 10% palladium-carbon (0.20 g) in acetic acid (10 mL) was stirred under a hydrogen atmosphere of 5 atm at 80°C for 3 hr. The catalyst was filtered off and the filtrate was diluted with ethyl acetate, washed with 1N aqueous sodium hydroxide solution, saturated aqueous sodium hydrogen carbonate solution and brine, and dried. The solvent was concentrated under reduced pressure to give the title compound (0.31 g, 92%) as a pale-yellow oil.
MS(ESI+): 612 (M+H)

The chemical structure of the compound obtained in Example 61 is as shown in Table 16.

**[Table 16]**

| 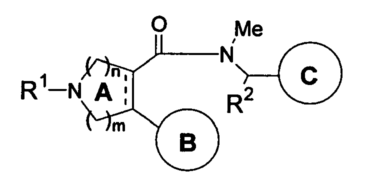 | | | | | | | |
|---|---|---|---|---|---|---|---|
| Ex. No. | R¹ | R² | 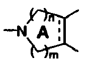 | 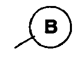 | 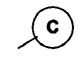 | Additives | MS (ESI) |
| 61 | 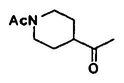 | H | 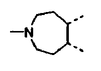 | 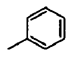 | 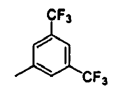 | | **612 (M+H)** |

### Example 62

### N-[3,5-bis(trifluoromethyl)benzyl]-5-(3,4-dichlorophenyl)-N-methyl-1,2,3,6-tetrahydropyridine-4-carboxamide hydrochloride

### (Step 1)

A solution of the compound (2.50 g) obtained in Reference Example 9 in thionyl chloride (10 mL) was stirred at 80°C for 1 hr. The reaction solution was concentrated under reduced pressure and the obtained residue was dissolved in acetonitrile (20 mL). Et₃N (3.94 mL) and [3,5-bis(trifluoromethyl)benzyl]methylamine (2.76 g) were added at 0°C, and the mixture was stirred at room temperature for 14 hr. The reaction mixture was poured into water, and the product was extracted with ethyl acetate. The organic layer was washed with saturated aqueous ammonium chloride solution and brine, and dried. The solvent was evaporated under reduced pressure to give crude 1-benzyl-N-[3,5-bis(trifluoromethyl)benzyl]-5-(3,4-dichlorophenyl)-N-methyl-1,2,3,6-tetrahydropyridine-4-carboxamide (3.63 g) as a white powder.

### (Step 2)

To a solution of the compound (3.63 g) obtained in Step 1 in chloroform (10 mL) was added 1-chloroethyl chloroformate (8.0 mL) at room temperature, and the mixture was stirred at 100°C for 1 hr. The reaction solution was concentrated under reduced pressure and the obtained residue was dissolved in methanol (30 mL), and the mixture was stirred at 80°C for 1 hr. The reaction solution was concentrated under reduced pressure and the obtained residue was dissolved in acetonitrile (20 mL). Et₃N (1.26 mL) and Boc₂O (1.32 g) were added, and the mixture was stirred at room temperature for 14 hr. The reaction mixture was poured into water, and the product was extracted with ethyl acetate. The organic layer was washed with saturated aqueous ammonium chloride solution and brine and dried, and the solvent was evaporated under reduced pressure. The obtained residue was purified by silica gel column chromatography (solvent gradient; 10→20% ethyl acetate/hexane) to give a colorless oil (2.61 g). To a solution of the obtained oil (0.80 g) in methanol (20 mL) was added 4N hydrogen chloride/ethyl acetate (1.31 mL) solution, and the mixture was stirred at 50°C for 2 hr. The reaction solution was concentrated under reduced pressure and the precipitated product was collected by filtration to give the title compound as a white powder (0.72 g).
MS(ESI+): 511(M-HCl+H)

### Example 63

### 1-[(1-acetylpiperidin-4-yl)carbonyl]-N-[3,5-bis(trifluoromethyl)benzyl]-5-(3,4-dichlorophenyl)-N-methyl-1,2,3,6-tetrahydropyridine-4-carboxamide

Using the compound obtained in Example 62, and by the reaction and treatment in the same manner as in Example 30, the title compound was obtained as a white amorphous solid (0.20 g, 82%).
MS(ESI+): 664 (M+H)

### Example 64

### 1-[[4-(acetylamino)-4-phenylpiperidin-1-yl]carbonyl]-N-[3,5-bis(trifluoromethyl)benzyl]-5-(3,4-dichlorophenyl)-N-methyl-1,2,3,6-tetrahydropyridine-4-carboxamide

To a solution of the compound (0.20 g) obtained in Example 62 and Et₃N (0.10 mL) in acetonitrile (5.0 mL) was added 4-nitrophenyl chloroformate (0.089 g) at 0°C, and the mixture was stirred at room temperature for 1 hr. The reaction mixture was poured into water, and the product was extracted with ethyl acetate. The organic layer was washed with saturated aqueous ammonium chloride solution and brine and dried, and the solvent was evaporated under reduced pressure. To a solution of the obtained residue in DMF (5.0 mL) were added 4-acetylamino-4-phenylpiperidine hydrochloride (0.19 g) and potassium carbonate (0.10 g), and the mixture was stirred at 50°C for 2 hr. The reaction mixture was poured into water, and the product was extracted with ethyl acetate. The organic layer was washed with saturated aqueous ammonium chloride solution and brine and dried, and the solvent was evaporated under reduced pressure. The residue was purified by preparative HPLC to give the title compound as a colorless oil (0.19 g, 68%).
MS(ESI+): 755(M+H)

### Example 65

### 1-[[4-(acetylamino)-4-phenylpiperidin-1-yl]acetyl]-N-[3,5-bis(trifluoromethyl)benzyl]-5-(3,4-dichlorophenyl)-N-methyl-1,2,3,6-tetrahydropyridine-4-carboxamide hydrochloride

To a solution of the compound (0.20 g) obtained in Example 62 and Et₃N (0.10 mL) in acetonitrile (10 mL) was added bromoacetyl chloride (0.061 mL) at 0°C, and the mixture was stirred at room temperature for 15 min. The reaction mixture was poured into water, and the product was extracted with ethyl acetate. The organic layer was washed with saturated aqueous ammonium chloride solution and brine and dried, and the solvent was evaporated under reduced pressure. To a solution of the obtained residue in DMF (5.0 mL) were added 4-acetylamino-4-phenylpiperidine hydrochloride (0.19 g) and potassium carbonate (0.10 g), and the mixture was stirred at 50°C for 14 hr. The reaction mixture was poured into water, and the product was extracted with ethyl acetate. The organic layer was washed with saturated aqueous ammonium chloride solution and brine and dried, and the solvent was evaporated under reduced pressure. The residue was purified by preparative HPLC to give a colorless oil (0.17 g, 60%). The obtained product was treated with 1 equivalent of 4N hydrogen chloride/ethyl acetate solution to give the title compound as a white powder.
MS(ESI+): 769(M+H)

### Example 66

### 1-[3-[4-(acetylamino)-4-phenylpiperidin-1-yl]propanoyl]-N-[3,5-bis(trifluoromethyl)benzyl]-5-(3,4-dichlorophenyl)-N-methyl-1,2,3,6-tetrahydropyridine-4-carboxamide hydrochloride

Using the compound (0.20 g) obtained in Example 62 and 3-bromopropionyl chloride, and by the reaction and treatment in the same manner as in Example 65, the title compound was obtained as a white powder.
MS(ESI+): 783(M+H)

The chemical structures of the compounds obtained in Examples 62-66 are as shown in Table 17.

**[Table 17]**

| 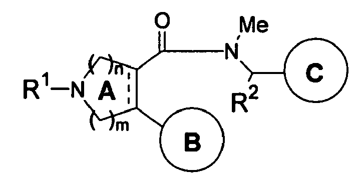 | | | | | | | |
|---|---|---|---|---|---|---|---|
| Ex. No. | R¹ | R² | 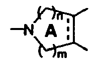 | 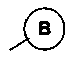 | 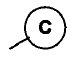 | Additives | MS (ESI) |
| 62 | H | H | 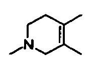 | 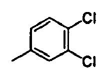 | 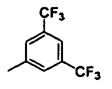 | **HCl** | **511 (M-HCl+H)** |
| 63 | 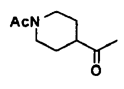 | H | 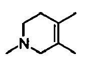 | 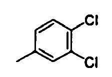 | 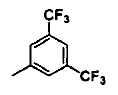 | | **664 (M+H)** |
| 64 | 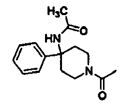 | H | 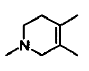 | 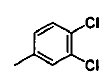 | 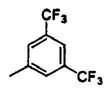 | | **755 (M+H)** |
| 65 | 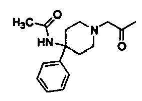 | H | 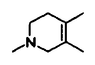 | 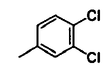 | 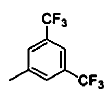 | **HCl** | **769 (M-HCl+H)** |
| 66 | 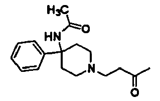 | H | 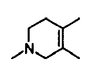 | 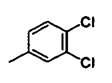 | 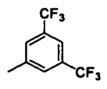 | **HCl** | **783 (M-HCl+H)** |

### Example 67

### (3R*,4R*)-N-[3,5-bis(trifluoromethyl)benzyl]-3-(4-fluoro-2-methylphenyl)-N-methyl-1-[(2-oxopyrrolidin-1-yl)acetyl]piperidine-4-carboxamide

To a solution of the compound (0.20 g) obtained in Example 12, (2-oxopyrrolidin-1-yl)acetic acid (0.13 g) and Et₃N (0.070 mL) in DMF (5.0 mL) were added WSC·HCl (0.14 g) and HOBt·H2O (0.12 g), and the mixture was stirred at room temperature for 24 hr. The reaction mixture was poured into water, and the product was extracted with ethyl acetate. The organic layer was washed with 10% aqueous citric acid solution and brine and dried, and the solvent was evaporated under reduced pressure. The obtained residue was purified by preparative HPLC to give the title compound as a white powder (0.15 g, 62%).
MS(ESI+): 602 (M+H)

Using the compound obtained in Example 12 and corresponding each carboxylic acid, and in the same manner as in Example 67, the following compounds were obtained.

### Example 68

### (3R*,4R*)-N-[3,5-bis(trifluoromethyl)benzyl]-1-[(2,5-dioxopyrrolidin-1-yl)acetyl]-3-(4-fluoro-2-methylphenyl)-N-methylpiperidine-4-carboxamide

### Example 69

### tert-butyl [trans-4-[[(3R*,4R*)-4-[[[3,5-bis(trifluoromethyl)benzyl](methyl)amino]carbonyl]-3-(4-fluoro-2-methylphenyl)piperidin-1-yl]carbonyl]cyclohexyl]carbamate

### Example 70

### (3R*,4R*)-1-[trans-(4-aminocyclohexyl)carbonyl]-N-[3,5-bis(trifluoromethyl)benzyl]-3-(4-fluoro-2-methylphenyl)-N-methylpiperidine-4-carboxamide hydrochloride

To a solution of the compound (2.10 g) obtained in Example 69 in methanol (10 mL) was added 4N hydrogen chloride/ethyl acetate solution (3.0 mL), and the mixture was stirred at 50°C for 2 hr. The reaction mixture was concentrated under reduced pressure and crystallized from hexane to give the title compound as a white powder (2.0 g, 100%).
MS(ESI+): 602(M-HCl+H)

Using the compound obtained in Example 70, and in the same manner as in Example 28, the following compounds were obtained.

### Example 71

### (3R*,4R*)-1-[[trans-4-(acetylamino)cyclohexyl]carbonyl]-N-[3,5-bis(trifluoromethyl)benzyl]-3-(4-fluoro-2-methylphenyl)-N-methylpiperidine-4-carboxamide

### Example 72

### ethyl [(3R*,4R*)-4-[[[3,5-bis(trifluoromethyl)benzyl](methyl)amino]carbonyl]-3-(4-fluoro-2-methylphenyl)piperidin-1-yl](oxo)acetate

To a solution of the compound (0.30 g) obtained in Example 12 and Et₃N (0.12 mL) in CH₂Cl₂ (10 mL) was added ethyl chloroglyoxylate (0.20 mL) at 0°C, and the mixture was stirred at room temperature for 24 hr. The reaction mixture was poured into water, and the product was extracted with ethyl acetate. The organic layer was washed with saturated aqueous ammonium chloride solution and brine, and dried. The solvent was evaporated under reduced pressure to give the title compound as a white powder (0.33 g, 97%).
MS(ESI+): 577 (M+H)

### Example 73

### (3R*,4R*)-1-[amino(oxo)acetyl]-N-[3,5-bis(trifluoromethyl)benzyl]-3-(4-fluoro-2-methylphenyl)-N-methylpiperidine-4-carboxamide

A solution of the compound (0.10 g) obtained in Example 72 and 28% aqueous ammonia (1.0 mL) in ethanol (1.0 mL) was stirred at 100°C for 4 hr in a sealed tube. The reaction mixture was poured into water, and the product was extracted with ethyl acetate. The organic layer was washed with saturated aqueous ammonium chloride solution and brine and dried, and the solvent was evaporated under reduced pressure. The obtained residue was purified by silica gel column chromatography (solvent gradient; 0→10% methanol/ethyl acetate-hexane (1:1)) to give the title compound as a white amorphous solid (0.030 g, 33%).

¹H-NMR(CDCl₃)δ: 1.80-2.10 (2H, m), 2.30-3.70 (11H, m), 4,20 (1H, d, J=15.0Hz), 4.50-5.66 (3H, m), 4.84 (1H, d, J=15.0Hz), 6.70-7.10 (3H, m), 7.35-7.46 (2H, m), 7.73-7.85 (1H, m)
MS(ESI+): 548(M+H)

Using the compound obtained in Example 72 and 40% aqueous methylamine solution, and in the same manner as in Example 73, the following compounds were obtained.

### Example 74

### (3R*,4R*)-N-[3,5-bis(trifluoromethyl)benzyl]-3-(4-fluoro-2-methylphenyl)-N-methyl-1-[(methylamino)(oxo)acetyl]piperidine-4-carboxamide

### Example 75

### (3R*,4R*)-N⁴-[3,5-bis(trifluoromethyl)benzyl]-3-(4-fluoro-2-methylphenyl)-N⁴-methylpiperidine-1,4-dicarboxamide

To a solution of the compound (0.15 g) obtained in Example 12 and Et₃N (0.082 mL) in THF (3 mL) was added triphosgene (0.17 g) at 0°C and the mixture was stirred for 20 min. 28% Aqueous ammonia (1.0 mL) was added, and the mixture was stirred at room temperature for 24 hr. The reaction mixture was poured into water, and the product was extracted with ethyl acetate. The organic layer was washed with saturated aqueous ammonium chloride solution and brine and dried, and the solvent was evaporated under reduced pressure. The obtained residue was purified by silica gel column chromatography (solvent gradient; 0→10% methanol/ethyl acetate-hexane (1:1)) to give the title compound as a white powder (0.10 g, 67%).
¹H-NMR(CDCl₃)δ 1.90-2.10 (2H, m), 2.34-2.47 (3H, m), 2.75-3.20 (6H, m), 3.40-3.55 (1H, m), 3.80-3.95 (1H, m), 4.10-4.30 (1H, m), 4.21 (1H, d, J=15.0Hz), 4.40-4.60 (2H, m), 4.82 (1H, d, J=15.0Hz), 6.70-6.86 (2H, m), 7.00-7.08 (1H, m), 7.35-7.45 (2H, m), 7.73-7.84 (1H, m)
MS (ESI+) : 520 (M+H)

Using the compound obtained in Example 12 and 40% aqueous methylamine solution, and in the same manner as in Example 75, the following compounds were obtained.

### Example 76

### (3R*,4R*)-N⁴-[3,5-bis(trifluoromethyl)benzyl]-3-(4-fluoro-2-methylphenyl)-N¹,N⁴-dimethylpiperidine-1,4-dicarboxamide

### Example 77

### (3R*,4R*)-1-(N-acetylglycyl)-N-(3,5-bis(trifluoromethyl)benzyl]-3-(4-fluoro-2-methylphenyl)-N-methylpiperidine-4-carboxamide

To a solution of the compound (0.20 g) obtained in Example 12, N-acetylglycine (0.060 g) and Et₃N (0.082 mL) in DMF (5.0 mL) were added WSC·HCl (0.11 g) and HOBt·H2O (0.090 g), and the mixture was stirred at room temperature for 24 hr. The reaction mixture was poured into water, and the product was extracted with ethyl acetate. The organic layer was washed with saturated aqueous sodium hydrogen carbonate solution and brine and dried, and the solvent was evaporated under reduced pressure. The obtained residue was purified by preparative HPLC to give the title compound as a white powder (0.17 g, 76%)
¹H-NMR(CDCl₃)δ: 1.80-2.10 (5H, m), 2.35-4.25 (14H, m), 4.45-4.90 (2H, m), 6.60-7.10 (4H, m), 7.34-7.46 (2H, m), 7.73-7.85 (1H, m)
MS(ESI+): 576(M+H)

Using the compound obtained in Example 12 and (2,6-dioxopiperidin-4-yl)carboxylic acid, and in the same manner as in Example 77, the following compounds were obtained.

### Example 78

### (3R*,4R*)-N-[3,5-bis(trifluoromethyl)benzyl]-1-[(2,6-dioxopiperidin-4-yl)carbonyl]-3-(4-fluoro-2-methylphenyl)-N-methylpiperidine-4-carboxamide

### Example 79

### (3R*,4R*)-N-[3,5-bis(trifluoromethyl)benzyl]-3-(4-fluoro-2-methylphenyl)-N-methyl-1-[(1-methyl-2,6-dioxopiperidin-4-yl)carbonyl]piperidine-4-carboxamide

A solution of the compound (0.20 g) obtained in Example 78, methyl iodide (0.085 mL) and potassium carbonate (0.090 g) in DMF (5.0 mL) was stirred at 60°C for 4 hr. The reaction mixture was poured into water, and the product was extracted with ethyl acetate. The organic layer was washed with 10% aqueous citric acid solution and brine and dried, and the solvent was evaporated under reduced pressure. The obtained residue was purified by preparative HPLC to give the title compound as a white powder (0.21 g, 100%).
MS(ESI+): 630(M+H)

Using the compound obtained in Example 12 and aqueous O-methylhydroxyamine solution, and in the same manner as in Example 75, the following compounds were obtained.

### Example 80

### (3R*,4R*)-N⁴-[3,5-bis(trifluoromethyl)benzyl]-3-(4-fluoro-2-methylphenyl)-N¹-methoxy-N⁴-methylpiperidine-1,4-dicarboxamide

The chemical structures of the compounds obtained in Examples 67-80 are as shown in Table 18.

**[Table 18]**

| | | | | | | | |
|---|---|---|---|---|---|---|---|
| 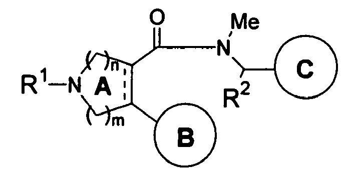 | | | | | | | |

| Ex. No. | R¹ | R² | 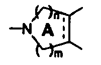 | 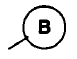 | 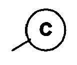 | Additives | MS (ESI) |
|---|---|---|---|---|---|---|---|
| 67 | 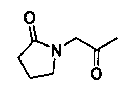 | H | 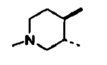 | 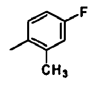 | 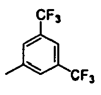 | | **602 (M+H)** |
| 68 | 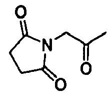 | H | 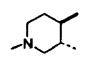 | 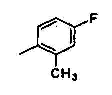 | 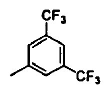 | | **616 (M+H)** |
| 69 | 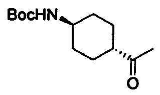 | H | 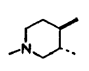 | 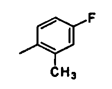 | 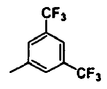 | | **702 (M+H)** |
| 70 | 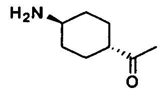 | H | 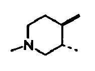 | 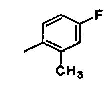 | 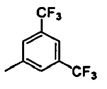 | HCl | **602 (M-HCl+H)** |
| 71 | 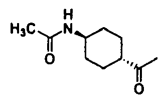 | H | 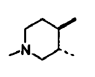 | 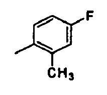 | 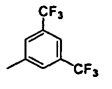 | | **644 (M+H)** |
| 72 | 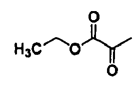 | H | 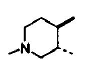 | 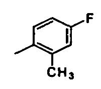 | 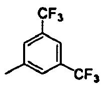 | | **577 (M+H)** |
| 73 | 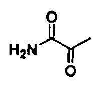 | H | 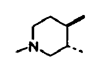 | 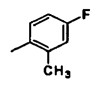 | 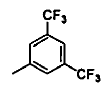 | | **548 (M+H)** |
| 74 | 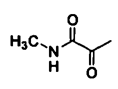 | H | 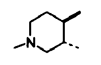 | 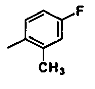 | 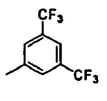 | | **562 (M+H)** |
| 75 | 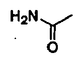 | H | 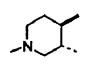 | 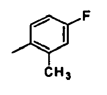 | 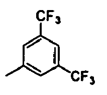 | | **520 (M+H)** |
| 76 | 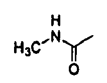 | H | 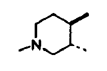 | 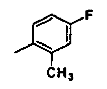 | 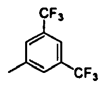 | | **534 (M+H)** |
| 77 | 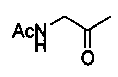 | H | 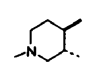 | 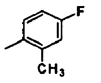 | 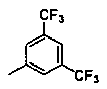 | | **576 (M+H)** |
| 78 | 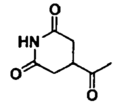 | H | 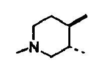 | 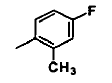 | 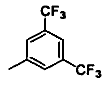 | | **616 (M+H)** |
| 79 | 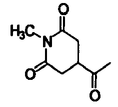 | H | 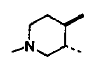 | 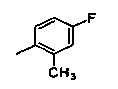 | 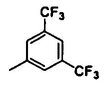 | | **630 (M+H)** |
| 80 | 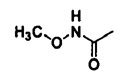 | H | 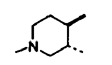 | 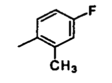 | 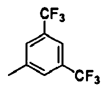 | | **550 (M+H)** |

### Preparative Example 1

| | | |
|---|---|---|
| (1) | Compound of Example 1 | 10 mg |
| (2) | Lactose | 60 mg |
| (3) | Corn starch | 35 mg |
| (4) | Hydroxypropylmethylcellulose | 3 mg |
| (5) | Magnesium stearate | 2 mg |

A mixture of the compound (10 mg) obtained in Example 1, lactose (60 mg) and corn starch (35 mg) is granulated using an aqueous solution (0.03 mL) of 10 wt% hydroxypropylmethylcellulose (3 mg as hydroxypropylmethylcellulose), and then dried at 40°C and sieved. The obtained granules are mixed with magnesium stearate (2 mg) and compressed. The obtained uncoated tablets are sugar-coated with an aqueous suspension of sucrose, titanium dioxide, talc and gum Arabic. The thus-coated tablets are glazed with bees wax to obtain finally-coated tablets.

### Preparative Example 2

| | | |
|---|---|---|
| (1) | Compound of Example 1 | 10 mg |
| (2) | Lactose | 70 mg |
| (3) | Corn starch | 50 mg |
| (4) | Soluble starch | 7 mg |
| (5) | Magnesium stearate | 3 mg |

The compound obtained in Example 1 (10 mg) and magnesium stearate (3 mg) are granulated with an aqueous soluble starch solution (0.07 mL, 7 mg as soluble starch), dried, and mixed with lactose (70 mg) and corn starch (50 mg). The mixture is compressed to obtain tablets.

### Reference Preparative Example 1

| | | |
|---|---|---|
| (1) | Rofecoxib | 5.0 mg |
| (2) | Sodium chloride | 20.0 mg |
| (3) | Distilled water | to 2.0 mL of total volume |

Rofecoxib (5.0 mg) and sodium chloride (20.0 mg) are dissolved in distilled water, and water is added to make the total volume 2.0 mL. The solution is filtered, and filled into ampoule (2 mL) under sterile condition. The ampoule is sterilized, and then sealed to obtain a solution for injection.

### Reference Preparative Example 2

| | | |
|---|---|---|
| (1) | Rofecoxib | 50 mg |
| (2) | Lactose | 34 mg |
| (3) | Corn starch | 10.6 mg |
| (4) | Corn starch (paste) | 5 mg |
| (5) | Magnesium stearate | 0.4 mg |
| (6) | Calcium carboxymethylcellulose | 20 mg |
| | total | 120 mg |

The above-mentioned (1) to (6) were mixed according to a conventional method and tableted by a tablet machine to obtain tablets.

### Preparative Example 3

The formulation prepared in Preparative Example 1 or 2, and the formulation prepared in Reference Preparative Example 1 or 2 are combined.

### Experimental Example 1

### Radioligand receptor binding inhibitory activity (Binding inhibitory activity using receptor from human lymphoblast cells (IM-9))

The method of M. A. Cascieri et al., [Molecular Pharmacology, vol. 42, p. 458 (1992)] was modified and used. The receptor was prepared from human lymphoblast cells (IM-9). IM-9 cells (2 X 10⁵ cells/mL) were incubated for 3 days (one liter), which was then subjected to centrifugation for 5 minutes at 500 X G to obtain cell pellets. The obtained pellets were washed once with phosphate buffer (Flow Laboratories, CAT. No. 28-103-05), which were then homogenized using Polytron homogenizer ("Kinematika", Germany) in 30 mL of 50 mM Tris-HCl buffer (pH 7.4) containing 120 mM sodium chloride, 5 mM potassium chloride, 2 µg/mL chymostatin, 40 µg/mL bacitracin, 5 µg/mL phosphoramidon, 0.5 mM phenylmethylsulfonyl fluoride, 1 mM ethylenediamine tetraacetate, which was subjected to centrifugation at 40,000 X G for 20 minutes. The residue was washed twice with 30 mL of the above-mentioned buffer, which was then preserved frozen (-80°C) as a specimen of the receptor.

The specimen was suspended in a reaction buffer (50 mM Tri-HCl buffer (pH 7.4), 0.02% bovine serum albumin, 1 mM phenylmethylsulfonyl fluoride, 2 µg/mL chymostatin, 40 µg/mL bacitracin and 3 mM manganese chloride) to have protein in the concentration of 0.5 mg/mL of protein and 100 µl portion of the suspension was used in the reaction. After addition of the sample and ¹²⁵I-BHSP (0.46 KBq), the reaction was allowed to proceed in 0.2 mL of reaction buffer at 25°C for 30 minutes. The amount of nonspecific binding was determined by adding substance P at a final concentration of 2 X 10⁻⁶ M.

After the reaction, using a cell harvester (290 PHD, Cambridge Technology, Inc, U.S.A.), filtration was carried out through a glass filter (GF/B, Whatman, U.S.A.), which was immersed in 0.1% polyethyleneimine for 24 hours and dried. After washing three times with 250 µL of 50 mM Tris-HCl buffer (pH 7.4) containing 0.02% bovine serum albumin, the radioactivity remaining on the filter was determined with a gamma counter.

The antagonistic activity of each compound obtained in Examples was determined in terms of the concentration necessary to cause 50% inhibition (IC₅₀ value) under the above-described conditions, and the results were shown in Table 19.

**[Table 19]**

| Example No. | IC₅₀ (nM) |
|---|---|
| 12 | 0.051 |
| 30 | 0.049 |
| 35 | 0.098 |
| 40 | 0.058 |
| 41 | 0.025 |
| 42 | 0.022 |
| 43 | 0.064 |
| 44 | 0.019 |
| 45 | 0.052 |
| 46 | 0.047 |
| 54 | 0.036 |
| 73 | 0.052 |
| 75 | 0.035 |
| 77 | 0.043 |

Radioligand means substance P labeled with [¹²⁵I].

From Table 19, it is understood that the compounds of the present invention have an excellent antagonistic action for the substance P receptor.

This application is based on a patent application No. 2004-7373 filed in Japan, the contents of which are incorporated in full herein by this reference.

## Claims

1. A compound represented by the formula 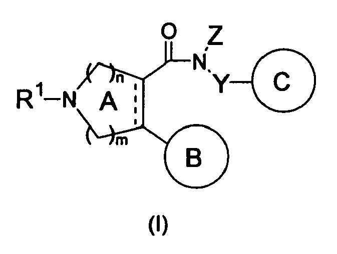 wherein ring A is a nitrogen-containing heterocycle optionally further having substituent(s), ring B and ring C are each an aromatic ring optionally having substituent(s), R¹ is a hydrogen atom, a hydrocarbon group optionally having substituent(s), an acyl group or a heterocyclic group optionally having substituent(s), Z is an optionally halogenated C₁₋₆ alkyl group, Y is a methylene group optionally having substituent(s), m and n are each an integer of 0 to 5, m+n is an integer of 2 to 5, and 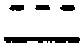 is a single bond or a double bond, or a salt thereof.

2. The compound of claim 1, wherein ring A is any of the rings shown by 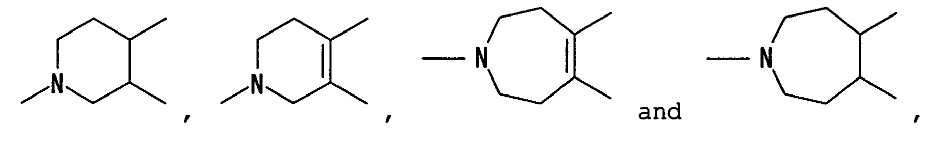 ring B is an optionally substituted phenyl group,
ring C is a phenyl group optionally having, as a substituent, a C₁₋₆ alkyl group optionally substituted by a halogen atom,
Z is a C₁₋₆ alkyl group, and
Y is a methylene group optionally substituted by a C₁₋₄ alkyl group.

3. The compound of claim 1, wherein ring A is any of the rings shown by 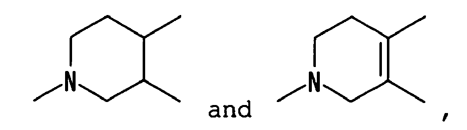 ring B is a phenyl group optionally substituted by substituent(s) selected from a group consisting of
(1) a halogen atom and
(2) a C₁₋₆ alkyl group,
ring C is a phenyl group optionally having, as a substituent, a C₁₋₆ alkyl group optionally substituted by a halogen atom,
R¹ is (1) a hydrogen atom,
(2) a C₁₋₄ alkyl group having, as a substituent, a 5- to 7-membered aromatic or non-aromatic heterocyclic group containing, besides carbon atoms, 1 to 4 heteroatoms selected from a group consisting of an oxygen atom, a sulfur atom and a nitrogen atom and optionally having one or two oxo as substituents, or
(3) an acyl group represented by the formula: -(C=O)-R^{2'}, -(C=O)-OR^{2'} or -(C=O)-NR^{2'}R³
wherein R^{2'} is
(a) a hydrogen atom,
(b) a 5- to 7-membered non-aromatic heterocyclic group containing, besides carbon atoms, 1 or 2 nitrogen atoms, and optionally having 1 to 3 substituents selected from a group consisting of oxo, C₁₋₆ alkyl, phenyl, C₁₋₆ alkyl-carbonyl and C₁₋₆ alkyl-carbonylamino,
(c) a C₁₋₆ alkyl group optionally having substituent(s) selected from a group consisting of
(i) a 5- to 7-membered aromatic or non-aromatic heterocyclic group containing, besides carbon atoms, 1 to 4 nitrogen atoms, and optionally having one or two oxo as substituents,
(ii) a C₁₋₆ alkyl-carbonylamino group,
(iii) a mono- or di-C₁₋₆ alkylamino group, and
(iv) a C₁₋₆ alkoxy group,
(d) a C₁₋₆ alkoxy group,
(e) a C₃₋₈ cycloalkyl group optionally having 1 or 2 substituents selected from a group consisting of a C₁₋₆ alkyl-carbonylamino group, a C₁₋₆ alkoxy-carbonylamino group and an amino group,
(f) a carbamoyl group,
(g) a C₁₋₆ alkoxy-carbonyl group, or
(h) a C₁₋₆ alkyl-carbamoyl group, and
R³ is a hydrogen atom or a C₁₋₆ alkyl group,
Z is a C₁₋₆ alkyl group, and
Y is a methylene group optionally substituted by a C₁₋₄ alkyl group.

4. A compound selected from a group consisting of
(3R*,4S*)-1-[(1-acetylpiperidin-4-yl)carbonyl]-N-[3,5-bis(trifluoromethyl)benzyl]-N-methyl-3-phenylpiperidine-4-carboxamide,
(3R*,4R*)-N-[3,5-bis(trifluoromethyl)benzyl]-3-(4-fluoro-2-methylphenyl)-N-methyl-1-[(5-oxo-4,5-dihydro-1H-1,2,4-triazol-3-yl)methyl]piperidine-4-carboxamide,
(3R*,4R*)-1-[amino(oxo)acetyl]-N-[3,5-bis(trifluoromethyl)benzyl]-3-(4-fluoro-2-methylphenyl)-N-methylpiperidine-4-carboxamide,
(3R*,4R*)-N⁴-[3,5-bis(trifluoromethyl)benzyl]-3-(4-fluoro-2-methylphenyl)-N⁴-methylpiperidine-1,4-dicarboxamide, and
(3R*,4R*)-1-(N-acetylglycyl)-N-[3,5-bis(trifluoromethyl)benzyl]-3-(4-fluoro-2-methylphenyl)-N-methylpiperidine-4-carboxamide, or a salt thereof.

5. A prodrug of the compound of claim 1.

6. A pharmaceutical agent comprising the compound of claim 1 or a prodrug thereof.

7. The pharmaceutical agent of claim 6, which is a tachykinin receptor antagonist.

8. The pharmaceutical agent of claim 6, which is an agent for the prophylaxis or treatment of an abnormality of lower urinary tract functions, a digestive organ disease or a central nerve disease.

9. The pharmaceutical agent of claim 6, which is an agent for the prophylaxis or treatment of an overactive bladder, an irritable bowel syndrome, an inflammatory bowel disease, vomiting, nausea, depression, anxiety neurosis, an anxiety symptom, a pelvic visceral pain or interstitial cystitis.

10. A method for the prophylaxis or treatment of an abnormality of lower urinary tract functions, a digestive organ disease or a central nerve disease, which comprises administering an effective amount of the compound of claim 1 or a prodrug thereof to a mammal.

11. Use of the compound of claim 1 or a prodrug thereof for the production of an agent for the prophylaxis or treatment of an abnormality of lower urinary tract functions, a digestive organ disease or a central nerve disease.
